# EUROPEAN PATENT SPECIFICATION

(11) **EP 0 719 273 B1**
(45) Date of publication and mention of the grant of the patent: **03.11.2010**
(21) Application number: 94929832.7
(22) Date of filing: 16.09.1994
(51) Int. Cl.: C07H 19/10, C07H 19/20, A61K 31/70, C07F 9/6561, C07F 9/6509, C07F 9/6571, C07F 9/6558, A61K 31/685, C07F 9/6564, C07F 9/36, C07F 9/40

(54) **NUCLEOTIDE ANALOGS**
NUKLEOTIDANALOGE
ANALOGUES DE NUCLEOTIDES

(30) Priority: 17.09.1993 US 123483; 08.02.1994 US 193341
(43) Date of publication of application: 03.07.1996
(73) Proprietor: GILEAD SCIENCES, INC., Foster City, CA 94404 (US)
(72) Inventor: BISCHOFBERGER, Norbert, W., San Carlos, CA 94070 (US); JONES, Robert, J., Millbrane, CA 94030 (US); ARIMILLI, Murty, N., Fremont, CA 94555 (US); LIN, Kuei-Ying, Fremont, CA 94555 (US); LOUIE, Michael, S., San Mateo, CA 94403 (US); MCGEE, Lawwrence, R., Pacifica, CA 94044 (US); PRISBE, Ernest, J., Los Altos, CA 94024 (US); LEE, William, A., Los Altos, CA 94024 (US); CUNDY, Kenneth, C., Belmont, CA 94002 (US)
(74) Representative: Riedl, Peter
(86) International application number: PCT/US1994/010539
(87) International publication number: WO 1995/007920

(56) References cited:
- EP-A- 0 369 409
- EP-A- 0 398 231
- EP-A- 0 481 214
- WO-A-92/13869
- DE-A- 2 009 834
- DE-A- 4 138 584
- NUCLEOSIDES AND NUCLEOTIDES, vol.12, no.3-4, 1993 pages 279 - 294 D.WOLFF-KUGEL ET AL. 'Studies Towards the Synthesis of the Saturated and Unsaturated Carbocyclic Methylene Phosphonate Analogs of Dideoxyadenosine.'
- NUCLEOSIDES AND NUCLEOTIDES, vol.12, no.2, 1993 pages 157 - 162 J-L KRAUS 'New Phosphonate Analogues of 3'-thia-2',3'-dideoxycytidine (BCH-189).'
- TETRAHEDRON LETTERS., vol.33, no.37, 8 September 1992, OXFORD GB pages 5335 - 5338 G.JAHNE ET AL. 'Preparation of Carbocyclic Phosphonate Nucleosides.'
- JOURNAL OF MEDICINAL CHEMISTRY, vol.35, no.17, 21 August 1992, WASHINGTON US pages 3192 - 3196 G.A.FREEMAN ET AL '3'-Azido-3',5'-dideoxyt hymidine-5'-methylphosphonic Acid Diphosphate: Synthesis and HIV-1 Reverse Transcriptase Inhibition.'

## Description

### Background of the Invention

The present invention relates to novel nucleotide analog amidates and esters, their pharmaceutically acceptable acid addition salts, a process for their production, and to their use. The nucleotides of the present invention exhibit antitumor/antineoplastic activity, a broad spectrum of antimicrobial activity and certain other desirable activities.

Compounds related to the nucleotide analogs of the present invention may be found in: U.S. Patent Numbers 5,043,339, 5,108,994 and 5,166,198; EP 206 459; EP 253 412; EP 269 947; EP 270 885; EP 319 228; EP 343133; EP 398 231; EP 404 296; EP 465 297; EP 468 119; EP 468 866; EP 479 640; EP 481214; EP 494 370; EP 531597; PCT/GB91/01171; PCT/US92/01020; PCT/US92/05208; WO 91/19721; Bronson et al, Bioorg Medicinal Chem Lett (1992) 2:685-690; Bronson et al, J Med Chem, (1989) 32:1457-1463; Bronson et al, Nucleoside Analogs as Antiviral Agents, ACS Symposium Series 401, J.C. Martin, Ed., p. 72-87, American Chemical Society, Washington, DC (1989); Colla, et al, J Med Chem (1983) 26:602-604; Curley, et al, Antiviral Res (1990) 14:345-356; De Clercq, et al, Nature, (1986) 323:464-467; Farrow, et al, J Med Chem (1990) 33:1400-1406; Farquhar, et al, I. Pharm Sci (1983) 72:324-325; Freed, et al, Biochem Pharmacol (1989) 19:3193-3198; Freeman, et al, J Med Chem (1992) 35:3192-3196; Gabrielsen, B., et al, Antiviral Res Suppl J (1992) 17:149; Gumport, et al, Proc Natl Acad Sci (1971) 2559-2563; Juodka, et al, Coll Czech Chem Commun (1974) 39:963-968; Kim, et al, Bioorg Medicinal Chem Lett (1992) 2:367-370; Kim, et al, Tet Lett (1992) 33:25-28; Kim, et al, J Med Chem (1990) 33:1207-1213; Kumar, et al, J Med Chem (1990) 33:2368-2375; McGuigan, et al, Antiviral Chem Chemother (1993) 4:97-101; McGuigan, et al, Antiviral Res (1991) 15:255-263; Rosenberg, et al, Coll Czech Chem Commun (1988) 53:2753-2777; Rosenberg, et al, Coll Czech Chem Commun (1988) 52:2792-2800; Rosenberg, et al, Coll Czech Chem Commun (1988) 52:2801-2808; Starrett, et al, Antiviral Res (1992) 19:267-273; Yu, et al, J Med Chem (1992) 35:2958-2969; Wolff-Kugel, et al, Tet Lett (1991) 32:6341-6344.

A characteristic of nucleotide analogs or nucleotides having a phosphonate or a phosphate group is the presence of one or two negative charges associated with the phosphorus group at physiologic pH. The charge associated with moieties such as phosphate or phosphonate groups is believed to generally limit bioavailability by limiting cell membrane permeation via passive diffusion (Liebman, et al, J. Biol. Chem., (1955) 216:823-830; Roll, et al, J Biol Chem, (1956) 220:439-444; Srivastava, et al, Bioorg Chem (1984) 12:118-129; Palu, et al, Antiviral Res (1991) 16:115-119; Sastry, et al, Mol Pharmacol (1992) 41:441-445). These compounds are often, therefore, given parenterally in order to enhance bioavailability by increasing serum or intracellular levels.

Other characteristics of nucleotide analogs that can limit their efficacy include unfavorable pharmacokinetic or pharmacodynamic properties, insufficient potency and/or unfavorable toxicity characteristics.

Studies were conducted to ameliorate one or more of the above-mentioned problems associated with nucleotide analog drugs. The present invention includes novel nucleotide analogs that are hydrolyzable in vivo. The nucleotide analogs can have improved bioavailability, improved pharmacokinetic or pharmacodynamic properties, enhanced potency and/or improved toxicity characteristics compared to the corresponding unmodified nucleotide analog. Methods to synthesize and use the compounds and methods to obtain and use antibodies that recognize the compounds are also disclosed.

### Summery of the Invention

In a principal embodiment, the objects of this invention are accomplished by a nucleotide analog amidate comprising a phosphonate radical wherein the improvement comprises an amino acid residue or polypeptide radical in which an amino group of the amino acid or polypeptide is bonded to the phosphorus atom of the nucleotide analog by an amidate bond, a carboxyl group of the amino acid residue or polypeptide radical is positioned such that it is capable as the free acid of hydrolyzing the phosphoroamidate bond, and the carboxyl group is blocked (such as by moieties including esters or amides). The nucleotide analog amidates of this invention are hydrolyzed in vivo to the corresponding nucleotide analog and are thus precursors of the corresponding nucleotide analog.

In accordance with this invention the nucleotide analog amidates or a physiologically acceptable salt thereof, have the structure of formula Id 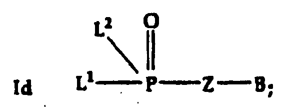 wherein L¹ is of the formula III 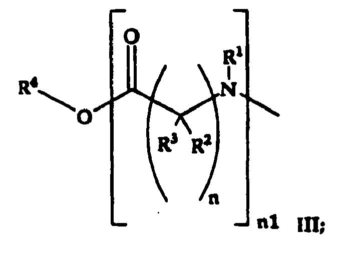 wherein L² is OR, SR or is independently of the formula III;
wherein n and n1 are 1;
R is N-ethylmorpholino, pivaloyloxymethyl, phenyl, benzyl, isopropyl, t-butyl, ethyl, butyl, adamantoyloxymethyl, 3-methoxyphenyl, 2-carboethoxyphenyl, 4-fluorophenyl, 2,4-difluorophenyl, 3,5-dimethoxyphenyl, 2,4-dichlorophenyl, 2-ethoxyphenyl, 3-dimethylaminophenyl, 4-trifluoromethylbenzyl, 2-ethylsalicyl, -CH₂-O-C(O)-C₁₀H₁₅, -C₆H₄-CH₂-N(CH₃)₂, -CH₂-CH₂F, -CH₂-CH₂Cl, -CH₂-CF₃, -CH₂-CCl₃, R⁵, NHR⁶ or N(R⁶)₂**;**
wherein,
R⁵ is CH₂C(O)N(R⁶)₂, CH₂C(O)OR⁶, CH₂OC(O)R⁶, CH(R⁶)OC(O)R⁶, CH₂C(R⁶)₂CH₂OH, or CH₂OR⁶, and
R⁶ is C₁-C₂₀ alkyl which is unsubstituted or substituted by substituents independently selected from the group consisting of OH, O, N and halogen (1 to 5 halogen atoms), 4-C₂₀ aryl which is unsubstituted or substituted by substituents independently selected from the group consisting of OH, O, N and halogen (1 to 5 halogen atoms) or C₇-C₂₀ aryl-alkyl which is unsubstituted or substituted by substituents independently selected from the group consisting of OH, O, N and halogen (1 to 5 halogen atoms);
R¹ is H, methyl, ethyl, isopropyl, phenyl or benzyl;
R² is H;
R³ is H, -CH₃, -CH(CH₃)₂, -CH₂-CH(CH₃)₂, -CHCH₃-CH₂-CH₃, -CH₂-C₆H₅, -CH₂CH₂-S-CH₃, -CH₂OH, -CH(OH)-CH₃, -CH₂-SH, -CH₂-C₆H₄OH,-CH₂-CO-NH₂, -CH₂-CH₂CO-NH₂, -CH₂-COOH, -CH₂CH₂-COOH, -(CH₂)₄-NH₂, -(CH₂)₃-NH-C(NH₂)-NH₂, 1-guanidinoprop-3-yl, benzyl, 4-hydroxybenzyl, imidazol-4-yl, indol-3-yl, methoxyphenyl or ethoxyphenyl;
R⁴ is methyl, ethyl, propyl, isopropyl, butyl, t-butyl, phenyl, benzyl, 1-pyridyl, 3-pyridyl, 1-pyrimidinyl, pivaloyloxymethyl, N-ethylmorpholino, N-2-propylmorpholino, methoxyethyl, 4-N-methylpiperidyl, 3-N-methylpiperidyl, 2-, 3-, and 4-N,N-dimethylaminophenyl and 2-, 3-, and 4-N,N-diethylaminophenyl or 1-ethylpiperazinyl;
Z is -CH₂-O-CH₂-CH₂-, or is of formula IV or V 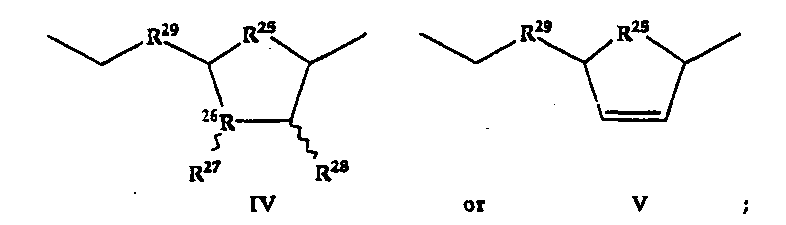 wherein
R²⁵ and R²⁹ are O;
R²⁶ is CH;
R²⁷ and R²⁸ are H; and
B is adenin-9-yl, 1-deazaadenin-9-yl, 3-deazaadenin-9-yl, 7-deaza-8-azaadenin-9-yl, 8-azaadenin-9-yl, guanin-9-yl, 2, 6-diaminopurin-9-yl, 2-aminopurin-9-yl, thymin-1-yl, cytosin-1-yl, 5-fluorocytosin-1-yl, 6-azacytosin-1-yl, 5-methylcytosin-1-yl, 5-bromovinyluracil-1-yl, 5-fluorouracil-1-yl or 5-trifluoromethyluracil-1-yl.

The substructure Z can have a range of atoms between the base, B, and the phosphorus atom. For example, four atoms separate the heterocyclic base and phosphorus moieties when Z is of the formula -CH₂-O-CH₂-CH₂-. In general, there will be from 2 to 16 atoms, preferably from 3 to 9 atoms, more preferably from 4 to 6 atoms that separate the heterocyclic base and the phosphorus atom.

The nucleotide analog amidate and ester compounds of the instant invention include the corresponding salts, which may be base salts of the phosphonic acid moiety or an acid addition salt of the base in addition to the zwitterionic forms and/or solvates of compounds of formula I.

Some of the compounds of the present invention can exist as optical isomers and both racemic or scalemic and diastereomeric mixtures of these isomers which may exist for certain compounds as well as the individual optical isomers which are all within the scope of the present invention. Compounds of formula IIa in the *R, S* or *RS* configuration at the chiral carbon, designated # herein, are examples of compounds having optical isomers. While the scalemic mixtures can be separated into their individual isomers through well-known techniques such as, for example, the separation of diastereomeric salts formed with optically active adjuncts, e.g. acids or bases followed by conversion back to the optically active substrates; in most instances, for compounds of the present invention, the preferred optical isomer can be synthesized by means of stereospecific reactions, beginning with the appropriate stereoisomer of the desired starting material.

As indicated, the present invention also pertains to the salts, including pharmaceutically acceptable non-toxic salts of these compounds. Such salts may include those derived by combination of appropriate cations such as alkali and alkaline earth metal ions or ammonium and quaternary amino ions with the acid anion moiety of the phosphonic acid group. In addition salts may be formed from acid addition of certain organic and inorganic acids with basic centers of the purine, specifically guanine, or pyrimidine base. Finally it is to be understood that compounds of the present invention in their un-ionized as well as zwitterionic form and/or in the form of solvates are also considered part of the present invention.

In other embodiments, the foregoing nucleotide analog amidates and esters or their dihydroxy phosphonate hydrolysis products are labeled with a detectable tag such as a radioisotope (including ³²P, ³⁵S, 14C, ³H, ¹²⁵I), a fluorescent moiety, an enzyme (including peroxidase, phosphatase) or the like.

In other embodiments, the foregoing nucleotide analog amidates comprise amino acid, dipeptide or tripeptide compounds (monosubstituted or disubstituted with identical or different amino acid, dipeptide or tripeptide substituents) that are capable of entry into eukaryotic cells via amino acid or peptide transporters present in eukaryotic cells *in vivo* or *in vitro.*

### Chemical Structures

Structural formulas and substructures are represented as roman numerals (I, II, III, IV, V, etc) or as letters (B, Z, L¹, L², R¹, R², etc). The substructure Z represents linking groups between the heterocyclic base (B) and the phosphorus atom (P) of the phosphonate group in the nucleotide analogs described herein. The heterocyclic base (B) is covalently linked to the unfilled valence on the right side of the structure and the phosphorus atom is linked to the unfilled valence on the left side.

### Detailed Description of the Invention

Amino Acid Residues. When groups L¹ or L² comprise an amino acid residue they comprise any naturally-occurring or synthetic amino acid residue, i.e., any moiety comprising at least one carboxyl and at least one amino residue directly linked by at least one carbon atom, typically a single (α) carbon atom. The nature and identity of the intervening structure located between the carboxyl and amino (amidate) groups can have a variety of structures including those described herein. All that is necessary is that the group have sufficient conformation and length to be capable of acid catalysis of the phosphoroamidate bond and release of the phosphonate when the free carboxyl is generated in vivo*.* e.g. by deesterification, deamidation or peptidolytic cleavage of the precursor. In general, the amino acids corresponding to the residues employed in the compounds of this invention are naturally occurring and have no pharmacological activity per se. However, optimal pharmacokinetic activity (substantially complete autocatalytic hydrolysis upon hydrolysis of the distal amide or ester bond) may be achieved by using non-naturally occurring amino acid residues. The intervening structure may be as simple as methylene (when the residue is glycyl) or substituted methylene (other α amino acids). The structure ordinarily contains up to about 5 carbon or hetero atoms in the direct linkage between the carboxyl carbon and the amidate nitrogen, as for example in the case of intervening ethylene, propylene, butylene, or pentylene groups or their substituted analogs, such as for example oxyesters in which ○ replaces carbon and, as appropriate, hydrogen. An example of such an intervening structure would be -CH-O-CH(R³)(R²)-. In general, fewer intervening atoms are employed when more rapid hydrolysis is desired, although it will be understood that larger structures are suitable if they possess sufficient flexibility or have conformations in which the carboxyl group is positioned in proximity to the amidate bond.

In general, the amino acid residue has the structure shown in formula III. Ordinarily, n is 1 or 2, R² is H and R³ is a moiety containing one or more of the following groups: amino, carboxyl, amide, carboxyl ester, hydroxyl, C₆-C₇ aryl, ether, n-, s- or t-alkyl (C₁ - C₆), guanidinyl, imidazolyl, indolyl, sulfhydryl, sulfoxide, and phosphoryl. The R² and R³ substituents can have a wide variety of structures including those disclosed herein.

Ordinarily R² is H and R³ is a side chain or group of a naturally occurring amino acid. With respect to the carboxyl-containing side chains it will be understood that if the C atom of the subject carboxyl is linked by 5 or less atoms to the phosphoamide N then the carboxyl optionally will be blocked, e.g. by esterification or amidation wherein the ester or amide bonds are hydrolyzable in vivo. R³ also is taken together with R¹ to form a proline residue (R³ = -CH₂-)₃) Thus, R³ is generally a side group such as H, -CH₃, -CH(CH₃)₂, -CH₂-CH(CH₃)₂, -CHCH₃-CH₂-CH₃, -CH₂-C₆H₅, -CH₂CH₂-S-CH₃, -CH₂OH, -CH(OH)-CH₃, -CH₂-SH, -CH₂-C₆H₄OH, -CH₂-CO-NH₂, -CH₂-CH₂-CO-NH₂, -CH₂-COOH, -CH₂-CH₂-COOH, -(CH₂)₄-NH₂ and -(CH₂)₃-NH-C(NH₂)-NH₂. R³ also includes 1-guanidinoprop-3-yl, benzyl, 4-hydroxybenzyl, imidazol-4-yl, indol-3-yl, methoxyphenyl and ethoxyphenyl. The optimal R³ group is readily selected using routine assays.

When the amino acid residues contain one or more chiral centers, any of the D, L, meso, threo or erythro (as appropriate) racemates, scalemates or mixtures thereof, fall within the scope of this invention. In general, if it is desired to rely on non-enzymatic means of hydrolysis, D isomers should be used. On the other hand, L isomers may be more versatile since they can be susceptible to both non-enzymatic as well as potential targeted enzymatic hydrolysis, and are more efficiently transported by amino acid or dipeptidyl transport systems in the gastrointestinal tract.

Examples of suitable amino acid residues include the following:
Glycyl;
Aminopolycarboxylic acids, e.g., aspartic acid, β-hydroxyaspartic acid, glutamic acid, β-hydroxyglutamic acid, β-methytaspartic acid, β-methylglutamic acid, β,β-dimethylaspartic acid, γ-hydroxyglutamic acid, β,γ-dihydroxyglutamic acid, β-phenylglutamic acid, γ-methyleneglutamic acid, 3-aminoadipic acid, 2-aminopimelic acid, 2-aminosuberic acid and 2-aminosebacic acid residues;
Amino acid amides such as glutaminyl and asparaginyl;
Polyamino- or polybasic-monocarboxylic acids such as arginine, lysine, β-aminoalanine, γ-aminobutyrine, ornithine, citruline, homoarginine, homocitrulline, 5-hydroxy-2,6-diaminohexanoic acid (commonly, hydroxylysine, including allohydroxylysine) and diaminobutyric acid residues;
Other basic amino acid residues such as histidinyl;
Diaminodicarboxylic acids such as α,α'-diaminosuccinic acid, α,α'-diaminoglutaric acid, α,α'-diaminoadipic acid, α,α'-diaminopimelic acid, α,α'-diamino-β-hydroxypimelic acid, α,α'-diaminosuberic acid, α,α'-diaminoazelaic acid, and α,α'-diaminosebacic acid residues;
Imino acids such as proline, 4- or 3-hydroxy-2-pyrrolidinecarboxylic acid (commonly, hydroxyproline, including allohydroxyproline), γ-methylproline, pipecolic acid, 5-hydroxypipecolic acid, -N([CH₂]ₙCOOR⁴)₂, wherein n and R⁴ are as defined above, and azetidine-2-carboxylic acid residues;
A mono- or di-alkyl (typically C₁ - C₈ branched or normal) amino acid such as alanine, valine, leucine, allylglycine, butyrine, norvaline, norleucine, heptyline, α-methylserine, α-amino-α-methyl-γ-hydroxyvaleric acid, α-amino-α-methyl-δ-hydroxyvaleric acid, α-amino-α-methyl-ε-hydroxycaproic acid, isovaline, α-methylglutamic acid, α-aminoisobutyric acid, α-aminodiethylacetic acid, α-aminodiisopropylacetic acid, α-aminodi-n-propylacetic acid, α-aminodiisobutylacetic acid, α-aminodi-n-butylacetic acid, α-aminoethylisopropylacetic acid, α-amino-n-propylacetic acid, α-aminodiisoamyacetic acid, α-methylaspartic acid, α-methylglutamic acid, 1-aminocyclopropane-1-carboxylic acid; isoleucine, alloisoleucine, tert-leucine, β-methyltryptophan and α-amino-β-ethyl-β-phenylpropionic acid residues; β-phenylserinyl;
Aliphatic α-amino-β-hydroxy acids such as serine, β-hydroxyleucine, β-hydroxynorleucine, β-hydroxynorvaline, and α-amino-β-hydroxystearic acid residues;
α-Amino, α-, γ-, δ- or ε-hydroxy acids such as homoserine, γ-hydroxynorvaline, δ-hydroxynorvaline and epsilon-hydroxynorleucine residues; canavinyl and canalinyl; γ-hydroxyornithinyl;
2-hexosaminic acids such as D-glucosaminic acid or D-galactosaminic acid residues;
α-Amino-β-thiols such as penicillamine, β-thiolnorvaline or β-thiolbutyrine residues;
Other sulfur containing amino acid residues including cysteine; homocystine; β-phenylmethionine; methionine; S-allyl-L-cysteine sulfoxide; 2-thiolhistidine; cystathionine; and thiol ethers of cysteine or homocysteine;
Phenylalanine, tryptophan and ring-substituted a amino acids such as the phenyl- or cyclohexylamino acids α-aminophenylacetic acid, α-aminocyclohexylacetic acid and α-amino-β-cyclohexylpropionic acid; phenylalanine analogues and derivatives comprising aryl, lower alkyl, hydroxy, guanidino, oxyalkylether, nitro, sulfur or halo-substituted phenyl (e.g., tyrosine, methyltyrosine and o-chloro-, p-chloro-, 3,4-dicloro, o-, m- or p-methyl-, 2,4,6-trimethyl-, 2-ethoxy-5-nitro, 2-hydroxy-5-nitro and p-nitro-phenylalanine); furyl-, thienyl-, pyridyl-, pyrimidinyl-, purine or naphthylalanines; and tryptophan analogues and derivatives including kynurenine, 3-hydroxykynurenine, 2-hydroxytryptophan and 4-carboxytryptophan residues;
α-Amino substituted amino acid residues including sarcosine (N-methylglycine), N-benzylglycine, N-methylalanine, N-benzylalanine, N-methylphenylalanine, N-benzylphenylalanine, N-methylvaline and N-benzylvaline; and
α-Hydroxy and substituted α-hydroxy amino acid residues including serine, threonine, allothreonine, phosphoserine and phosphothreonine residues.

Any one of the foregoing or other known amino acids are suitably employed in this invention provided that they are capable of autocatalytically hydrolyzing the amidate bond. Thus, they must contain, or must, upon being converted (hydrolyzed) in vivo, contain a free carboxyl group. In general, the amino acids corresponding to the residues employed in the compounds of this invention are naturally occurring and have no pharmacological activity. However, optimal pharmacokinetic activity may be achieved by the use of non-naturally occurring amino acid residues.

Of particular interest are hydrophobic residues such as mono-or di-alkyl or aryl amino acids, cycloalkylamino acids and the like. These residues, together with R⁴, contribute to cell permeability by increasing the partition coefficient of the nucleotide analog amidate. Typically, the residue does not contain a sulfhydryl or guarudino substituent. 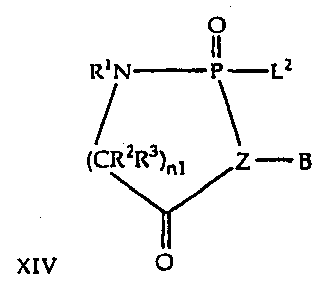 wherein L², R¹, R², R³, Z, n1 and B are as defined above. Typically, in this embodiment R³ is not carboxyl, R² is H, and n1 is 1.

Hydrolysis of the cyclic amidates of formulas IIa-c and IV leaves a hydroxyl-substituted substructure Z and the free carboxyl, which in turn will autolyze the amidate. Substructures Z in which the methylene backbone is substituted with hydroxymethyl are advantageous in this embodiment, particularly linkers in compounds of the formula -CH₂OCH(CH₂O-)CH₂-B.

Heterocyclic bases. The compounds of this invention comprise naturally-occurring heterocycle found in nucleic acids, nucleotides or nucleosides, or analogs thereof. The radicals of such heterocyclic bases, designated herein as B, are generally the purine, pyrimidine or related heterocycles.

B includes both protected and unprotected forms of the heterocyclic bases. Protecting groups for exocyclic amines and other groups are known (Greene and include N-benzoyl, isobutyryl, 4,4'-dimethoxytrityl (DMT) and the like. The selection of a protecting group will be apparent to the ordinary artisan and will depend on the nature of the labile group and the chemistry

The invention compounds are optionally alkylated at the α-nitrogen atom of the amino acid by the R¹ group defined above. Exemplary R¹ groups include H, CH₃, CH₂CH₃, benzyl.

The invention compounds are optionally esterified at the amino acid carboxyl moiety by the R⁴ group defined above. Exemplary R⁴ groups include H, methyl, ethyl, propyl, isopropyl, butyl, t-butyl (C(CH₃)₃), phenyl (-C₆H₅), benzyl (-CH₂-C₆H₅), 1-pyridyl, 3-pyridyl, 1-pyrimidinyl, N-ethylmorpholino ( -CH₂-CH₂-N[(CH₂)₂(CH₂)₂]O), N-2-propylmorpholino (-CH(CH₃)-CH₂-N[(CH₂)₂(CH₂)₂]O), methoxyethyl (-CH₂-CH₂-CH₃), 4-N-methylpiperidyl (-CH[(CH₂)₂(CH₂)₂]N(CH₃)), 3-N-methylpiperidyl, 2-, 3-, and 4-N,N-dimethylaminophenol and 2-, 3-, and 4-N,N-diethylaminophenol), 1-ethylpiperazinyl.

As used herein, and unless modified by the immediate context: 1) the term alkyl, alkenyl and alkynyl refer to straight chain, branched and cyclic residues. Thus, C₁-C₄ alkyl includes methyl, ethyl, propyl, cyclopropyl, isopropyl, n-, sec-, iso- and tert-butyl, cyclobutyl and the like while alkenyl includes ethenyl, propenyl, isopropenyl, 1-, 2- and 3-butenyl, 1- and 2-isobutenyl and the like. The term alkyl also includes cyclic N-, S- or O-heterocarbonyl (such as piperidyl and morpholino). 2) The term aryl includes N-, S- or O- heteroaryl, including phenyl; 2- and 3-pyrrolyl, 2- and 3-thienyl, 2- and 4-imidazolyl, 2-, 4- and 5-oxazolyl, 3- and 4-isoxazolyl, 2-, 4- and 5-thiazolyl, 3-, 4- and 5-isothiazolyl, 3- and 4-pyrazolyl, 2-, 3- and 4-pyridinyl, 2-, 4- and 5-pyrimidinyl. When "O" or "N" are substituted into aryl or alkyl this means that a ring or chain methyne or methylene is replaced by O, N or NH as the case may be. The term acyl means R^{X}-C(O)-, acyloxy means R^{X}-C(O)-O-, acyloxymethyl means R^{X}-C(O)-O-CH₂- and thus, for example, C₃₋₆ acyloxymethyl means R^{X}-C(O)-O-CH₂- wherein R^{X} is a 1 to 4 carbon alkyl or aryl group (substituted or unsubstituted).

Nucleoside Phosphonates. Table 1 lists a group of exemplary nucleotide analogs of formula I having the structure (L¹)(L²)P(O)-Z-B. These compounds generally have L¹ and L² groups that, when amino acids, are identical, although one of the amino acid groups can be different or replaced by another hydrolyzable group such as -O-CH₂-O-C(O)-C(CH₃)₃ or -O-C₆H₅ as listed below.

**TABLE 1**

| **L1, L^{2*}** | **-Z-B**** |
|---|---|
| 1 -NH-CH₂-C(O)-OR⁴ | 1 -CH₂-O-CH₂-CH₂-B |
| 2 -NH-CH(CH₃)-C(O)-OR⁴ | 2 -CH₂-O-C#H(CH₂-OR⁴)-CH₂-B- |
| 3 -NH-CH(CH₃)₂-C(O)-OR⁴ | 3 -CH₂-O-C#H(CH₃)-CH₂-B |
| 4 -NH-CH(CH(CH₃)₂)-C(O)-OR₄ | 4 -CH₂-O-C#H(CH₂F)-CH₂-B |
| 5 -NH-CH(CH₃)(CH₃)₂-C(O)-OR⁴ | 5 -CH₂-O-C#H(CH=CH₂)-CH₂-B |
| 6 -NH-CH₂-CH₂-CH₂-CH-C(O)-OR⁴ | 6 -CH₂-O-C#H(CH₂N₃)-CH₂-B |
| 7 -NH-CH(CH₂-C₆H₅)C(O)-OR⁷ | 7 *** |
| 8 -NH-CH(CH₂-C₈NH₆)-C(O)-OR⁴ | 8 **** |
| 9 -NH-CH(CH₂-CH₂-S-CH₃)-C(O)-OR⁴ | |
| 10 -NH-CH(CH₂OH)-C(O)-OR⁴ | |
| 11 -NH-CH(CH(OH)(CH₃)-C(O)-OR⁴ | |
| 12 -NH-CH(-CH₂SH)-C(O)-OR⁴ | |
| 13 -NH-CH(CH₂-C₆H₅OH)-C(O)OR⁴ | |
| 14 -NH-CH(CH₂-C(O)-NH₂)-C(O)-OR⁴ | |
| 15 -NH-CH(CH₂-CH₂-C(O)-NH₂)-C(O)-OR⁴ | |
| 16 -NH-CH(CH₂C(O)OR⁴)-C(O)-OR⁴ | |
| 17 -NH-CH(CH₂CH₂C(O)OR⁴)-C(O)-OR⁴ | |
| 18 -NH-CH(CH₂CH₂CH₂CH₂NH₂)-C(O)-OR⁴ | |
| 19 -NH-CH(CH₂CH₂CH₂NHC(NH)(NH₂))-C(O)-OR⁴ | |
| 20 -NH-CH(CH₂C₃N₂H₃)-C(O)-OR⁴ | |
| 21 -NH-CH(CH₃)₂-CH₂-C(O)-OR⁴ | |
| 22 -NH-CH₂-CH₂-C(O)-OR⁴ | |
| 23 -NH-CH(CH₂-C₆H₅)-CH₂-C(O)-OR⁴ | |
| 24 -NH-CH(CH₂CH₂CH₂NH₂)-CH₂-C(O)-OR⁴ | |
| 25 -NH-CH(CH₂CH₂CH₂CH₂NH₂)-CH₂-C(O)OR⁴ | |
| 26 -NH-CH(CH₂CH₂NHC(NH)(NH₂))-CH₂-C(O)-OR⁴ | |
| 27 -NH-CH(C(O)OR⁴)-CH₂-C(O)-OR⁴ | |
| 28 -NH-CH(CH₂C(O)OR⁴)-CH₂-C(O)-OR⁴ | |
| 29 -NH-CH(CH₂CH₂C(O)OR⁴)-CH₂-C(O)-OR⁴ | |
| 30 -N(CH₃)-CH₂-C(O)-OR⁴ | |
| 31 -NHR⁶ | |
| 32 -O-CH₂-CH₂-N[CH₂)₂(CH₂)₂]O | |
| 33 -O-CH₂-O-C(O)-C(CH₃)₃ | |
| 34 -O-CH₂-O-C(O)-CH(CH₃)₂ | |
| 35 -O-CH₂-O-C(O)-CH₂C₆H₄-O-CH₂CH₃ | |
| 36 -O-CH₂-O-C(O)-C₁₀H₁₅ | |
| 37 -O-CH₂-C₆H₅ | |
| 38 -O-C₆H₅ | |
| 39 -O-CH₂-C₆H₄N(CH₃)₂ | |
| 40 -OH | |
| | |
| **B** | |
| 1 adenin-9-yl | |
| 2 guanin-9-yl | |
| 3 cytosin-1-yl | |
| 4 2, 6-diaminopurin-9-yl | |
| 5 2-aminopurin-9-yl | |
| 6 6-azacytosin-1-yl | |
| 7 1-deazaadenin-9-yl | |
| 8 3-deazaadenin-9-yl | |
| 9 8-azaadenin-9-yl | |
| 10 7-deaza-8-azaadenin-9-yl | |

| | |
|---|---|
| * - R⁴ includes H, propyl, isopropyl, t-butyl, phenyl, benzyl, 1-pyridinyl, 1-pyrimidinyl, N-ethylmorpholino, methoxyethyl, 4-hydroxy-N-methylpiperidinyl, 3-hydroxy-N-methylpiperidinyl, 1-ethylpiperazinyl; atoms with unfilled valences are linked to each other. ** - The carbon atom on the left of each structure is attached to the phosphorus atom; ^{#} - carbon atom having linked substituents in the *R, S* or *RS* configuration. *** - Z-B substructure 7 is of formula V where R²⁵ and R²⁹ are O and B is thymin-1-yl (base 11) or one of the heterocyclic bases listed (1-10). **** - Z-B substructure 8 is of formula IV where R²⁵ and R²⁹ are O, R²⁶ is S, R²⁷ is absent, R²⁸ is H and B is thymin-1-yl (base 11) or one of the heterocyclic bases listed (1-10) and includes the (+) and (-) enantiomers. | |

Compounds listed in Table 1 are designated herein by numbers assigned to L¹, L², Z and B according to the following convention, L¹.L².Z.B. Thus, compound 1.2.1.1, where R⁴ is benzyl, represents L¹ structure 1 (-NH-CH₂-C(O)-O-CH₂-C₆H₅), L² structure 2 (-NH-CH(CH₃)C(O)-O-CH₂-C₆H₅), Z structure 1 (-CH₂-O-CH₂-CH₂-) and B structure 1 (adenin-9-yl). This compound would have the structure 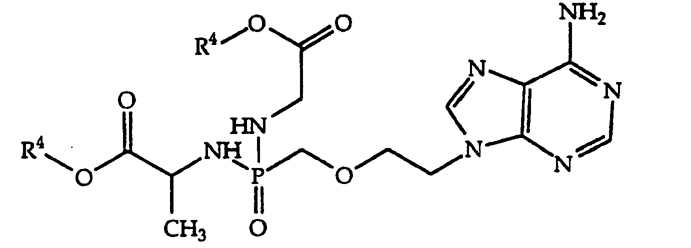 which corresponds to the compound designated herein bis(alanyl benzyl ester)PMEA. Similarly, for the compound 7.7.1.1, L¹ structure 7 (NH-CH(CH₂-C₆H₅)-C(O)-OR⁴), L² structure 7 (NH-CH(CH₂-C₆H₅)-C(O)-OR⁴), Z structure 2 (-CH₂-O-CH₂-CH₂-) and B structure 1 (adenin-9-yl) would have, when R⁴ is methyl, the structure 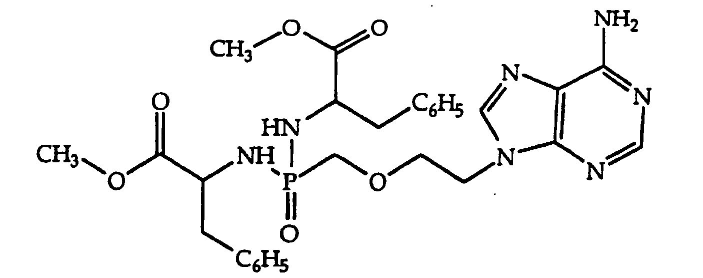 and would represent the compound designated herein bis(phenylalanyl methyl ester)PMEA. Exemplary compounds include 1.1.1.1, 2.1.1.1, 3.1.1.1, 4.1.1.1, 5.1.1.1, 6.1.1.1, 7.1.1.1, 8.1.1.1, 9.1.1.1, 10.1.1.1, 11.1.1.1, 12.1.1.1, 13.1.1.1, 14.1.1.1, 15.1.1.1, 16.1.1.1, 17.1.1.1, 18.1.1.1, 19.1.1.1, 20.1.1.1, 21.1.1.1, 22.1.1.1, 23.1.1.1, 24.1.1.1, 25.1.1.1, 26.1.1.1, 27.1.1.1, 28.1.1.1, 29.1.1.1, 30.1.1.1, 31.1.1.1, 32.1.1.1, 33.1.1.1, 34.1.1.1, 35.1.1.1, 36.1.1.1, 37.1.1.1, 38.1.1.1, 39.1.1.1, 40.1.1.1, 1.2.1.1, 2.2.1.1, 3.2.1.1, 4.2.1.1, 5.2.1.1, 6.2.1.1, 7.2.1.1, 8.2.1.1, 9.2.1.1, 10.2.1.1, 11.2.1.1, 12.2.1.1, 13.2.1.1, 14.2.1.1, 15.2.1.1, 16.2.1.1, 17.2.1.1, 18.2.1.1, 19.2.1.1, 20.2.1.1, 21.2.1.1, 22.2.1.1, 23.2.1.1, 24.2.1.1, 25.2.1.1, 26.2.1.1, 27.2.1.1, 28.2.1.1, 29.2.1.1, 30.2.1.1, 31.2.1.1, 32.2.1.1, 33.2.1.1, 34.2.1.1, 35.2.1.1, 36.2.1.1, 37.2.1.1, 38.2.1.1, 39.2.1.1, 40.2.1.1, 1.3.1.1, 2.3.1.1, 3.3.1.1, 4.3.1.1, 5.3.1.1, 6.3.1.1, 7.3.1.1, 8.3.1.1, 9.3.1.1, 10.3.1.1, 11.3.1.1, 12.3.1.1, 13.3.1.1, 14.3.1.1, 15.3.1.1, 16.3.1.1, 17.3.1.1, 18.3.1.1, 19.3.1.1, 20.3.1.1, 21.3.1.1, 22.3.1.1, 23.3.1.1, 24.3.1.1, 25.3.1.1, 26.3.1.1, 27.3.1.1, 28.3.1.1, 29.3.1.1, 30.3.1.1, 31.3.1.1, 32.3.1.1, 33.3.1.1, 34.3.1.1, 35.3.1.1, 36.3.1.1, 37.3.1.1, 38.3.1.1, 39.3.1.1, 40.3.1.1, 1.4.1.1, 2.4.1.1, 3.4.1.1, 4.4.1.1, 5.4.1.1, 6.4.1.1, 7.4.1.1, 8.4.1.1, 9.4.1.1, 10.4.1.1, 11.4.1.1, 12.4.1.1, 13.4.1.1, 14.4.1.1, 15.4.1.1, 16.4.1.1, 17.4.1.1, 18.4.1.1, 19.4.1.1, 20.4.1.1, 21.4.1.1, 22.4.1.1, 23.4.1.1, 24.4.1.1, 25.4.1.1, 26.4.1.1, 27.4.1.1, 28.4.1.1, 29.4.1.1, 30.4.1.1, 31.4.1.1, 32.4.1.1, 33.4.1.1, 34.4.1.1, 35.4.1.1, 36.4.1.1, 37.4.1.1, 38.4.1.1, 39.4.1.1, 40.4.1.1, 1.5.1.1, 2.5.1.1, 3.5.1.1, 4.5.1.1, 5.5.1.1, 6.5.1.1, 7.5.1.1, 8.5.1.1, 9.5.1.1, 10.5.1.1, 11.5.1.1, 12.5.1.1, 13.5.1.1, 14.5.1.1, 15.5.1.1, 16.5.1.1, 17.5.1.1, 18.5.1.1, 19.5.1.1, 20.5.1.1, 21.5.1.1, 22.5.1.1, 23.5.1.1, 24.5.1.1, 25.5.1.1, 26.5.1.1, 27.5.1.1, 28.5.1.1, 29.5.1.1, 30.5.1.1, 31.5.1.1, 32.5.1.1, 33.5.1.1, 34.5.1.1, 35.5.1.1, 36.5.1.1, 37.5.1.1, 38.5.1.1, 39.5.1.1, 40.5.1.1, 1.6.1.1, 2.6.1.1, 3.6.1.1, 4.6.1.1, 5.6.1.1, 6.6.1.1, 7.6.1.1, 8.6.1.1, 9.6.1.1, 10.6.1.1, 11.6.1.1, 12.6.1.1, 13.6.1.1, 14.6.1.1, 15.6.1.1, 16.6.1.1, 17.6.1.1, 18.6.1.1, 19.6.1.1, 20.6.1.1, 21.6.1.1, 22.6.1.1, 23.6.1.1, 24.6.1.1, 25.6.1.1, 26.6.1.1, 27.6.1.1, 28.6.1.1, 29.6.1.1, 30.6.1.1, 31.6.1.1, 32.6.1.1, 33.6.1.1, 34.6.1.1, 35.6.1.1, 36.6.1.1, 37.6.1.1, 38.6.1.1, 39.6.1.1, 40.6.1.1, 1.7.1.1, 2.7.1.1, 3.7.1.1, 4.7.1.1, 5.7.1.1, 6.7.1.1, 7.7.1.1, 8.7.1.1, 9.7.1.1, 10.7.1.1, 11.7.1.1,12.7.1.1,13.7.1.1,14.7.1.1,15.7.1.1, 16.7.1.1, 17.7.1.1, 18.7.1.1, 19.7.1.1, 20.7.1.1, 21.7.1.1, 22.7.1.1, 23.7.1.1, 24.7.1.1, 25.7.1.1, 26.7.1.1, 27.7.1.1, 28.7.1.1, 29.7.1.1, 30.7.1.1, 31.7.1.1, 32.7.1.1, 33.7.1.1, 34.7.1.1, 35.7.1.1, 36.7.1.1, 37.7.1.1, 38.7.1.1, 39.7.1.1, 40.7.1.1, 1.8.1.1, 2.8.1.1, 3.8.1.1, 4.8.1.1, 5.8.1.1, 6.8.1.1, 7.8.1.1, 8.8.1.1, 9.8.1.1, 10.8.1.1, 11.8.1.1, 12.8.1.1, 13.8.1.1, 14.8.1.1, 15.8.1.1, 16.8.1.1, 17.8.1.1, 18.8.1.1, 19.8.1.1, 20.8.1.1, 21.8.1.1, 22.8.1.1, 23.8.1.1, 24.8.1.1, Z5.8.1.1, 26.8.1.1, 27.8.1.1, 28.8.1.1, 29.8.1.1, 30.8.1.1, 31.8.1.1, 32.8.1.1, 33.8.1.1, 34.8.1.1, 35.8.1.1, 36.8.1.1, 37.8.1.1, 38.8.1.1, 39.8.1.1, 40.8.1.1, 1.9.1.1, 2.9.1.1, 3.9.1.1, 4.9.1.1, 5.9.1.1, 6.9.1.1, 7.9.1.1, 8.9.1.1, 9.9.1.1, 10.9.1.1, 11.9.1.1, 12.9.1.1, 13.9.1.1, 14.9.1.1, 15.9.1.1, 16.9.1.1, 17.9.1.1, 18.9.1.1, 19.9.1.1, 20.9.1.1, 21.9.1.1, 22.9.1.1, 23.9.1.1, 24.9.1.1, 25.9.1.1, 26.9.1.1, 27.9.1.1, 28.9.1.1, 29.9.1.1, 30.9.1.1, 31.9.1.1, 32.9.1.1, 33.9.1.1, 34.9.1.1, 35.9.1.1, 36.9.1.1, 37.9.1.1, 38.9.1.1, 39.9.1.1, 40.9.1.1, 1.10.1.1, 2.10.1.1, 3.10.1.1, 4.10.1.1, 5.10.1.1, 6.10.1.1, 7.10.1.1, 8.10.1.1, 9.10.1.1, 10.10.1.1, 11.10.1.1, 12.10.1.1, 13.10.1.1, 14.10.1.1, 15.10.1.1, 16.10.1.1, 17.10.1.1, 18.10.1.1, 19.10.1.1, 20.10.1.1, 21.10.1.1, 22.10.1.1, 23.10.1.1, 24.10.1.1, 25.10.1.1, 26.10.1.1, 27.10.1.1, 28.10.1.1, 29.10.1.1, 30.10.1.1, 31.10.1.1, 32.10.1.1, 33.10.1.1, 34.10.1.1, 35.10.1.1, 36.10.1.1, 37.10.1.1, 38.10.1.1, 39.10.1.1, 40.10.1.1, 1.11.1.1, 2.11.1.1, 3.11.1.1, 4.11.1.1, 5.11.1.1, 6.11.1.1, 7.11.1.1, 8.11.1.1, 9.11.1.1, 10.11.1.1, 11.11.1.1, 12.11.1.1, 13.11.1.1, 14.11.1.1, 15.11.1.1, 16.11.1.1, 17.11.1.1, 18.11.1.1, 19.11.1.1, 20.11.1.1, 21.11.1.1, 22.11.1.1, 23.11.1.1, 24.11.1.1, 25.11.1.1, 26.11.1.1, 27.11.1.1, 28.11.1.1, 29.11.1.1, 30.11.1.1, 31.11.1.1, 32.11.1.1, 33.11.1.1, 34.11.1.1, 35.11.1.1, 36.11.1.1, 37.11.1.1, 38.11.1.1, 39.11.1.1, 40.11.1.1, 1.12.1.1, 2.12.1.1, 3.12.1.1, 4.12.1.1, 5.12.1.1, 6.12.1.1, 7.12.1.1, 8.12.1.1, 9.12.1.1, 10.12.1.1, 11.12.1.1, 12.12.1.1, 13.12.1.1, 14.12.1.1, 15.12.1.1, 16.12.1.1, 17.12.1.1, 18.12.1.1, 19.12.1.1, 20.12.1.1, 21.12.1.1, 22.12.1.1, 23.12.1.1, 24.12.1.1, 25.12.1.1, 26.12.1.1, 27.12.1.1, 28.12.1.1, 29.12.1.1, 30.12.1.1, 31.12.1.1, 32.12.1.1, 33.12.1.1, 34.12.1.1, 35.12.1.1, 36.12.1.1, 37.12.1.1, 38.12.1.1, 39.12.1.1, 40.12.1.1, 1.13.1.1, 2.13.1.1, 3.13.1.1, 4.13.1.1, 5.13.1.1, 6.13.1.1, 7.13.1.1, 8.13.1.1, 9.13.1.1, 10.13.1.1, 11.13.1.1, 12.13.1.1, 13.13.1.1, 14.13.1.1, 15.13.1.1, 16.13.1.1, 17.13.1.1, 18.13.1.1, 19.13.1.1, 20.13.1.1, 21.13.1.1, 22.13.1.1, 23.13.1.1, 24.13.1.1, 25.13.1.1, 26.13.1.1, 27.13.1.1, 28.13.1.1, 29.13.1.1, 30.13.1.1, 31.13.1.1, 32.13.1.1, 33.13.1.1, 34.13.1.1, 35.13.1.1, 36.13.1.1, 37.13.1.1, 38.13.1.1, 39.13.1.1, 40.13.1.1,1.14.1.1, 2.14.1.1, 3.14.1.1, 4.14.1.1, 5.14.1.1, 6.14.1.1, 7.14.1.1, 8.14.1.1, 9.14.1.1, 10.14.1.1, 11.14.1.1, 12.14.1.1, 13.14.1.1, 14.14.1.1, 15.14.1.1, 16.14.1.1, 17.14.1.1, 18.14.1.1, 19.14.1.1, 20.14.1.1, 21.14.1.1, 22.14.1.1, 23.14.1.1, 24.14.1.1, 25.14.1.1, 26.14.1.1, 27.14.1.1, 28.14.1.1, 29.14.1.1, 30.14.1.1, 31.14.1.1, 32.14.1.1, 33.14.1.1, 34.14.1.1, 35.14.1.1, 36.14.1.1, 37.14.1.1, 38.14.1.1, 39.14.1.1, 40.14.1.1, 1.15.1.1, 2.15.1.1, 3.15.1.1, 4.15.1.1, 5.15.1.1, 6.15.1.1, 7.15.1.1, 8.15.1.1, 9.15.1.1, 10.15.1.1, 11.15.1.1, 12.15.1.1, 13.15.1.1, 14.15.1.1, 15.15.1.1, 16.15.1.1, 17.15.1.1, 18.15.1.1, 19.15.1.1, 20.15.1.1, 21.15.1.1, 22.15.1.1, 23.15.1.1, 24.15.1.1, 25.15.1.1, 26.15.1.1, 27.15.1.1, 28.15.1.1, 29.15.1.1, 30.15.1.1, 31.15.1.1, 32.15.1.1, 33.15.1.1, 34.15.1.1, 35.15.1.1, 36.15.1.1, 37.15.1.1, 38.15.1.1, 39.15.1.1, 40.15.1.1, 1.16.1.1, 2.16.1.1, 3.16.1.1, 4.16.1.1, 5.16.1.1, 6.16.1.1, 7.16.1.1, 8.16.1.1, 9.16.1.1, 10.16.1.1, 11.16.1.1, 12.16.1.1, 13.16.1.1, 14.16.1.1, 15.16.1.1, 16.16.1.1, 17.16.1.1, 18.16.1.1, 19.16.1.1, 20.16.1.1, 21.16.1.1, 22.16.1.1, 23.16.1.1, 24.16.1.1, 25.16.1.1, 26.16.1.1, 27.16.1.1, 28.16.1.1, 29.16.1.1, 30.16.1.1, 31.16.1.1, 32.16.1.1, 33.16.1.1, 34.16.1.1, 35.16.1.1, 36.16.1.1, 37.16.1.1, 38.16.1.1, 39.16.1.1, 40.16.1.1, 1.17.1.1, 2.17.1.1, 3.17.1.1, 4.17.1.1, 5.17.1.1, 6.17.1.1, 7.17.1.1, 8.17.1.1, 9.17.1.1, 10.17.1.1, 11.17.1.1, 12.17.1.1, 13.17.1.1, 14.17.1.1, 15.17.1.1, 16.17.1.1, 17.17.1.1, 18.17.1.1, 19.17.1.1, 20.17.1.1, 21.17.1.1, 22.17.1.1, 23.17.1.1, 24.17.1.1, 25.17.1.1, 26.17.1.1, 27.17.1.1, 28.17.1.1, 29.17.1.1, 30.17.1.1, 31.17.1.1, 32.17.1.1, 33.17.1.1, 34.17.1.1, 35.17.1.1, 36.17.1.1, 37.17.1.1, 38.17.1.1, 39.17.1.1, 40.17.1.1, 1.18.1.1, 2.18.1.1, 3.18.1.1, 4.18.1.1, 5.18.1.1, 6.18.1.1, 7.18.1.1, 8.18.1.1, 9.18.1.1, 10.18.1.1, 11.18.1.1, 12.18.1.1, 13.18.1.1, 14.18.1.1, 15.18.1.1, 16.18.1.1, 17.18.1.1, 18.18.1.1, 19.18.1.1, 20.18.1.1, 21.18.1.1, 22.18.1.1, 23.18.1.1, 24.18.1.1, 25.18.1.1, 26.18.1.1, 27.18.1.1, 28.18.1.1, 29.18.1.1, 30.18.1.1, 31.18.1.1, 32.18.1.1, 33.18.1.1, 34.18.1.1, 35.18.1.1, 36.18.1.1, 37.18.1.1, 38.18.1.1, 39.18.1.1, 40.18.1.1, 1.19.1.1, 2.19.1.1, 3.19.1.1, 4.19.1.1, 5.19.1.1, 6.19.1.1, 7.19.1.1, 8.19.1.1, 9.19.1.1, 10.19.1.1, 11.19.1.1, 12.19.1.1, 13.19.1.1, 14.19.1.1, 15.19.1.1,16.19.1.1, 17.19.1.1, 18.19.1.1, 19.19.1.1, 20.19.1.1, 21.19.1.1, 22.19.1.1, 23.19.1.1, 24.19.1.1, 25.19.1.1, 26.19.1.1, 27.19.1.1, 28.19.1.1, 29.19.1.1, 30.19.1.1, 31.19.1.1, 32.19.1.1, 33.19.1.1, 34.19.1.1, 35.19.1.1, 36.19.1.1, 37.19.1.1, 38.19.1.1, 39.19.1.1, 40.19.1.1, 1.20.1.1, 2.20.1.1, 3.20.1.1, 4.20.1.1, 5.20.1.1, 6.20.1.1, 7.20.1.1, 8.20.1.1, 9.20.1.1, 10.20.1.1, 11.20.1.1, 12.20.1.1, 13.20.1.1, 14.20.1.1, 15.20.1.1, 16.20.1.1, 17.20.1.1, 18.20.1.1, 19.20.1.1, 20.20.1.1, 21.20.1.1, 22.20.1.1, 23.20.1.1, 24.20.1.1, 25.20.1.1, 26.20.1.1, 27.20.1.1, 28.20.1.1, 29.20.1.1, 30.20.1.1, 31.20.1.1, 32.20.1.1, 33.20.1.1, 34.20.1.1, 35.20.1.1, 36.20.1.1, 37.20.1.1, 38.20.1.1, 39.20.1.1, 40.20.1.1,1.21.1.1, 2.21.1.1, 3.21.1.1, 4.21.1.1, 5.21.1.1, 6.21.1.1, 7.21.1.1, 8.21.1.1, 9.21.1.1, 10.21.1.1, 11.21.1.1, 12.21.1.1, 13.21.1.1, 14.21.1.1, 15.21.1.1, 16.21.1.1, 17.21.1.1, 18.21.1.1, 19.21.1.1, 20.21.1.1, 21.21.1.1, 22.21.1.1, 23.21.1.1, 24.21.1.1, 25.21.1.1, 26.21.1.1, 27.21.1.1, 28.21.1.1, 29.21.1.1, 30.21.1.1, 31.21.1.1, 32.21.1.1, 33.21.1.1, 34.21.1.1, 35.21.1.1, 36.21.1.1, 37.21.1.1, 38.21.1.1, 39.21.1.1, 40.21.1.1, 1.22.1.1, 2.22.1.1, 3.22.1.1, 4.22.1.1, 5.22.1.1, 6.22.1.1, 7.22.1.1, 8.22.1.1, 9.22.1.1, 10.22.1.1, 11.22.1.1, 12.22.1.1, 13.22.1.1, 14.22.1.1, 15.22.1.1, 16.22.1.1, 17.22.1.1, 18.22.1.1, 19.22.1.1, 20.22.1.1, 21.22.1.1, 22.22.1.1, 23.22.1.1, 24.22.1.1, 25.22.1.1, 26.22.1.1, 27.22.1.1, 28.22.1.1, 29.22.1.1, 30.22.1.1, 31.22.1.1, 32.22.1.1, 33.22.1.1, 34.22.1.1, 35.22.1.1, 36.22.1.1, 37.22.1.1, 38.22.1.1, 39.22.1.1, 40.22.1.1, 1.23.1.1, 2.23.1.1, 3.23.1.1, 4.23.1.1, 5.23.1.1, 6.23.1.1, 7.23.1.1, 8.23.1.1, 9.23.1.1, 10.23.1.1, 11.23.1.1, 12.23.1.1, 13.23.1.1, 14.23.1.1, 15.23.1.1, 16.23.1.1, 17.23.1.1, 18.23.1.1, 19.23.1.1, 20.23.1.1, 21.23.1.1, 22.23.1.1, 23.23.1.1, 24.23.1.1, 25.23.1.1, 26.23.1.1, 27.23.1.1, 28.23.1.1, 29.23.1.1, 30.23.1.1, 31.23.1.1, 32.23.1.1, 33.23.1.1, 34.23.1.1, 35.23.1.1, 36.23.1.1, 37.23.1.1, 38.23.1.1, 39.23.1.1, 40.23.1.1, 1.24.1.1, 2.24.1.1, 3.24.1.1, 4.24.1.1, 5.24.1.1, 6.24.1.1, 7.24.1.1, 8.24.1.1, 9.24.1.1, 10.24.1.1, 11.24.1.1, 12.24.1.1, 13.24.1.1, 14.24.1.1, 15.24.1.1, 16.24.1.1, 17.24.1.1, 18.24.1.1, 19.24.1.1, 20.24.1.1, 21.24.1.1, 22.24.1.1, 23.24.1.1, 24.24.1.1, 25.24.1.1, 26.24.1.1, 27.24.1.1, 28.24.1.1, 29.24.1.1, 30.24.1.1, 31.24.1.1, 32.24.1.1, 33.24.1.1, 34.24.1.1, 35.24.1.1, 36.24.1.1, 37.24.1.1, 38.24.1.1, 39.24.1.1, 40.24.1.1, 1.25.1.1, 2.25.1.1, 3.25.1.1, 4.25.1.1, 5.25.1.1, 6.25.1.1, 7.25.1.1, 8.25.1.1, 9.25.1.1, 10.25.1.1, 11.25.1.1, 12.25.1.1, 13.25.1.1, 14.25.1.1, 15.25.1.1, 16.25.1.1, 17.25.1.1, 18.25.1.1, 19.25.1.1, 20.25.1.1, 21.25.1.1, 22.25.1.1, 23.25.1.1, 24.25.1.1, 25.25.1.1, 26.25.1.1, 27.25.1.1, 28.25.1.1, 29.25.1.1, 30.25.1.1, 31.25.1.1, 32.25.1.1, 33.25.1.1, 34.25.1.1, 35.25.1.1, 36.25.1.1, 37.25.1.1, 38.25.1.1, 39.25.1.1, 40.25.1.1, 1.26.1.1, 2.26.1.1, 3.26.1.1, 4.26.1.1, 5.26.1.1, 6.26.1.1, 7.26.1.1, 8.26.1.1, 9.26.1.1, 10.26.1.1, 11.26.1.1, 12.26.1.1, 13.26.1.1, 14.26.1.1, 15.26.1.1, 16.26.1.1, 17.26.1.1, 18.26.1.1, 19.26.1.1, 20.26.1.1, 21.26.1.1, 22.26.1.1, 23.26.1.1, 24.26.1.1, 25.26.1.1, 26.26.1.1, 27.26.1.1, 28.26.1.1, 29.26.1.1, 30.26.1.1, 31.26.1.1, 32.26.1.1, 33.26.1.1, 34.26.1.1, 35.26.1.1, 36.26.1.1, 37.26.1.1, 38.26.1.1, 39.26.1.1, 40.26.1.1, 1.27.1.1, 2.27.1.1, 3.27.1.1, 4.27.1.1, 5.27.1.1, 6.27.1.1, 7.27.1.1, 8.27.1.1, 9.27.1.1, 10.27.1.1, 11.27.1.1, 12.27.1.1, 13.27.1.1, 14.27.1.1, 15.27.1.1, 16.27.1.1, 17.27.1.1, 18.27.1.1, 19.27.1.1, 20.27.1.1, 21.27.1.1, 22.27.1.1, 23.27.1.1, 24.27.1.1, 25.27.1.1, 26.27.1.1, 27.27.1.1, 28.27.1.1, 29.27.1.1, 30.27.1.1, 31.27.1.1, 32.27.1.1, 33.27.1.1, 34.27.1.1, 35.27.1.1, 36.27.1.1, 37.27.1.1, 38.27.1.1, 39.27.1.1, 40.27.1.1,1.28.1.1, 2.28.1.1, 3.28.1.1, 4.28.1.1, 5.28.1.1, 6.28.1.1, 7.28.1.1, 8.28.1.1, 9.28.1.1,10.28.1.1, 11.28.1.1, 12.28.1.1, 13.28.1.1, 14.28.1.1, 15.28.1.1, 16.28.1.1, 17.28.1.1, 18.28.1.1, 19.28.1.1, 20.28.1.1, 21.28.1.1, 22.28.1.1, 23.28.1.1, 24.28.1.1, 25.28.1.1, 26.28.1.1, 27.28.1.1, 28.28.1.1, 29.28.1.1, 30.28.1.1, 31.28.1.1, 32.28.1.1, 33.28.1.1, 34.28.1.1, 35.28.1.1, 36.28.1.1, 37.28.1.1, 38.28.1.1, 39.28.1.1, 40.28.1.1, 1.29.1.1, 2.29.1.1, 3.29.1.1, 4.29.1.1, 5.29.1.1, 6.29.1.1, 7.29.1.1, 8.29.1.1, 9.29.1.1, 10.29.1.1, 11.29.1.1, 12.29.1.1, 13.29.1.1, 14.29.1.1, 15.29.1.1, 16.29.1.1, 17.29.1.1, 18.29.1.1, 19.29.1.1, 20.29.1.1, 21.29.1.1, 22.29.1.1, 23.29.1.1, 24.29.1.1, 25.29.1.1, 26.29.1.1, 27.29.1.1, 28.29.1.1, 29.29.1.1, 30.29.1.1, 31.29.1.1, 32.29.1.1, 33.29.1.1, 34.29.1.1, 35.29.1.1, 36.29.1.1, 37.29.1.1, 38.29.1.1, 39.29.1.1, 40.29.1.1, 1.1.1.2, 2.1.1.2, 3.1.1.2, 4.1.1.2, 5.1.1.2, 6.1.1.2, 7.1.1.2, 8.1.1.2, 9.1.1.2, 10.1.12, 11.1.1.2, 12.1.1.2, 13.1.1.2, 14.1.1.2, 15.1.1.2, 16.1.1.2, 17.1.1.2, 18.1.1.2, 19.1.1.2, 20.1.1.2, 21.1.1.2, 22.1.1.2, 23.1.1.2, 24.1.1.2, 25.1.1.2, 26.1.1.2, 27.1.1.2, 28.1.1.2, 29.1.1.2, 30.1.1.2, 31.1.1.2, 32.1.1.2, 33.1.1.2, 34.1.1.2, 35.1.1.2, 36.1.1.2, 37.1.1.2, 38.1.1.2, 39.1.1.2, 40.1.1.2, 1.2.1.2, 2.2.1.2, 3.2.1.2, 4.2.1.2, 5.2.1.2, 6.2.1.2, 7.2.1.2, 8.2.1.2, 9.2.1.2, 10.2.1.2, 11.2.1.2, 12.2.1.2, 13.2.1.2, 14.2.1.2, 15.2.1.2, 16.2.1.2, 17.2.1.2, 18.2.1.2, 19.2.1.2, 20.2.1.2, 21.2.1.2, 22.2.1.2, 23.2.1.2, 24.2.1.2, 25.2.1.2, 26.2.1.2, 27.2.1.2, 28.2.1.2, 29.2.1.2, 30.2.1.2, 31.2.1.2, 32.2.1.2, 33.2.1.2, 34.2.1.2, 35.2.1.2, 36.2.1.2, 37.2.1.2, 38.2.1.2, 39.2.1.2, 40.2.1.2, 1.3.1.2, 2.3.1.2, 3.3.1.2, 4.3.1.2, 5.3.1.2, 6.3.1.2, 7.3.1.2, 8.3.1.2, 9.3.1.2, 10.3.1.2, 11.3.1.2, 12.3.1.2, 13.3.1.2, 14.3.1.2, 15.3.1.2, 16.3.1.2, 17.3.1.2, 18.3.1.2, 19.3.1.2, 20.3.1.2, 21.3.1.2, 22.3.1.2, 23.3.1.2, 24.3.1.2, 25.3.1.2, 263.1.2, 27.3.1.2, 28.3.1.2, 29.3.1.2, 30.3.1.2, 31.3.1.2, 32.3.1.2, 33.3.1.2, 34.3.1.2, 35.3.1.2, 36.3.1.2, 37.3.1.2, 38.3.1.2, 39.3.1.2, 40.3.1.2, 1.4.1.2, 2.4.1.2, 3.4.1.2, 4.4.1.2, 5.4.1.2, 6.4.1.2, 7.4.1.2, 8.4.1.2, 9.4.1.2, 10.4.1.2, 11.4.1.2, 12.4.1.2, 13.4.1.2, 14.4.1.2, 15.4.1.2, 16.4.1.2, 17.4.1.2, 18.4.1.2, 19.4.1.2, 20.4.1.2, 21.4.1.2, 22.4.1.2, 23.4.1.2, 24.4.1.2, 25.4.1.2, 26.4.1.2, 27.4.1.2, 28.4.1.2, 29.4.1.2, 30.4.1.2, 31.4.1.2, 32.4.1.2, 33.4.1.2, 34.4.1.2, 35.4.1.2, 36.4.1.2, 37.4.1.2, 38.4.1.2, 39.4.1.2, 40.4.1.2, 1.5.1.2, 2.5.1.2, 3.5.1.2, 4.5.1.2, 5.5.1.2, 6.5.1.2, 7.5.1.2, 8.5.1.2, 9.5.1.2, 10.5.1.2, 11.5.1.2, 12.5.1.2, 13.5.1.2, 14.5.1.2, 15.5.1.2, 16.5.1.2, 17.5.1.2, 18.5.1.2, 19.5.1.2, 20.5.1.2, 21.5.1.2, 22.5.1.2, 23.5.1.2, 24.5.1.2, 25.5.1.2, 26.5.1.2, 27.5.1.2, 28.5.1.2, 29.5.1.2, 30.5.1.2, 31.5.1.2, 32.5.1.2, 33.5.1.2, 34.5.1.2, 35.5.1.2, 36.5.1.2, 37.5.1.2, 38.5.1.2, 39.5.1.2, 40.5.1.2, 1.6.1.2, 2.6.1.2, 3.6.1.2, 4.6.1.2, 5.6.1.2, 6.6.1.2, 7.6.1.2, 8.6.1.2, 9.6.1.2, 10.6.1.2, 11.6.1.2, 12.6.1.2, 13.6.1.2, 14.6.1.2, 15.6.1.2, 16.6.1.2, 17.6.1.2, 18.6.1.2, 19.6.1.2, 20.6.1.2, 21.6.1.2, 22.6.1.2, 23.6.1.2, 24.6.1.2, 25.6.1.2, 26.6.1.2, 27.6.1.2, 28.6.1.2, 29.6.1.2, 30.6.1.2, 31.6.1.2, 32.6.1.2, 33.6.1.2, 34.6.12, 35.6.1.2, 36.6.1.2, 37.6.1.2, 38.6.1.2, 39.6.1.2, 40.6.1.2, 1.7.1.2, 2.7.1.2, 3.7.1.2, 4.7.1.2, 5.7.1.2, 6.7.1.2, 7.7.1.2, 8.7.1.2, 9.7.1.2, 10.7.1.2, 11.7.1.2, 12.7.1.2, 13.7.1.2, 14.7.1.2, 15.7.1.2, 16.7.1.2, 17.7.1.2, 18.7.1.2, 19.7.1.2, 20.7.1.2, 21.7.1.2, 22.7.1.2, 23.7.1.2, 24.7.1.2, 25.7.1.2, 26.7.1.2, 27.7.1.2, 28.7.1.2, 29.7.1.2, 30.7.1.2, 31.7.1.2, 32.7.1.2, 33.7.1.2, 34.7.1.2, 35.7.1.2, 36.7.1.2, 37.7.1.2, 38.7.1.2, 39.7.1.2, 40.7.1.2, 1.8.1.2, 2.8.1.2, 3.8.1.2, 4.8.1.2, 5.8.1.2, 6.8.1.2, 7.8.1.2, 8.8.1.2, 9.8.1.2, 10.8.1.2, 11.8.1.2, 12.8.1.2, 13.8.1.2, 14.8.1.2, 15.8.1.2, 16.8.1.2, 17.8.1.2, 18.8.1.2, 19.8.1.2, 20.8.1.2, 21.8.1.2, 22.8.1.2, 23.8.1.2, 24.8.1.2, 25.8.1.2, 26.8.1.2, 27.8.1.2, 28.8.1.2, 29.8.1.2, 30.8.1.2, 31.8.1.2, 32.8.1.2, 33.8.1.2, 34.8.1.2, 35.8.1.2, 36.8.1.2, 37.8.1.2, 38.8.1.2, 39.8.1.2, 40.8.1.2,1.9.1.2, 2.9.1.2, 3.9.1.2, 4.9.1.2, 5.9.1.2, 6.9.1.2, 7.9.1.2, 8.9.1.2, 9.9.1.2, 10.9.1.2, 11.9.1.2, 12.9.1.2, 13.9.1.2, 14.9.1.2, 15.9.1.2, 16.9.1.2, 17.9.1.2, 18.9.1.2, 19.9.1.2, 20.9.1.2, 21.9.1.2, 22.9.1.2, 23.9.1.2, 24.9.1.2, 25.9.1.2, 26.9.1.2, 27.9.1.2, 28.9.1.2, 29.9.1.2, 30.9.1.2, 31.9.1.2, 32.9.1.2, 33.9.1.2, 34.9.1.2, 35.9.1.2, 36.9.1.2, 37.9.1.2, 38.9.1.2, 39.9.1.2, 40.9.1.2, 1.10.1.2, 2.10.1.2, 3.10.1.2, 4.10.1.2, 5.10.1.2, 6.10.1.2, 7.10.1.2, 8.10.1.2, 9.10.1.2, 10.10.1.2, 11.10.1.2, 12.10.1.2, 13.10.1.2, 14.10.1.2, 15.10.1.2, 16.10.1.2, 17.10.1.2, 18.10.1.2, 19.10.1.2, 20.10.1.2, 21.10.1.2, 22.10.1.2, 23.10.1.2, 24.10.1.2, 25.10.1.2, 26.10.1.2, 27.10.1.2, 28.10.1.2, 29.10.1.2, 30.10.1.2, 31.10.1.2, 32.10.1.2, 33.10.1.2, 34.10.1.2, 35.10.1.2, 36.10.1.2, 37.10.1.2, 38.10.1.2, 39.10.1.2, 40.10.1.2, 1.11.1.2, 2.11.1.2, 3.11.1.2, 4.11.1.2, 5.11.1.2, 6.11.1.2, 7.11.1.2, 8.11.1.2, 9.11.1.2, 10.11.1.2, 11.11.1.2, 12.11.1.2, 13.11.1.2, 14.11.1.2, 15.11.1.2, 16.11.1.2, 17.11.1.2, 18.11.1.2, 19.11.1.2, 20.11.1.2, 21.11.1.2, 22.11.1.2, 23.11.1.2, 24.11.1.2, 25.11.1.2, 26.11.1.2, 27.11.1.2, 28.11.1.2, 29.11.1.2, 30.11.1.2, 31.11.1.2, 32.11.1.2, 33.11.1.2, 34.11.1.2, 35.11.1.2, 36.11.1.2, 37.11.12, 38.11.1.2, 39.11.1.2, 40.11.1.2, 1.12.1.2, 2.12.1.2, 3.12.1.2, 4.12.1.2, 5.12.1.2, 6.12.1.2, 7.12.1.2, 8.12.1.2, 9.12.1.2, 10.12.1.2, 11.12.1.2, 12.12.12, 13.12.1.2, 14.12.1.2, 15.12.1.2, 16.12.1.2, 17.12.1.2, 18.12.1.2, 19.12.1.2, 20.12.1.2, 21.12.1.2, 22.12.1.2, 23.12.1.2, 24.12.1.2, 25.12.1.2, 26.12.1.2, 27.12.1.2, 28.12.1.2, 29.12.1.2, 30.12.1.2, 31.12.1.2, 32.12.1.2, 33.12.1.2, 34.12.1.2, 35.12.1.2, 36.12.1.2, 37.12.1.2, 38.12.1.2, 39.12.1.2, 40.12.1.2,1.13.1.2, 2.13.1.2, 3.13.1.2, 4.13.1.2, 5.13.1.2, 6.13.1.2, 7.13.1.2, 8.13.1.2, 9.13.1.2, 10.13.1.2, 11.13.1.2, 12.13.1.2, 13.13.1.2, 14.13.1.2, 15.13.1.2, 16.13.1.2, 17.13.1.2, 18.13.1.2, 19.13.1.2, 20.13.1.2, 21.13.1.2, 22.13.1.2, 23.13.1.2, 24.13.1.2, 25.13.1.2, 26.13.1.2, 27.13.1.2, 28.13.1.2, 29.13.1.2, 30.13.1.2, 31.13.1.2, 32.13.1.2, 33.13.1.2, 34.13.1.2, 35.13.1.2, 36.13.1.2, 37.13.1.2, 38.13.1.2, 39.13.1.2, 40.13.1.2, 1.14.1.2, 2.14.1.2, 3.14.1.2, 4.14.1.2, 5.14.1.2, 6.14.1.2, 7.14.1.2, 8.14.1.2, 9.14.1.2,10.14.1.2,11.14.1.2, 12.14.1.2,13.14.1.2,14.14.1.2,15.14.1.2, 16.14.1.2, 17.14.1.2, 18.14.1.2, 19.14.1.2, 20.14.1.2, 21.14.1.2, 22.14.1.2, 23.14.1.2, 24.14.1.2, 25.14.1.2, 26.14.1.2, 27.14.1.2, 28.14.1.2, 29.14.1.2, 30.14.1.2, 31.14.1.2, 32.14.1.2, 33.14.1.2, 34.14.1.2, 35.14.1.2, 36.14.1.2, 37.14.1.2, 38.14.1.2, 39.14.1.2, 40.14.1.2, 1.15.1.2, 2.15.1.2, 3.15.1.2, 4.15.1.2, 5.15.1.2, 6.15.1.2, 7.15.1.2, 8.15.1.2, 9.15.1.2, 10.15.1.2, 11.15.1.2, 12.15.1.2, 13.15.1.2, 14.15.1.2, 15.15.1.2, 16.15.1.2, 17.15.1.2, 18.15.1.2, 19.15.1.2, 20.15.1.2, 21.15.1.2, 22.15.1.2, 23.15.1.2, 24.15.1.2, 25.15.1.2, 26.15.1.2, 27.15.1.2, 28.15.1.2, 29.15.1.2, 30.15.1.2, 31.15.1.2, 32.15.1.2, 33.15.1.2, 34.15.1.2, 35.15.1.2, 36.15.1.2, 37.15.1.2, 38.15.1.2, 39.15.1.2, 40.15.1.2, 1.16.1.2, 2.16.1.2, 3.16.1.2, 4.16.1.2, 5.16.1.2, 6.16.1.2, 7.16.1.2, 8.16.1.2, 9.16.1.2, 10.16.1.2, 11.16.1.2, 12.16.1.2, 13.16.1.2, 14.16.1.2, 15.16.1.2, 16.16.1.2, 17.16.1.2, 18.16.1.2, 19.16.1.2, 20.16.1.2, 21.16.1.2, 22.16.1.2, 23.16.1.2, 24.16.1.2, 25.16.1.2, 26.16.1.2, 27.16.1.2, 28.16.1.2, 29.16.1.2, 30.16.1.2, 31.16.1.2, 32.16.1.2, 33.16.1.2, 34.16.1.2, 35.16.1.2, 36.16.1.2, 37.16.1.2, 38.16.1.2, 39.16.1.2, 40.16.1.2, 1.17.1.2, 2.17.1.2, 3.17.1.2, 4.17.1.2, 5.17.1.2, 6.17.1.2, 7.17.1.2, 8.17.1.2, 9.17.1.2, 10.17.1.2, 11.17.1.2, 12.17.1.2, 13.17.1.2, 14.17.1.2, 15.17.1.2, 16.17.1.2,17.17.1.2,18.17.1.2,19.17.1.2, 20.17.1.2, 21.17.1.2, 22.17.1.2, 23.17.1.2, 24.17.1.2, 25.17.1.2, 26.17.1.2, 27.17.1.2, 28.17.1.2, 29.17.1.2, 30.17.1.2, 31.17.1.2, 32.17.1.2, 33.17.1.2, 34.17.1.2, 35.17.1.2, 36.17.1.2, 37.17.1.2, 38.17.1.2, 39.17.1.2, 40.17.1.2, 1.18.1.2, 2.18.1.2, 3.18.1.2, 4.18.1.2, 5.18.1.2, 6.18.1.2, 7.18.1.2, 8.18.1.2, 9.18.1.2, 10.18.1.2, 11.18.1.2, 12.18.1.2, 13.18.1.2, 4.18.1.2, 15.18.1.2, 16.18.1.2, 17.18.1.2, 18.18.1.2, 19.18.1.2, 20.18.1.2, 21.18.1.2, 22.18.1.2, 23.18.1.2, 24.18.1.2, 25.18.1.2, 26.18.1.2, 27.18.1.2, 28.18.1.2, 29.18.1.2, 30.18.1.2, 31.18.1.2, 32.18.1.2, 33.18.1.2, 34.18.1.2, 35.18.1.2, 36.18.1.2, 37.18.1.2, 38.18.1.2, 39.18.1.2, 40.18.1.2, 1.19.1.2, 2.19.1.2, 3.19.1.2, 4.19.1.2, 5.19.1.2, 6.19.1.2, 7.19.1.2, 8.19.1.2, 9.19.1.2, 10.19.1.2, 11.19.1.2, 12.19.1.2, 13.19.1.2, 14.19.1.2, 15.19.1.2, 16.19.1.2, 17.19.1.2, 18.19.1.2, 19.19.1.2, 20.19.1.2, 21.19.1.2, 22.19.1.2, 23.19.1.2, 24.19.1.2, 25.19.1.2, 26.19.1.2, 27.19.1.2, 28.19.1.2, 29.19.1.2, 30.19.1.2, 31.19.1.2, 32.19.1.2, 33.19.1.2, 34.19.1.2, 35.19.1.2, 36.19.1.2, 37.19.1.2, 38.19.1.2, 39.19.1.2, 40.19.1.2, 1.20.1.2, 2.20.1.2, 3.20.1.2, 4.20.1.2, 5.20.1.2, 6.20.1.2, 7.20.1.2, 8.20.1.2, 9.20.1.2,10.20.1.2, 11.20.1.2, 12.26.1.2, 13.20.1.2, 14.20.1.2, 15.20.1.2, 16.20.1.2, 17.20.1.2, 18.20.1.2, 19.20.1.2, 20.20.1.2, 21.20.1.2, 22.20.1.2, 23.20.1.2, 24.20.1.2, 25.20.1.2, 26.20.1.2, 27.20.1.2, 28.20.12, 29.20.1.2, 30.20.1.2, 31.20.1.2, 32.20.1.2, 33.20.1.2, 34.20.1.2, 35.20.1.2, 36.20.1.2, 37.20.1.2, 38.20.1.2, 39.20.1.2, 40.20.1.2, 1.21.1.2, 2.21.1.2, 3.21.1.2, 4.21.1.2, 5.21.1.2, 6.21.1.2, 7.21.1.2, 8.21.1.2, 9.21.1.2, 10.21.1.2, 11.21.1.2, 12.21.1.2, 13.21.1.2, 14.21.1.2, 15.21.1.2, 16.21.1.2, 17.21.1.2, 18.21.1.2, 19.21.1.2, 20.21.1.2, 21.21.1.2, 22.21.1.2, 23.21.1.2, 24.21.1.2, 25.21.1.2, 26.21.1.2, 27.21.1.2, 28.21.1.2, 29.21.1.2, 30.21.1.2, 31.21.1.2, 32.21.1.2, 33.21.1.2, 34.21.1.2, 35.21.1.2, 36.21.1.2, 37.21.1.2, 38.21.1.2, 39.21.1.2, 40.21.1.2, 1.22.1.2, 2.22.1.2, 3.22.1.2, 4.22.1.2, 5.22.1.2, 6.22.1.2, 7.22.1.2, 8.22.1.2, 9.22.1.2, 10.22.1.2, 11.22.1.2, 12.22.1.2, 13.22.1.2, 14.22.1.2, 15.22.1.2, 16.22.1.2, 17.22.1.2, 18.22.1.2, 19.22.1.2, 20.22.1.2, 21.22.1.2, 22.22.1.2, 23.22.1.2, 24.22.1.2, 25.22.1.2, 26.22.1.2, 27.22.1.2, 28.22.1.2, 29.22.1.2, 30.22.1.2, 31.22.1.2, 32.22.1.2, 33.22.1.2, 34.22.1.2, 35.22.1.2, 36.22.1.2, 37.22.1.2, 38.22.1.2, 39.22.1.2, 40.22.1.2, 1.23.1.2, 2.23.1.2, 3.23.1.2, 4.23.1.2, 5.23.1.2, 6.23.1.2, 7.23.1.2, 8.23.1.2, 9.23.1.2, 10.23.1.2, 11.23.1.2, 12.23.1.2, 13.23.1.2, 14.23.1.2, 15.23.1.2, 16.23.1.2, 17.23.1.2, 18.23.1.2, 19.23.1.2, 20.23.1.2, 21.23.1.2, 22.23.1.2, 23.23.1.2, 24.23.1.2, 25.23.1.2, 26.23.1.2, 27.23.1.2, 28.23.1.2, 29.23.1.2, 30.23.1.2, 31.23.1.2, 32.23.1.2, 33.23.1.2, 34.23.1.2, 35.23.1.2, 36.23.1.2, 37.23.1.2, 38.23.1.2, 39.23.1.2, 40.23.1.2, 1.24.1.2, 2.24.1.2, 3.24.1.2, 4.24.1.2, 5.24.1.2, 6.24.1.2, 7.24.1.2, 8.24.1.2, 9.24.1.2, 10.24.1.2, 11.24.1.2, 12.24.1.2, 13.24.1.2, 14.24.1.2, 15.24.1.2, 16.24.1.2,17.24.1.2,18.24.1.2,19.24.1.2, 20.24.1.2, 21.24.1.2, 22.24.1.2, 23.24.1.2, 24.24.1.2, 25.24.1.2, 26.24.1.2, 27.24.1.2, 28.24.1.2, 29.24.1.2, 3024.1.2, 31.24.1.2, 32.24.1.2, 33.24.1.2, 34.24.1.2, 35.24.1.2, 36.24.1.2, 37.24.1.2, 38.24.1.2, 39.24.1.2, 40.24.1.2, 1.25.1.2, 2.25.1.2, 3.25.1.2, 4.25.1.2, 5.25.1.2, 6.25.1.2, 7.25.1.2, 8.25.1.2, 9.25.1.2, 10.25.1.2, 11.25.1.2, 12.25.1.2, 13.25.1.2, 14.25.1.2, 15.25.1.2, 16.25.1.2, 17.25.1.2, 18.25.1.2, 19.25.1.2, 20.25.1.2, 21.25.1.2, 22.25.1.2, 23.25.1.2, 24.25.1.2, 25.25.1.2, 26.25.1.2, 27.25.1.2, 28.25.1.2, 29.25.1.2, 30.25.1.2, 31.25.1.2, 32.25.1.2, 33.25.1.2, 34.25.1.2, 35.25.1.2, 36.25.1.2, 37.25.1.2, 38.25.1.2, 39.25.1.2, 40.25.1.2, 1.26.1.2, 2.26.1.2, 3.26.1.2, 4.26.1.2, 5.26.1.2, 6.26.1.2, 7.26.1.2, 8.26.1.2, 9.26.12, 10.26.1.2, 11.26.1.2, 12.26.1.2, 13.26.1.2, 14.26.1.2, 15.26.1.2, 16.26.1.2, 17.26.1.2, 18.26.1.2, 19.26.1.2, 20.26.1.2, 21.26.1.2, 22.26.1.2, 23.26.1.2, 24.26.1.2, 25.26.1.2, 26.26.1.2, 27.26.1.2, 28.26.1.2, 29.26.1.2, 30.26.1.2, 31.26.1.2, 32.26.1.2, 33.26.1.2, 34.26.1.2, 35.26.1.2, 36.26.1.2, 37.26.1.2, 38.26.1.2, 39.26.1.2, 40.26.1.2,1.27.1.2, 2.27.1.2, 3.27.1.2, 4.27.1.2, 5.27.1.2, 6.27.1.2, 7.27.1.2, 8.27.1.2, 9.27.1.2, 10.27.1.2, 11.27.1.2, 12.27.1.2, 13.27.1.2, 14.27.1.2, 15.27.1.2, 16.27.1.2, 17.27.1.2, 18.27.1.2, 19.27.1.2, 20.27.1.2, 21.27.1.2, 22.27.1.2, 23.27.1.2, 24.27.1.2, 25.27.1.2, 26.27.1.2, 27.27.1.2, 28.27.1.2, 29.27.1.2, 30.27.1.2, 31.27.1.2, 32.27.1.2, 33.27.1.2, 34.27.1.2, 35.27.1.2, 36.27.1.2, 37.27.1.2, 38.27.1.2, 39.27.1.2, 40.27.1.2, 1.28.1.2, 2.28.1.2, 3.28.1.2, 4.28.1.2, 5.28.1.2, 6.28.1.2, 7.28.1.2, 8.28.1.2, 9.28.1.2,10.28.1.2,11.28.1.2, 12.28.1.2, 13.28.1.2, 14.28.1.2, 15.28.1.2, 16.28.1.2, 17.28.1.2, 18.28.1.2, 19.28.1.2, 20.28.1.2, 21.28.1.2, 22.28.1.2, 23.28.1.2, 24.28.1.2, 25.28.1.2, 26.28.1.2, 27.28.1.2, 28.28.1.2, 29.28.1.2, 30.28.1.2, 31.28.1.2, 32.28.1.2, 33.28.1.2, 34.28.1.2, 35.28.1.2, 36.28.1.2, 37.28.1.2, 38.28.1.2, 39.28.1.2, 40.28.1.2, 1.29.1.2, 2.29.1.2, 3.29.1.2, 4.29.1.2, 5.29.1.2, 6.29.1.2, 7.29.1.2, 8.29.1.2, 9.29.1.2, 10.29.1.2, 11.29.1.2, 12.29.1.2, 13.29.1.2, 14.29.1.2, 15.29.1.2, 16.29.1.2, 17.29.1.2, 18.29.1.2, 19.29.1.2, 20.29.1.2, 21.29.1.2, 22.29.1.2, 23.29.1.2, 24.29.1.2, 25.29.1.2, 26.29.1.2, 27.29.1.2, 28.29.1.2, 29.29.1.2, 30.29.1.2, 31.29.1.2, 32.29.1.2, 33.29.1.2, 34.29.1.2, 35.29.1.2, 36.29.1.2, 37.29.1.2, 38.29.1.2, 39.29.1.2, 40.29.1.2,1.1.3.1, 2.1.3.1, 3.1.3.1, 4.1.3.1, 5.1.3.1, 6.1.3.1, 7.1.3.1, 8.1.3.1, 9.1.3.1, 10.1.3.1, 11.1.3.1, 12.1.3.1, 13.1.3.1, 14.1.3.1, 15.1.3.1, 16.1.3.1, 17.1.3.1, 18.1.3.1, 19.1.3.1, 20.1.3.1, 21.1.3.1, 22.1.3.1, 23.1.3.1, 24.1.3.1, 25.1.3.1, 26.1.3.1, 27.1.3.1, 28.1.3.1, 29.1.3.1, 30.1.3.1, 31.1.3.1, 32.1.3.1, 33.1.3.1, 34.1.3.1, 35.1.3.1, 36.1.3.1, 37.1.3.1, 38.1.3.1, 39.1.3.1, 40.1.3.1, 1.2.3.1, 2.23.1, 3.2.3.1, 4.2.3.1, 5.2.3.1, 6.2.3.1, 7.2.3.1, 8.2.3.1, 9.2.3.1, 10.2.3.1, 11.2.3.1, 12.2.3.1, 13.2.3.1, 14.2.3.1, 15.2.3.1, 16.2.3.1, 17.2.3.1, 18.2.3.1, 19.2.3.1, 20.2.3.1, 21.2.3.1, 22.2.3.1, 23.2.3.1, 24.2.3.1, 25.2.3.1, 26.2.3.1, 27.2.3.1, 28.2.3.1, 29.2.3.1, 30.2.3.1, 31.2.3.1, 32.2.3.1, 33.2.3.1, 34.2.3.1, 35.2.3.1, 36.2.3.1, 37.2.3.1, 38.2.3.1, 39.2.3.1, 40.2.3.1, 1.3.3.1, 2.3.3.1, 3.3.3.1, 4.3.3.1, 5.3.3.1, 6.3.3.1, 7.3.3.1, 8.3.3.1, 9.3.3.1, 10.3.3.1, 11.3.3.1, 12.3.3.1, 13.3.3.1, 14.3.3.1, 15.3.3.1, 16.3.3.1, 17.3.3.1, 18.3.3.1, 19.3.3.1, 20.3.3.1, 21.3.3.1, 22.3.3.1, 23.3.3.1, 24.3.3.1, 25.3.3.1, 26.3.3.1, 27.3.3.1, 28.3.3.1, 29.3.3.1, 30.3.3.1, 31.3.3.1, 32.3.3.1, 33.3.3.1, 34.3.3.1, 35.3.3.1, 36.3.3.1, 37.3.3.1, 38.3.3.1, 39.3.3.1, 40.3.3.1, 1.4.3.1, 2.4.3.1, 3.4.3.1, 4.4.3.1, 5.4.3.1, 6.4.3.1, 7.4.3.1, 8.4.3.1, 9.4.3.1, 10.4.3.1, 11.4.3.1, 12.4.3.1, 13.4.3.1, 14.4.3.1, 15.4.3.1, 16.4.3.1, 17.4.3.1, 18.4.3.1, 19.43.1, 20.4.3.1, 21.4.3.1, 22.4.3.1, 23.4.3.1, 24.4.3.1, 25.4.3.1, 26.4.3.1, 27.4.3.1, 28.4.3.1, 29.4.3.1, 30.4.3.1, 31.4.3.1, 32.4.3.1, 33.4.3.1, 34.4.3.1, 35.4.3.1, 36.4.3.1, 37.4.3.1, 38.4.3.1, 39.4.3.1, 40.4.3.1,1.5.3.1, 2.5.3.1, 3.5.3.1, 4.5.3.1, 5.5.3.1, 6.5.3.1, 7.5.3.1, 8.5.3.1, 9.5.3.1, 10.5.3.1, 11.53.1, 12.5.3.1, 13.5.3.1, 14.5.3.1, 15.5.3.1, 16.5.3.1, 17.5.3.1, 18.5.3.1, 19.5.3.1, 20.5.3.1, 21.5.3.1, 22.5.3.1, 23.5.3.1, 24.5.3.1, 25.5.3.1, 26.5.3.1, 27.5.3.1, 28.5.3.1, 29.5.3.1, 30.5.3.1, 31.5.3.1, 32.5.3.1, 33.5.3.1, 34.5.3.1, 35.5.3.1, 36.5.3.1, 37.5.3.1, 38.5.3.1, 39.5.3.1, 40.5.3.1, 1.6.3.1, 2.6.3.1, 3.6.3.1, 4.6.3.1, 5.6.3.1, 6.6.3.1, 7.6.3.1, 8.6.3.1, 9.6.3.1, 10.6.3.1, 11.6.3.1, 12.6.3.1, 13.6.3.1, 14.6.3.1, 15.6.3.1, 16.6.3.1, 17.6.3.1, 18.6.3.1, 19.6.3.1, 20.6.3.1, 21.6.3.1, 22.6.3.1, 23.6.3.1, 24.6.3.1, 25.6.3.1, 26.6.3.1, 27.6.3.1, 28.6.3.1, 29.6.3.1, 30.6.3.1, 31.6.3.1, 32.6.3.1, 33.6.3.1, 34.6.3.1, 35.6.3.1, 36.6.3.1, 37.6.3.1, 38.6.3.1, 39.6.3.1, 40.6.3.1, 1.7.3.1, 2.7.3.1, 3.7.3.1, 4.7.3.1, 5.7.3.1, 6.7.3.1, 7.7.3.1, 8.7.3.1, 9.7.3.1, 10.7.3.1, 11.7.3.1, 12.7.3.1, 13.7.3.1, 14.7.3.1, 15.7.3.1, 16.7.3.1, 17.7.3.1, 18.7.3.1,19.7.3.1, 20.7.3.1, 21.7.3.1, 22.7.3.1, 23.7.3.1, 24.7.3.1, 25.7.3.1, 26.7.3.1, 27.7.3.1, 28.7.3.1, 29.7.3.1, 30.7.3.1, 31.7.3.1, 32.7.3.1, 33.7.3.1, 34.7.3.1, 35.7.3.1, 36.7.3.1, 37.7.3.1, 38.7.3.1, 39.7.3.1, 40.7.3.1,1.8.3.1, 2.8.3.1, 3.8.3.1, 4.8.3.1, 5.8.3.1, 6.8.3.1, 7.8.3.1, 8.8.3.1, 9.8.3.1, 10.8.3.1, 11.8.3.1, 12.8.3.1, 13.8.3.1, 14.8.3.1, 15.8.3.1, 16.8.3.1, 17.8.3.1, 18.8.3.1, 19.8.3.1, 20.8.3.1, 21.8.3.1, 22.8.3.1, 23.8.3.1, 24.8.3.1, 25.8.3.1, 26.8.3.1, 27.8.3.1, 28.8.3.1, 29.8.3.1, 30.8.3.1, 31.8.3.1, 32.8.3.1, 33.8.3.1, 34.8.3.1, 35.8.3.1, 36.8.3.1, 37.8.3.1, 38.8.3.1, 39.8.3.1, 40.8.3.1, 1.9.3.1, 2.9.3.1, 3.9.3.1, 4.9.3.1, 5.9.3.1, 6.9.3.1, 7.9.3.1, 8.9.3.1, 9.9.3.1, 10.9.3.1, 11.9.3.1, 12.9.3.1, 13.9.3.1, 14.9.3.1, 15.9.3.1,16.9.3.1,17.9.3.1,18.9.3.1,19.9.3.1, 20.9.3.1, 21.9.3.1, 22.9.3.1, 23.9.3.1, 24.9.3.1, 25.9.3.1, 26.9.3.1, 27.9.3.1, 28.9.3.1, 29.9.3.1, 30.9.3.1, 31.9.3.1, 32.9.3.1, 33.9.3.1, 34.9.3.1, 35.9.3.1, 36.9.3.1, 37.9.3.1, 38.9.3.1, 39.9.3.1, 40.9.3.1, 1.10.3.1, 2.10.3.1, 3.10.3.1, 4.10.3.1, 5.10.3.1, 6.10.3.1, 7.10.3.1, 8.10.3.1, 9.10.3.1, 10.10.3.1, 11.10.3.1, 12.10.3.1, 13.10.3.1, 14.10.3.1, 15.10.3.1, 16.10.3.1, 17.10.3.1, 18.10.3.1, 19.10.3.1, 20.10.3.1, 21.10.3.1, 22.10.3.1, 23.10.3.1, 24.10.3.1, 25.10.3.1, 26.10.3.1, 27.10.3.1, 28.10.3.1, 29.10.3.1, 30.10.3.1, 31.10.3.1, 32.10.3.1, 33.10.3.1, 34.10.3.1, 35.10.3.1, 36.10.3.1, 37.10.3.1, 38.10.3.1, 39.10.3.1, 40.10.3.1, 1.11.3.1, 2.11.3.1, 3.11.3.1, 4.11.3.1, 5.11.3.1, 6.11.3.1, 7.11.3.1, 8.11.3.1, 9.11.3.1,10.11.3.1,11.11.3.1, 12.11.3.1, 13.11.3.1, 14.11.3.1, 15.11.3.1, 16.11.3.1, 17.11.3.1, 18.11.3.1, 19.11.3.1, 20.11.3.1, 21.11.3.1, 22.11.3.1, 23.11.3.1, 24.11.3.1, 25.11.3.1, 26.11.3.1, 27.11.3.1, 28.11.3.1, 29.11.3.1, 30.11.3.1, 31.11.3.1, 32.11.3.1, 33.11.3.1, 34.11.3.1, 35.11.3.1, 36.11.3.1, 37.11.3.1, 38.11.3.1, 39.11.3.1, 40.11.3.1,1.12.3.1, 2.12.3.1, 3.12.3.1, 4.12.3.1, 5.12.3.1, 6.12.3.1, 7.12.3.1, 8.12.3.1, 9.12.3.1, 10.12.3.1, 11.12.3.1, 12.12.3.1, 13.12.3.1, 14.12.3.1, 15.12.3.1, 16.12.3.1, 17.12.3.1, 18.12.3.1, 19.12.3.1, 20.12.3.1, 21.12.3.1, 22.12.3.1, 23.12.3.1, 24.12.3.1, 25.12.3.1, 26.12.3.1, 27.12.3.1, 28.12.3.1, 29.12.3.1, 30.12.3.1, 31.12.3.1, 32.12.3.1, 33.123.1, 34.12.3.1, 35.12.3.1, 36.12.3.1, 37.12.3.1, 38.12.3.1, 39.12.3.1, 40.12.3.1,1.13.3.1, 2.13.3.1, 3.13.3.1, 4.13.3.1, 5.13.3.1, 6.13.3.1, 7.13.3.1, 8.13.3.1, 9.13.3.1, 10.133.1, 11.13.3.1, 12.13.3.1, 13.13.3.1, 14.133.1, 15.13.3.1, 16.133.1, 17.13.3.1, 18.13.3.1, 19.13.3.1, 20.13.3.1, 21.13.3.1, 22.133.1, 23.13.3.1, 24.13.3.1, 25.13.3.1, 26.13.3.1, 27.13.3.1, 28.13.3.1, 29.13.3.1, 30.13.3.1, 31.13.3.1, 32.13.3.1, 33.13.3.1, 34.13.3.1, 35.13.3.1, 36.13.3.1, 37.13.3.1, 38.13.3.1, 39.13.3.1, 40.13.3.1, 1.14.3.1, 2.14.3.1, 3.14.3.1, 4.14.3.1, 5.14.3.1, 6.14.3.1, 7.14.3.1, 8.14.3.1, 9.14.3.1, 10.14.3.1, 11.14.3.1, 12.14.3.1, 13.14.3.1, 14.14.3.1, 15.14.3.1, 16.14.3.1,17.14.3.1,18.14.3.1,19.14.3.1, 20.14.3.1, 21.14.3.1, 22.14.3.1, 23.14.3.1, 24.14.3.1, 25.14.3.1, 26.14.3.1, 27.14.3.1, 28.14.3.1, 29.14.3.1, 30.14.3.1, 31.14.3.1, 32.14.3.1, 33.14.3.1, 34.14.3.1, 35.14.3.1, 36.14.3.1, 37.14.3.1, 38.14.3.1, 39.14.3.1, 40.14.3.1, 1.15.3.1, 2.15.3.1, 3.15.3.1, 4.15.3.1, 5.15.3.1, 6.15.3.1, 7.15.3.1, 8.15.3.1, 9.15.3.1, 10.15.3.1, 11.15.3.1, 12.15.3.1, 13.15.3.1, 14.15.3.1, 15.15.3.1, 16.15.3.1, 17.15.3.1, 18.15.3.1, 19.15.3.1, 20.15.3.1, 21.15.3.1, 22.15.3.1, 23.15.3.1, 24.15.3.1, 25.15.3.1, 26.15.3.1, 27.15.3.1, 28.15.3.1, 29.15.3.1, 30.15.3.1, 31.15.3.1, 32.15.3.1, 33.15.3.1, 34.15.3.1, 35.15.3.1, 36.15.3.1, 37.15.3.1, 38.15.3.1, 39.15.3.1, 40.15.3.1, 1.16.3.1, 2.16.3.1, 3.16.3.1, 4.16.3.1, 5.16.3.1, 6.16.3.1, 7.16.3.1, 8.16.3.1, 9.16.3.1, 10.16.3.1, 11.16.3.1, 12.16.3.1, 13.16.3.1, 14.16.3.1, 15.16.3.1, 16.16.3.1, 17.16.3.1, 18.16.3.1, 19.16.3.1, 20.16.3.1, 21.16.3.1, 22.16.3.1, 23.16.3.1, 24.16.3.1, 25.16.3.1, 26.16.3.1, 27.16.3.1, 28.16.3.1, 29.16.3.1, 30.16.3.1, 31.16.3.1, 32.16.3.1, 33.16.3.1, 34.16.3.1, 35.16.3.1, 36:16.3.1, 37.16.3.1, 38.16.3.1, 39.16.3.1, 40.16.3.1,1.17.3.1, 2.17.3.1, 3.17.3.1, 4.17.3.1, 5.17.3.1, 6.17.3.1, 7.17.3.1, 8.17.3.1, 9.17.3.1, 10.17.3.1, 11.17.3.1, 12.17.3.1, 13.17.3.1, 14.17.3.1, 15.17.3.1, 16.17.3.1, 17.17.3.1, 18.17.3.1, 19.17.3.1, 20.17.3.1, 21.17.3.1, 22.173.1, 23.17.3.1, 24.17.3.1, 25.17.3.1, 26.17.3.1, 27.17.3.1, 28.17.3.1, 29.17.3.1, 30.17.3.1, 31.17.3.1, 32.17.3.1, 33.17.3.1, 34.17.3.1, 35.17.3.1, 36.17.3.1, 37.17.3.1, 38.17.3.1, 39.17.3.1, 40.17.3.1,1.18.3.1, 2.183.1, 3.18.3.1, 4.18.3.1, 5.18.3.1, 6.18.3.1, 7.18.3.1, 8.18.3.1, 9.18.3.1, 10.18.3.1, 11.18.3.1, 12.18.3.1, 13.18.3.1, 14.18.3.1, 15.183.1, 16.18.3.1, 17.18.3.1, 18.18.3.1, 19.18.3.1, 20.18.3.1, 21.18.3.1, 22.18.3.1, 23.18.3.1, 24.18.3.1, 25.18.3.1, 26.18.3.1, 27.18.3.1, 28.18.3.1, 29.18.3.1, 30.18.3.1, 31.18.3.1, 32.18.3.1, 33.18.3.1, 34.18.3.1, 35.18.3.1, 36.18.3.1, 37.18.3.1, 38.18.3.1, 39.18.3.1, 40.18.3.1,1.19.3.1, 2.19.3.1, 3.19.3.1, 4.19.3.1, 5.19.3.1, 6.19.3.1, 7.19.3.1, 8.19.3.1, 9.19.3.1, 10.19.3.1, 11.19.3.1, 12.193.1, 13.19.3.1, 14.19.3.1, 15.19.3.1, 16.19.3.1, 17.19.3.1, 18.19.3.1, 19.193.1, 20.19.3.1, 21.19.3.1, 22.19.3.1, 23.19.3.1, 24.19.3.1, 25.19.3.1, 26.19.3.1, 27.19.3.1, 28.19.3.1, 29.19.3.1, 30.19.3.1, 31.19.3.1, 32.19.3.1, 33.19.3.1, 34.19.3.1, 35.19.3.1, 36.19.3.1, 37.19.3.1, 38.19.3.1, 39.19.3.1, 40.19.3.1,1.20.3.1, 2.20.3.1, 3.203.1, 4.20.3.1, 5.20.3.1, 6.20.3.1, 7.20.3.1, 8.20.3.1, 9.20.3.1, 10.203.1, 11.20.3.1, 12.20.3.1, 13.20.3.1, 14.20.3.1, 15.20.3.1, 16.203.1, 17.20.3.1, 18.203.1, 19.20.3.1, 20.20.3.1, 21.20.3.1, 22.20.3.1, 23.20.3.1, 24.203.1, 25.20.3.1, 26.20.3.1, 27.20.3.1, 28.20.3.1, 29.20.3.1, 30.20.3.1, 31.20.3.1, 32.20.3.1, 33.20.3.1, 34.20.3.1, 35.20.3.1, 36.20.3.1, 37.20.3.1, 38.20.3.1, 39.20.3.1, 40.20.3.1, 1.21.3.1, 2.21.3.1, 3.21.3.1, 4.21.3.1, 5.21.3.1, 6.21.3.1, 7.21.3.1, 8.21.3.1, 9.21.3.1, 10.21.3.1, 11.21-3.1, 12.21.3.1, 13.21.3.1, 14.21.3.1, 15.213.1, 16.21.3.1, 17.21.3.1, 18.21.3.1, 19.21.3.1, 20.21.3.1, 21.21.3.1, 22.21.3.1, 23.21.3.1, 24.21.3.1, 25.21.3.1, 26.21.3.1, 27.21.3.1, 28.21.3.1, 29.21.3.1, 30.21.3.1, 31.21.3.1, 32.21.3.1, 33.21.3.1, 34.21.3.1, 35.21.3.1, 36.21.3.1, 37.21.3.1, 38.21.3.1, 39.21.3.1, 40.21.3.1,1.22.3.1, 2.22.3.1, 3.22.3.1, 4.22.3.1, 5.22.3.1, 6.22.3.1, 7.22.3.1, 8.22.3.1, 9.22.3.1, 10.22.3.1, 11.22.3.1, 12.22.3.1, 13.22.3.1, 14.22.3.1, 15.22.3.1, 16.22.3.1, 17.22.3.1, 18.22.3.1,19.22.3.1, 20.22.3.1, 21.22.3.1, 22.22.3.1, 23.22.3.1, 24.22.3.1, 25.22.3.1, 26.22.3.1, 27.22.3.1, 28.22.3.1, 29.22.3.1, 30.22.3.1, 31.22.3.1, 32.22.3.1, 33.22.3.1, 34.22.3.1, 35.22.3.1, 36.22.3.1, 37.22.3.1, 38.22.3.1, 39.22.3.1, 40.22.3.1, 1.23.3.1, 2.23.3.1, 3.23.3.1, 4.23.3.1, 5.23.3.1, 6.23.3.1, 7.23.3.1, 8.23.3.1, 9.23.3.1, 10.23.3.1, 11.23.3.1, 12.23.3.1, 13.23.3.1, 14.23.3.1, 15.23.3.1, 16.23.3.1, 17.23.3.1, 18.23.3.1, 19.23.3.1, 20.23.3.1, 21.23.3.1, 22.23.3.1, 23.23.3.1, 24.23.3.1, 25.23.3.1, 26.23.3.1, 27.23.3.1, 28.23.3.1, 29.23.3.1, 30.23.3.1, 31.23.3.1, 32.23.3.1, 33.23.3.1, 34.23.3.1, 35.23.3.1, 36.23.3.1, 37.23.3.1, 38.23.3.1, 39.23.3.1, 40.23.3.1,1.24.3.1, 2.24.3.1, 3.24.3.1, 4.24.3.1, 5.24.3.1, 6.24.3.1, 7.24.3.1, 8.24.3.1, 9.24.3.1, 10.24.3.1, 11.24.3.1, 12.24.3.1, 13.24.3.1, 14.24.3.1, 15.24.3.1, 16.24.3.1, 17.24.3.1, 18.24.3.1, 19.24.3.1, 20.24.3.1, 21.243.1, 22.24.3.1, 2324.3.1, 24.24.3.1, 25.24.3.1, 26.24.3.1, 27.24.3.1, 28.24.3.1, 29.24.3.1, 30.24.3.1, 31.24.3.1, 32.24.3.1, 33.24.3.1, 34.24.3.1, 35.24.3.1, 36.24.3.1, 37.24.3.1, 38.24.3.1, 39.24.3.1, 40.24.3.1, 1.25.3.1, 2.25.3.1, 3.25.3.1, 4.25.3.1, 5.25.3.1, 6.25.3.1, 7.25.3.1, 8.25.3.1, 9.25.3.1, 10.25.3.1, 11.25.3.1, 12.25.3.1, 13.25.3.1, 14.25.3.1, 5.25.3.1,16.25.3.1, 7.25.3.1, 18.25.3.1, 19.25.3.1, 20.25.3.1, 21.25.3.1, 22.25.3.1, 23.25.3.1, 24.25.3.1, 25.25.3.1, 26.25.3.1, 27.25.3.1, 28.25.3.1, 29.25.3.1, 30.25.3.1, 31.25.3.1, 32.25.3.1, 33.25.3.1, 34.25.3.1, 35.25.3.1, 36.25.3.1, 37.25.3.1, 38.25.3.1, 39.25.3.1, 40.25.3.1, 1.26.3.1, 2.26.3.1, 3.26.3.1, 4.26.3.1, 5.26.3.1, 6.26.3.1, 7.26.3.1, 8.26.3.1, 9.26.3.1, 10.263.1, 11.26.3.1, 12.26.3.1, 13.26.3.1, 14.26.3.1, 15.26.3.1, 16.26.3.1, 17.263.1, 18126.3.1, 19.26.3.1, 20.26.3.1, 21.26.3.1, 22.26.3.1, 23.26.3.1, 24.26.3.1, 25.26.3.1, 26.26.3.1, 27.26.3.1, 28.26.3.1, 29.26.3.1, 30.26.3.1, 31.26.3.1, 32.26.3.1, 33.263.1, 34.26.3.1, 35.26.3.1, 36.26.3.1, 37.26.3.1, 38.26.3.1, 39.26.3.1, 40.26.3.1, 1.27.3.1, 2.27.3.1, 3.27.3.1, 4.27.3.1, 5.27.3.1, 6.27.3.1, 7.27.3.1, 8.27.3.1, 9.27.3.1, 10.27.3.1, 11.27.3.1, 12.27.3.1, 13.27.3.1, 14.27.3.1, 15.27.3.1, 16.27.3.1, 17.27.3.1, 18.27.3.1, 19.27.3.1, 20.27.3.1, 21.27.3.1, 22.27.3.1, 23.27.3.1, 24.27.3.1, 25.27.3.1, 26.27.3.1, 27.27.3.1, 28.27.3.1, 29.27.3.1, 30.27.3.1, 31.27.3.1, 32.27.3.1, 33.27.3.1, 34.27.3.1, 35.27.3.1, 36.27.3.1, 3727.3.1, 38.27.3.1, 39.27.3.1, 40.27.3.1,128.3.1, 2.283.1, 3.28.3.1, 4.283.1, 5.28.3.1, 6.28.3.1, 7.28.3.1, 8.28.3.1, 9.28.3.1, 10.28.3.1, 11.28.3.1, 12.28.3.1, 13.28.3.1, 14.28.3.1, 15.28.3.1, 16.28.3.1, 17.28.3.1, 18.28.3.1, 19.28.3.1, 20.28.3.1, 21.28.3.1, 22.28.3.1, 23.28.3.1, 24.28.3.1, 25.28.3.1, 26.28.3.1, 27.28.3.1, 28.28.3.1, 29.28.3.1, 30.28.3.1, 31.28.3.1, 32.28.3.1, 33.28.3.1, 34.28.3.1, 35.28.3.1, 36.28.3.1, 37.28.3.1, 38.28.3.1, 39.28.3.1, 40.28.3.1, 1.29.3.1, 2.29.3.1, 3.29.3.1, 4.29.3.1, 5.29.3.1, 6.29.3.1, 7.29.3.1, 8.29.3.1, 9.29.3.1, 10.29.3.1, 11.29.3.1, 12.29.3.1, 13.29.3.1, 14.29.3.1, 15.29.3.1, 16.29.3.1, 17.29.3.1, 18.29.3.1, 19.29.3.1, 20.29.3.1, 21.29.3.1, 22.29.3.1, 23.29.3.1, 24.29.3.1, 25.29.3.1, 26.29.3.1, 27.29.3.1, 28.29.3.1, 29.29.3.1, 30.29.3.1, 31.29.3.1, 32.29.3.1, 33.29.3.1, 34.29.3.1, 35.29.3.1, 36.29.3.1, 37.29.3.1, 38.29.3.1, 3929.3.1, 40.29.3.1, 1.1.3.2, 2.13.2, 3.1.3.2, 4.13.2, 5.1.3.2, 6.1.3.2, 7.1.3.2, 8.1.3.2, 9.1.3.2, 10.1.3.2, 11.1.3.2, 12.1.3.2, 13.1.3.2, 14.1.3.2, 15.1.3.2, 16.1.3.2, 17.1.3.2, 18.1.3.2, 19.1.3.2, 20.1.3.2, 21.1.3.2, 22.1.3.2, 23.1.3.2, 24.1.3.2, 25.1.3.2, 26.1.3.2, 27.1.3.2, 28.1.3.2, 29.1.3.2, 30.1.3.2, 31.1.3.2, 32.1.3.2, 33.1.3.2, 34.1.3.2, 35.1.3.2, 36.1.3.2, 37.1.3.2, 38.1.3.2, 39.1.3.2, 40.1.3.2,1.2.3.2, 2.2.3.2, 3.2.3.2, 4.2.3.2, 5.2.3.2, 6.2.3.2, 7.2.3.2, 8.2.3.2, 9.2.3.2, 10.2.3.2, 11.2.3.2, 12.2.3.2, 13.2.3.2, 14.2.3.2, 15.2.3.2, 16.2.3.2, 17.2.3.2, 18.2.3.2, 19.2.3.2, 20.2.3.2, 21.2.3.2, 22.2.3.2, 23.2.3.2, 24.2.3.2, 25.2.3.2, 26.2.3.2, 27.2.3.2, 28.2.3.2, 29.2.3.2, 30.2.3.2, 31.2.3.2, 32.2.3.2, 33.2.3.2, 34.2.3.2, 35.2.3.2, 36.2.3.2, 37.2.3.2, 38.2.3.2, 39.2.3.2, 40.2.3.2, 1.3.3.2, 2.3.3.2, 3.3.3.2, 4.3.3.2, 5.3.3.2, 6.3.3.2, 7.3.3.2, 8.3.3.2, 9.3.3.2, 10.3.3.2, 11.3.3.2, 12.3.3.2, 13.3.3.2, 14.3.3.2, 15.3.3.2, 16.3.3.2, 17.3.3.2, 18.3.3.2, 19.3.3.2, 20.3.3.2, 21.3.3.2, 22.3.3.2, 23.3.3.2, 24.3.3.2, 25.3.3.2, 26.3.3.2, 27.3.3.2, 28.3.3.2, 29.3.3.2, 30.3.3.2, 31.3.3.2, 32.3.3.2, 33.3.3.2, 34.3.3.2, 35.3.3.2, 36.3.3.2, 37.3.3.2, 38.3.3.2, 393.3.2, 40.3.3.2, 1.4.3.2, 2.4.3.2, 3.4.3.2, 4.4.3.2, 5.4.3.2, 6.4.3.2, 7.4.3.2, 8.4.3.2, 9.4.3.2, 10.4.3.2, 11.4.3.2, 12.4.3.2, 13.4.3.2, 14.4.3.2, 15.4.3.2, 16.4.3.2, 17.4.3.2, 18.4.3.2, 19.4.3.2, 20.4.3.2, 21.4.3.2, 22.4.3.2, 23.4.3.2, 24.4.3.2, 25.4.3.2, 26.4.3.2, 27.4.3.2, 28.4.3.2, 29.4.3.2, 30.4.3.2, 31.4.3.2, 32.4.3.2, 33.4.3.2, 34.4.3.2, 35.4.3.2, 36.4.3.2, 37.4.3.2, 38.4.3.2, 39.4.3.2, 40.4.3.2, 1.5.3.2, 2.5.3.2, 3.5.3.2, 4.5.3.2, 5.5.3.2, 6.5.3.2, 7.5.3.2, 8.5.3.2, 9.5.3.2, 10.5.3.2, 11.5.3.2, 12.5.3.2, 13.5.3.2, 14.5.3.2, 15.5.3.2, 16.5.3.2, 17.5.3.2, 18.5.3.2, 19.5.3.2, 20.5.3.2, 21.5.3.2, 22.5.3.2, 23.5.3.2, 24.5.3.2, 25.5.3.2, 26.5.3.2, 27.5.3.2, 28.5.3.2, 29.5.3.2, 30.5.3.2, 31.5.3.2, 32.5.3.2, 33.5.3.2, 34.5.3.2, 35.5.3.2, 36.5.3.2, 37.5.3.2, 38.5.3.2, 39.5.3.2, 40.5.3.2, 1.6.3.2, 2.6.3.2, 3.6.3.2, 4.6.3.2, 5.6.3.2, 6.6.3.2, 7.6.3.2, 8.6.3.2, 9.6.3.2, 10.6.3.2, 11.6.3.2, 12.6.3.2, 13.6.3.2, 14.6.3.2, 15.6.3.2, 16.6.3.2, 17.6.3.2, 18.6.3.2, 19.6.3.2, 20.6.3.2, 21.6.3.2, 22.6.3.2, 23.6.3.2, 24.6.3.2, 25.6.3.2, 26.6.3.2, 27.6.3.2, 28.6.3.2, 29.6.3.2, 30.6.3.2, 31.6.3.2, 32.6.3.2, 33.6.3.2, 34.6.3.2, 35.6.3.2, 36.6.3.2, 37.6.3.2, 38.6.3.2, 39.6.3.2, 40.6.3.2, 1.7.3.2, 2.7.3.2, 3.7.3.2, 4.7.3.2, 5.7.3.2, 6.7.3.2, 7.73.2, 8.7.31, 9.73.2, 10.7.3.2, 11.7.3.2, 12.7.3.2, 13.7.3.2, 14.7.3.2, 15.7.3.2, 16.7.3.2, 17.7.3.2,18.7.3.2, 19.7.3.2, 20.7.3.2, 21.7.3.2, 22.7.3.2, 23.7.3.2, 24.7.3.2, 25.7.3.2, 26.7.3.2, 27.7.3.2, 28.7.3.2, 29.7.3.2, 30.7.3.2, 31.7.3.2, 32.7.3.2, 33.7.3.2, 34.7.3.2, 35.7.3.2, 36.7.3.2, 37.7.3.2, 38.7.3.2, 39.7.3.2, 40.7.3.2, 1.8.3.2, 2.8.3.2, 3.8.3.2, 4.8.3.2, 5.8.3.2, 6.8.3.2, 7.8.3.2, 8.8.3.2, 9.8.3.2, 10.8.3.2, 11.8.3.2, 12.8.3.2, 13.8.3.2, 14.8.3.2, 15.8.3.2, 16.8.3.2, 17.8.3.2, 18.8.3.2, 19.8.3.2, 20.8.3.2, 21.8.3.2, 22.8.3.2, 23.8.3.2, 24.8.3.2, 25.8.3.2, 26.8.3.2, 27.8.3.2, 28.8.3.2, 29.8.3.2, 30.8.3.2, 31.8.3.2, 32.8.3.2, 33.8.3.2, 34.8.3.2, 35.8.3.2, 36.8.3.2, 37.8.3.2, 38.8.3.2, 39.8.3.2, 40.8.3.2,1.9.3.2, 2.9.3.2, 3.9.3.2, 4.9.3.2, 5.9.3.2, 6.9.3.2, 7.9.3.2, 8.9.3.2, 9.9.3.2, 10.9.3.2, 11.9.3.2, 12.9.3.2, 13.9.3.2, 14.9.3.2, 15.9.3.2, 16.9.3.2, 17.9.3.2, 18.9.3.2, 19.9.3.2, 20.9.3.2, 21.9.3.2, 22.9.3.2, 23.9.3.2, 24.9.3.2, 25.9.3.2, 26.9.3.2, 27.9.3.2, 28.9.3.2, 29.9.3.2, 30.9.3.2, 31.9.3.2, 32.9.3.2, 33.9.3.2, 34.9.3.2, 35.9.3.2, 36.93.2, 37.9.3.2, 38.9.3.2, 39.9.3.2, 40.9.3.2, 1.10.3.2, 2.10.3.2, 3.10.3.2, 4.10.3.2, 5.10.3.2, 6.10.3.2, 7.10.3.2, 8.10.3.2, 9.10.3.2, 10.10.3.2, 11.10.3.2, 12.10.3.2, 13.10.3.2, 14.10.3.2, 15.10.3.2, 16.10.3.2, 17.10.3.2, 18.10.3.2, 19.10.3.2, 20.10.3.2, 21.10.3.2, 22.10.3.2, 23.10.3.2, 24.10.3.2, 25.10.3.2, 26.10.3.2, 27.10.3.2, 28.10.3.2, 29.10.3.2, 30.10.3.2, 31.10.3.2, 32.10.3.2, 33.10.3.2, 34.10.3.2, 35.10.3.2, 36.10.3.2, 37.10.3.2, 38.10.3.2, 39.10.3.2, 40.10.3.2, 1.11.3.2, 2.11.3.2, 3.11.3.2, 4.11.3.2, 5.11.3.2, 6.11.3.2, 7.11.3.2, 8.11.3.2, 9.11.3.2, 10.11.3.2, 11.11.3.2, 12.11.3.2, 13.11.3.2, 14.11.3.2, 15.11.3.2, 16.11.32, 17.11.3.2, 18.11.3.2, 19.11.3.2, 20.11.3.2, 21.11.3.2, 22.11.3.2, 23.11.3.2, 24.11.3.2, 25.11.3.2, 26.11.3.2, 27.11.3.2, 28.11.3.2, 29.11.3.2, 30.11.3.2, 31.11.3.2, 32.11.3.2, 33.11.3.2, 34.11.3.2, 35.11.3.2, 36.11.3.2, 37.11.3.2, 38.11.3.2, 39.11.3.2, 40.11.3.2, 1.12.3.2, 2.12.3.2, 3.12.3.2, 4.12.3.2, 5.12.3.2, 6.12.3.2, 7.12.3.2, 8.12.3.2, 9.12.3.2, 10.12.3.2, 11.12.3.2, 12.12.3.2, 13.12.3.2, 14.12.3.2, 15.12.3.2, 16.12.3.2, 17.12.3.2, 18.12.3.2, 19.123.2, 20.12.3.2, 21.12.3.2, 22.12.3.2, 23.12.3.2, 24.12.3.2, 25.12.3.2, 26.12.3.2, 27.12.3.2, 28.12.3.2, 29.12.3.2, 30.12.32, 31.12.3.2, 32.12.3.2, 33.12.3.2, 34.12.3.2, 35.12.3.2, 36.12.3.2, 37.12.3.2, 38.12.3.2, 39.12.3.2, 40.12.32,1.13.3.2, 2.13.3.2, 3.13.3.2, 4.13.3.2, 5.13.3.2, 6.13.3.2, 7.13.3.2, 8.13.3.2, 9.13.3.2, 10.13.3.2, 11.13.3.2, 12.13.3.2, 13.13.3.2, 14.13.3.2, 15.13.3.2, 16.13.3.2, 17.13.3.2, 18.13.3.2, 19.13.3.2, 20.13.3.2, 21.13.3.2, 22.133.2, 23.13.3.2, 24.133.2, 25.13.3.2, 26.13.3.2, 27.13.3.2, 28.13.3.2, 29.13.3.2, 30.13.3.2, 31.13.3.2, 32.13.3.2, 33.13.3.2, 34.13.3.2, 35.13.3.2, 36.13.3.2, 37.13.3.2, 38.13.3.2, 39.13.3.2, 40.13.3.2, 1.14.3.2, 2.14.3.2, 3.14.3.2, 4.14.3.2, 5.14.3.2, 6.14.3.2, 7.14.3.2, 8.14.3.2, 9.14.3.2, 10.14.3.2, 11.14.3.2, 12.14.3.2, 13.14.3.2, 14.14.3.2, 15.14.3.2, 16.14.3.2, 17.14.3.2, 18.14.3.2, 19.14.3.2, 20.14.3.2, 21.14.3.2, 22.14.3.2, 23.14.3.2, 24.14.3.2, 25.14.3.2, 26.14.3.2, 27.14.3.2, 28.14.3.2, 29.14.3.2, 30.14.3.2, 31.14.3.2, 32.14.3.2, 33.14.3.2, 34.14.3.2, 35.14.3.2, 36.14.3.2, 37.14.3.2, 38.14.3.2, 39.14.3.2, 40.14.3.2, 1.15.3.2, 2.15.3.2, 3.15.3.2, 4.15.3.2, 5.15.3.2, 6.15.3.2, 7.15.3.2, 8.15.3.2, 9.15.3.2, 10.15.3.2, 11.15.3.2, 12.15.3.2, 13.15.3.2, 14.15.3.2, 15.15.3.2, 16.15.3.2, 17.15.3.2, 18.15.3.2, 19.15.3.2, 20.15.3.2, 21.15.3.2, 22.15.3.2, 23.15.3.2, 24.15.3.2, 25.15.3.2, 26.15.3.2, 27.15.3.2, 28.15.3.2, 29.15.3.2, 30.15.3.2, 31.15.3.2, 32.15.3.2, 33.15.3.2, 34.15.3.2, 35.15.3.2, 36.15.3.2, 37.15.3.2, 38.15.3.2, 39.15.3.2, 40.15.3.2,1.16.3.2, 2.16.3.2, 3.16.3.2, 4.16.3.2, 5.16.3.2, 6.16.3.2, 7.16.3.2, 8.16.3.2, 9.16.3.2, 10.16.3.2, 11.16.3.2, 12.16.3.2, 13.16.3.2, 14.16.3.2, 15.16.3.2, 16.16.3.2, 17.16.3.2, 18.16.3.2, 19.16.3.2, 20.16.3.2, 21.16.3.2, 22.16.3.2, 23.16.3.2, 24.16.3.2, 25.16.3.2, 26.16.3.2, 27.16.3.2, 28.16.3.2, 29.16.3.2, 30.16.3.2, 31.16.3.2, 32.16.3.2, 33.16.3.2, 34.16.3.2, 35.16.3.2, 36.16.3.2, 37.16.3.2, 38.16.3.2, 39.16.3.2, 40.16.3.2, 1.17.3.2, 2.17.3.2, 3.17.3.2, 4.17.3.2, 5.17.3.2, 6.17.3.2, 7.17.3.2, 8.17.3.2, 9.17.3.2,10.17.3.2,11.17.3.2,12.17.3.2,13.17.3.2, 14.17.3.2, 15.17.3.2, 16.17.3.2, 17.17.3.2, 18.17.3.2, 19.17.3.2, 20.17.3.2, 21.17.3.2, 22.17.3.2, 23.17.3.2, 24.17.3.2, 25.17.3.2, 26.17.3.2, 27.17.3.2, 28.17.3.2, 29.17.3.2, 30.17.3.2, 31.17.3.2, 32.17.3.2, 33.17.3.2, 34.17.3.2, 35.17.3.2, 36.17.3.2, 37.17.3.2, 38.17.3.2, 39.17.3.2, 40.17.3.2,1.18.3.2, 2.18.3.2, 3.18.3.2, 4.18.3.2, 5.18.3.2, 6.18.3.2, 7.18.3.2, 8.18.3.2, 9.18.3.2, 10.18.3.2, 11.18.3.2, 12.18.3.2, 13.18.3.2, 14.18.3.2, 15.18.3.2, 16.18.3.2, 17.18.3.2, 18.18.3.2, 19.18.3.2, 20.18.3.2, 21.18.3.2, 22.18.3.2, 23.18.3.2, 24.18.3.2, 25.18.3.2, 26.18.3.2, 27.18.3.2, 28.18.3.2, 29.18.3.2, 30.18.3.2, 31.18.3.2, 32.18.3.2, 33.18.3.2, 34.18.3.2, 35.18.3.2, 36.18.3.2, 37.18.3.2, 38.18.3.2, 39.18.3.2, 40.18.3.2,1.19.3.2, 2.19.3.2, 3.19.3.2, 4.19.3.2, 5.19.3.2, 6.19.3.2, 7.19.3.2, 8.19.3.2, 9.19.3.2, 10.19.3.2, 11.19.3.2, 12.19.3.2, 13.193.2, 14.19.3.2, 15.193.2, 16.19.3.2, 17.19.3.2, 18.19.3.2, 19.19.3.2, 20.19.3.2, 21.19.3.2, 22.19.3.2, 23.19.3.2, 24.19.3.2, 25.19.3.2, 26.19.3.2, 27.19.3.2, 28.19.3.2, 29.19.3.2, 30.19.3.2, 31.19.3.2, 32.19.3.2, 33.19.3.2, 34.19.3.2, 35.19.3.2, 36.19.3.2, 37.19.3.2, 38.19.3.2, 39.19.3.2, 40.19.3.2, 1.20.3.2, 2.20.3.2, 3.20.3.2, 4.20.3.2, 5.20.3.2, 6.20.3.2, 7.20.3.2, 8.20.3.2, 9.20.3.2, 10.20.3.2, 11.20.3.2, 12.20.3.2, 13.20.3.2, 14.20.3.2, 15.20.3.2, 16.20.3.2, 17.20.3.2, 18.20.3.2, 19.20.3.2, 20.20.3.2, 21.20.3.2, 22.20.3.2, 23.20.3.2, 24.20.3.2, 25.20.3.2, 26.20.3.2, 27.20.3.2, 28.20.3.2, 29.20.3.2, 30.20.3.2, 31.20.3.2, 32.20.3.2, 33.20.3.2, 34.20.3.2, 35.20.3.2, 36.20.3.2, 37.20.3.2, 38.20.3.2, 39.20.3.2, 40.20.3.2, 1.21.3.2, 2.21.3.2, 3.21.3.2, 4.21.3.2, 5.21.3.2, 6.21.3.2, 7.21.3.2, 8.21.3.2, 9.21.3.2, 10.21.3.2, 11.213.2, 12.21.3.2, 13.21.312, 14.21.3.2, 15.21.3.2, 16.21.3.2, 17.21.3.2, 18.21.3.2, 19.21.3.2, 20.21.3.2, 21.21.3.2, 22.21.3.2, 23.21.3.2, 24.21.3.2, 25.21.3.2, 26.21.3.2, 27.21.3.2, 28.21.3.2, 29.21.3.2, 30.21.3.2, 31.21.3.2, 32.21.3.2, 33.21.3.2, 34.21.3.2, 35.21.3.2, 36.21.3.2, 37.21.3.2, 38.21.3.2, 39.21.3.2, 40.21.3.2, 1.22.3.2, 2.22.3.2, 3.22.3.2, 4.22.3.2, 5.22.3.2, 6.22.3.2, 7.22.3.2, 8.22.3.2, 9.22.3.2,10.22.3.2, 11.22.3.2, 12.22.3.2, 13.22.3.2, 14.22.3.2, 15.22.3.2, 16.223.2, 17.22.3.2, 18.22.3.2, 19.22.3.2, 20.22.3.2, 21.22.3.2, 22.22.3.2, 23.22.3.2, 24.22.3.2, 25.22.3.2, 26.22.3.2, 27.22.3.2, 28.22.3.2, 29.22.3.2, 30.22.3.2, 31.22.3.2, 32.22.3.2, 33.22.3.2, 34.22.3.2, 35.22.3.2, 36.22.3.2, 37.22.3.2, 38.22.3.2, 39.22.3.2, 40.22.3.2, 1.23.3.2, 2.23.3.2, 3.23.3.2, 4.23.3.2, 5.23.3.2, 6.23.3.2, 7.23.3.2, 8.23.3.2, 9.23.3.2, 10.23.3.2, 11.23.3.2, 12.23.3.2, 13.23.3.2, 14.23.3.2, 15.233.2, 16.23.3.2, 17.23.3.2, 18.23.3.2, 19.23.3.2, 20.23.3.2, 21.23.3.2, 22.23.3.2, 23.23.3.2, 24.23.3.2, 25.23.3.2, 26.23.3.2, 27.23.3.2, 28.23.3.2, 29.23.3.2, 30.23.3.2, 31.23.3.2, 32.23.3.2, 33.23.3.2, 34.23.3.2, 35.23.3.2, 36.23.3.2, 37.23.3.2, 38.23.3.2, 39.23.3.2, 40.23.3.2, 1.24.3.2, 2.24.3.2, 3.24.3.2, 4.24.3.2, 5.24.3.2, 6.24.3.2, 7.24.3.2, 8.24.3.2, 9.24.3.2, 10.24.3.2, 11.24.3.2, 12.24.3.2, 13.24.3.2, 14.24.3.2, 15.24.3.2, 16.24.3.2, 17.24.3.2, 18.24.3.2, 19.24.3.2, 20.24.3.2, 21.24.3.2, 22.24.3.2, 23.24.3.2, 24.24.3.2, 25.24.3.2, 26.24.3.2, 27.24.3.2, 28.24.3.2, 29.24.3.2, 30.24.3.2, 31.24.3.2, 32.24.3.2, 33.24.3.2, 34.24.3.2, 35.24.3.2, 36.24.3.2, 37.24.3.2, 38.24.3.2, 39.24.3.2, 40.24.3.2,1.25.3.2, 2.25.3.2, 3.25.3.2, 4.25.3.2, 5.25.3.2, 6.25.3.2, 7.25.3.2, 8.25.3.2, 9.25.3.2, 10.25.3.2, 11.25.3.2, 12.25.3.2, 13.25.3.2, 14.253.2, 15.25.3.2, 16.25.3.2, 17.25.3.2, 18.25.3.2, 19.25.3.2, 20.25.3.2, 21.25.3.2, 22.25.3.2, 23.25.3.2, 24.25.3.2, 25.25.3.2, 26.25.3.2, 27.25.3.2, 28.25.3.2, 29.25.3.2, 30.25.3.2, 31.25.3.2, 32.25.3.2, 33.25.3.2, 34.25.3.2, 35.25.3.2, 36.25.3.2, 37.25.3.2, 38.25.3.2, 39.25.3.2, 40.25.3.2, 1.26.3.2, 2.26.3.2, 3.26.3.2, 4.26.3.2, 5.26.3.2, 6.26.3.2, 7.26.3.2, 8.26.3.2, 9.26.3.2, 10.26.3.2, 11.26.3.2, 12.26.3.2, 13.263.2, 14.26.3.2, 15.26.3.2, 16.26.3.2, 17.26.3.2, 18.26.31, 19.26.3.2, 20.26.3.2, 21.26.3.2, 22.26.3.2, 23.26.3.2, 24.26.3.2, 25.26.3.2, 26.26.3.2, 27.26.3.2, 28.26.3.2, 29.26.3.2, 30.26.3.2, 31.26.3.2, 32.26.3.2, 33.26.3.2, 34.26.3.2, 35.26.3.2, 36.26.3.2, 37.26.3.2, 38.26.3.2, 39.26.3.2, 40.26.3.2, 1.27.3.2, 2.27.3.2, 3.27.3.2, 4.27.3.2, 5.27.3.2, 6.27.3.2, 7.27.3.2, 8.27.3.2, 9.27.3.2, 10.27.3.2, 11.27.3.2, 12.27.3.2, 13.27.3.2, 14.27.3.2, 15.27.3.2, 16.27.3.2, 17.27.3.2, 18.27.3.2, 19.27-3.2, 20.27.3.2, 21.27.3.2, 22.27.3.2, 23.27.3.2, 24.27.3.2, 25.27.3.2, 26.27.3.2, 27.27.3.2, 28.27.3.2, 29.27.3.2, 30.27.3.2, 31.27.3.2, 32.27.3.2, 33.27.3.2, 34.27.3.2, 35.27.3.2, 36.27.3.2, 37.27.3.2, 38.27.3.2, 39.27.3.2, 40.27.3.2, 1.28.3.2, 2.28.3.2, 3.28.3.2, 4.28.3.2, 5.28.3.2, 6.28.3.2, 7.28.3.2, 8.28.3.2, 9.28.3.2, 10.28.3.2, 11.28.3.2, 12.28.3.2, 13.28.3.2, 14.28.3.2, 15.28.3.2, 16.28.3.2, 17.28.3.2, 18.28.3.2, 19.28.3.2, 20.28.3.2, 21.28.3.2, 22.28.3.2, 23.28.3.2, 24.28.3.2, 25.283.2, 26.28.3.2, 27.28.3.2, 28.28.3.2, 29.28.3.2, 30.28.3.2, 31.28.3.2, 32.28.3.2, 33.28.3.2, 34.28.3.2, 35.28.3.2, 36.28.3.2, 37.28.3.2, 38.28.3.2, 39.28.3.2, 40.28.3.2, 1.29.3.2, 2.29.3.2, 3.29.3.2, 4.29.3.2, 5.29.3.2, 6.29.3.2, 7.29.3.2, 8.29.3.2, 9.29.3.2, 10.29.3.2, 11.29.3.2, 12.29.3.2, 13.29.3.2, 14.29.3.2, 15.29.3.2, 16.29.3.2, 17.29.3.2, 18.29.3.2, 19.29.3.2, 20.29.3.2, 21.29.3.2, 22.29.3.2, 23.29.3.2, 24.29.3.2, 25.29.3.2, 26.29.3.2, 27.29.3.2, 28.29.3.2, 29.29.3.2, 30.29.3.2, 31.29.3.2, 32.29.3.2, 33.29.3.2, 34.29.3.2, 35.29.32, 36.29.3.2, 37.29.3.2, 38.29.3.2, 39.29.3.2, 40.29.3.2, 1.1.3.4, 2.1.3.4, 3.1.3.4, 4.1.3.4, 5.1.3.4, 6.1.3.4, 7.1.3.4, 8.1.3.4, 9.1.3.4, 10.1.3.4, 11.1.3.4, 12.1.3.4, 13.1.3.4, 14.1.3.4, 15.1.3.4, 16.1.3.4, 17.1.3.4, 18.1.3.4, 19.1.3.4, 20.1.3.4, 21.1.3.4, 22.1.3.4, 23.1.3.4, 24.1.3.4, 25.1.3.4, 26.1.3.4, 27.1.3.4, 28.1.3.4, 29.1.3.4, 30.1.3.4, 31.1.3.4, 32.1.3.4, 33.1.3.4, 34.1.3.4, 35.1.3.4, 36.1.3.4, 37.1.3.4, 38.1.3.4, 39.1.3.4, 40.1.3.4, 1.2.3.4, 2.2.3.4, 3.2.3.4, 4.2.3.4, 5.2.3.4, 6.2.3.4, 7.2.3.4, 8.2.3.4, 9.2.3.4,10.2.3.4, 11.2.3.4, 12.2.3.4, 13.2.3.4, 14.2.3.4, 15.2.3.4, 16.2.3.4, 17.2.3.4, 18.2.3.4, 19.2.3.4, 20.2.3.4, 21.2.3.4, 22.2.3.4, 23.2.3.4, 24.2.3.4, 25.2.3.4, 26.2.3.4, 27.2.3.4, 28.2.3.4, 29.2.3.4, 30.2.3.4, 31.2.3.4, 32.2.3.4, 33.2.3.4, 34.2.3.4, 35.2.3.4, 36.2.3.4, 37.2.3.4, 38.2.3.4, 39.2.3.4, 40.2.3.4,1.3.3.4, 2.3.3.4, 3.3.3.4, 4.3.3.4, 5.3.3.4, 6.3.3.4, 7.3.3.4, 8.3.3.4, 9.3.3.4, 10.3.3.4, 11.3.3.4, 12.3.3.4, 13.3.3.4, 14.3.3.4, 15.3.3.4, 16.3.3.4, 17.3.3.4, 18.3.3.4, 19.3.3.4, 20.3.3.4, 21.3.3.4, 22.3.3.4, 23.3.3.4, 24.3.3.4, 25.3.3.4, 26.3.3.4, 27.3.3.4, 28.3.3.4, 29.3.3.4, 30.3.3.4, 31.3.3.4, 32.3.3.4, 33.3.3.4, 34.3.3.4, 35.3.3.4, 36.3.3.4, 37.3.3.4, 38.3.3.4, 39.3.3.4, 40.3.3.4, 1.4.3.4, 2.4.3.4, 3.4.3.4, 4.4.3.4, 5.4.3.4, 6.4.3.4, 7.4.3.4, 8.4.3.4,9.4.3.4, 10.4.3.4, 11.4.3.4, 12.4.3.4, 13.4.3.4, 14.4.3.4, 15.4.3.4, 16.4.3.4, 17.4.3.4, 18.4.3.4, 19.4.3.4, 20.4.3.4, 21.4.3.4, 22.4.3.4, 23.4.3.4, 24.4.3.4, 25.4.3.4, 26.4.3.4, 27.4.3.4, 28.4.3.4, 29.4.3.4, 30.4.3.4, 31.4.3.4, 32.4.3.4, 33.4.3.4, 34.4.3.4, 35.4.3.4, 36.4.3.4, 37.4.3.4, 38.4.3.4, 39.4.3.4, 40.4.3.4, 1.5.3.4, 2.5.3.4, 3.5.3.4, 4.5.3.4, 5.5.3.4, 6.5.3.4, 7.5.3.4, 8.5.3.4, 9.5.3.4, 10.5.3.4, 11.5.3.4, 12.5.3.4, 13.5.3.4, 14.5.3.4, 15.5.3.4, 16.5.3.4, 17.5.3.4, 18.5.3.4, 19.5.3.4, 20.5.3.4, 21.5.3.4, 22.5.3.4, 23.5.3.4, 24.5.3.4, 25.5.3.4, 26.5.3.4, 27.5.3.4, 28.5.3.4, 29.5.3.4, 30.5.3.4, 31.5.3.4, 32.5.3.4, 33.5.3.4, 34.5.3.4, 35.5.3.4, 36.5.3.4, 37.5.3.4, 38.5.3.4, 39.5.3.4, 40.5.3.4, 1.6.3.4, 2.6.3.4, 3.6.3.4, 4.6.3.4, 5.6.3.4, 6.6.3.4, 7.6.3.4, 8.6.3.4, 9.6.3.4, 10.6.3.4, 11.6.3.4, 12.6.3.4, 13.6.3.4, 14.6.3.4, 15.6.3.4, 16.6.3.4, 17.6.3.4, 18.6.3.4,19.6.3.4, 20.6.3.4, 21.6.3.4, 22.6.3.4, 23.6.3.4, 24.6.3.4, 25.6.3.4, 26.6.3.4, 27.6.3.4, 28.6.3.4, 29.6.3.4, 30.6.3.4, 31.6.3.4, 32.6.3.4, 33.6.3.4, 34.6.3.4, 35.6.3.4, 36.6.3.4, 37.6.3.4, 38.6.3.4, 39.6.3.4, 40.6.3.4,1.7.3.4, 2.7.3.4, 3.7.3.4, 4.7.3.4, 5.7.3.4, 6.7.3.4, 7.7.3.4, 8.7.3.4, 9.7.3.4, 10.7.3.4, 11.73.4, 12.7.3.4, 13.7.3.4, 14.7.3.4, 15.7.3.4, 16.7.3.4, 17.7.3.4, 18.7.3.4, 19.7.3.4, 20.7.3.4, 21.7.3.4, 22.7.3.4, 23.7.3.4, 24.7.3.4, 25.7.3.4, 26.7.3.4, 27.7.3.4, 28.7.3.4, 29.7.3.4, 30.7.3.4, 31.7.3.4, 32.7.3.4, 33.7.3.4, 34.7.3.4, 35.7.3.4, 36.7.3.4, 37.7.3.4, 38.7.3.4, 39.7.3.4, 40.7.3.4, 1.8.3.4, 2.8.3.4, 3.8.3.4, 4.8.3.4, 5.8.3.4, 6.8.3.4, 7.8.3.4, 8.8.3.4, 9.8.3.4, 10.8.3.4, 11.8.3.4, 12.8.3.4, 13.8.3.4, 14.8.3.4, 15.8.3.4, 16.8.3.4, 17.8.3.4, 18.8.3.4, 19.8.3.4, 20.8.3.4, 21.8.3.4, 22.8.3.4, 23.8.3.4, 24.8.3.4, 25.8.3.4, 26.8.3.4, 27.8.3.4, 28.8.3.4, 29.8.3.4, 30.8.3.4, 31.8.3.4, 32.8.3.4, 33.8.3.4, 34.8.3.4, 35.8.3.4, 36.8.3.4, 37.8.3.4, 38.8.3.4, 39.8.3.4, 40.8.3.4, 1.9.3.4, 2.9.3.4, 3.9.3.4, 4.9.3.4, 5.9.3.4, 6.9.3.4, 7.9.3.4, 8.9.3.4, 9.9.3.4, 10.9.3.4, 11.9.3.4, 12.9.3.4, 13.9.3.4, 14.9.3.4, 15.9.3.4, 16.9.3.4, 17.9.3.4, 18.9.3.4, 19.9.3.4, 20.9.3.4, 21.9.3.4, 22.9.3.4, 23.9.3.4, 24.9.3.4, 25.9.3.4, 26.9.3.4, 27.9.3.4, 28.9.3.4, 29.9.3.4, 30.9.3.4, 31.9.3.4, 32.9.3.4, 33.9.3.4, 34.9.3.4, 35.9.3.4, 36.9.3.4, 37.9.3.4, 38.9.3.4, 39.9.3.4, 40.9.3.4,1.10.3.4, 2.10.3.4, 3.10.3.4, 4.10.3.4, 5.10.3.4, 6.10.3.4, 7.10.3.4, 8.10.3.4, 9.10.3.4, 10.10.3.4, 11.10.3.4, 12.10.3.4, 13.10.3.4, 14.10.3.4, 15.10.3.4, 16.10.3.4, 17.10.3.4, 18.10.3.4, 19.10:3.4, 20.10.3.4, 21.10.3.4, 22.10.3.4, 23.10.3.4, 24.10.3.4, 25.10.3.4, 26.10.3.4, 27.10.3.4, 28.10.3.4, 29.10.3.4, 30.10.3.4, 31.10.3.4, 32.10.3.4, 33.10.3.4, 34.10.3.4, 35.10.3.4, 36.10.3.4, 37.10.3.4, 38.10.3.4, 39.10.3.4, 40.10.3.4, 1.11.3.4, 2.11.3.4, 3.11.3.4, 4.11.3.4, 5.11.3.4, 6.11.3.4, 7.11.3.4, 8.11.3.4, 9.11.3.4, 10.11.3.4, 11.11.3.4, 12.11.3.4, 13.11.3.4, 14.11.3.4, 15.11.3.4, 16.11.3.4, 17.11.3.4, 18.11.3.4, 19.11.3.4, 20.11.3.4, 21.11.3.4, 22.11.3.4, 23.11.3.4, 24.11.3.4, 25.11.3.4, 26.11.3.4, 27.11.3.4, 28.11.3.4, 29.11.3.4, 30.11.3.4, 31.11.3.4, 32.11.3.4, 33.11.3.4, 34.11.3.4, 35.11.3.4, 36.11.3.4, 37.11.3.4, 38.11.3.4, 39.11.3.4, 40.11.3.4, 1.12.3.4, 2.12.3.4, 3.12.3.4, 4.12.3.4, 5.12.3.4, 6.12.3.4, 7.12.3.4, 8.12.3.4, 9.12.3.4, 10.12.3.4, 11.12.3.4, 12.12.3.4, 13.12.3.4, 14.12.3.4, 15.12.3.4, 16.12.3.4, 17.12.3.4, 18.12.3.4, 19.12.3.4, 20.12.3.4, 21.12.3.4, 22.12.3.4, 23.12.3.4, 24.12.3.4, 25.12.3.4, 26.12.3.4, 27.12.3.4, 28.12.3.4, 29.12.3.4, 30.12.3.4, 31.12.3.4, 32.12.3.4, 33.12.3.4, 34.12.3.4, 35.12.3.4, 36.12.3.4, 37.12.3.4, 38.12.3.4, 39.12.3.4, 40.12.3.4, 1.13.3.4, 2.13.3.4, 3.13.3.4, 4.13.3.4, 5.13.3.4, 6.13.3.4, 7.13.3.4, 8.13.3.4, 9.13.3.4, 10.13.3.4, 11.13.3.4, 12.13.3.4, 13.13.3.4, 14.13.3.4, 15.13.3.4, 16.13.3.4, 17.13.3.4, 18.13.3.4, 19.13.3.4, 20.13.3.4, 21.13.3.4, 22.13.3.4, 23.13.3.4, 24.13.3.4, 25.13.3.4, 26.13.3.4, 27.13.3.4, 28.13.3.4, 29.13.3.4, 30.13.3.4, 31.13.3.4, 32.13.3.4, 33.13.3.4, 34.13.3.4, 35.13.3.4, 36.13.3.4, 37.13.3.4, 38.13.3.4, 39.13.3.4, 40.13.3.4,1.14.3.4, 2.14.3.4, 3.14.3.4, 4.14.3.4, 5.14.3.4, 6.14.3.4, 7.14.3.4, 8.14.3.4, 9.14.3.4, 10.14.3.4, 11.14.3.4, 12.14.3.4, 13.14.3.4, 14.14.3.4, 15.14.3.4, 16.14.3.4, 17.14.3.4, 18.14.3.4, 19.14.3.4, 20.14.3.4, 21.14.3.4, 22.14.3.4, 23.14.3.4, 24.14.3.4, 25.14.3.4, 26.14.3.4, 27.14.3.4, 28.14.3.4, 29.14.3.4, 30.14.3.4, 31.14.3.4, 32.14.3.4, 33.14.3.4, 34.14.3.4, 35.14.3.4, 36.14.3.4, 37.14.3.4, 38.14.3.4, 39.14.3.4, 40.14.3.4, 1.15.3.4, 2.15.3.4, 3.15.3.4, 4.15.3.4, 5.15.3.4, 6.15.3.4, 7.15.3.4, 8.15.3.4, 9.15.3.4, 10.15.3.4, 11.15.3.4, 12.15.3.4, 13.15.3.4, 14.15.3.4, 15.15.3.4, 16.15.3.4, 17.15.3.4, 18.15.3.4, 19.15.3.4, 20.15.3.4, 21.15.3.4, 22.15.3.4, 23.15.3.4, 24.15.3.4, 25.15.3.4, 26.15.3.4, 27.15.3.4, 28.15.3.4, 29.15.3.4, 30.15.3.4, 31.15.3.4, 32.15.3.4, 33.15.3.4, 34.15.3.4, 35.15.3.4, 36.15.3.4, 37.15.3.4, 38.15.3.4, 39.15.3.4, 40.15.3.4,1.16.3.4, 2.16.3.4, 3.16.3.4, 4.16.3.4, 5.16.3.4, 6.16.3.4, 7.16.3.4, 8.16.3.4, 9.16.3.4, 10.16.3.4, 11.16.3.4, 12.16.3.4, 13.16.34, 14.16.3.4, 15.16.3.4, 16.16.3.4, 17.16.3.4, 18.16.3.4, 19.16.3.4, 20.16.3.4, 21.16.3.4, 22.16.3.4, 23.16.3.4, 24.16.3.4, 25.16.3.4, 26.16.3.4, 27.16.3.4, 28.16.3.4, 29.16.3.4, 30.16.3.4, 31.16.3.4, 32.16.3.4, 33.16.3.4, 34.16.3.4, 35.16.3.4, 36.16.3.4, 37.16.3.4, 38.16.3.4, 39.16.3.4, 40.16.3.4, 1.17.3.4, 2.17.3.4, 3.17.3.4, 4.17.3.4, 5.17.3.4, 6.17.3.4, 7.17.3.4, 8.17.3.4, 9.17.3.4, 10.17.3.4, 11.17.3.4, 12.17.3.4, 13.17.3.4, 14.17.3.4, 15.17.3.4, 16.17.3.4, 17.17.3.4, 18.17.3.4, 19.17.3.4, 20.17.3.4, 21.17.3.4, 22.17.3.4, 23.17.3.4, 24.17.3.4, 25.17.3.4, 26.17.3.4, 27.17.3.4, 28.17.3.4, 29.17.3.4, 30.17.3.4, 31.17.3.4, 32.17.3.4, 33.17.3.4, 34.17.3.4, 35.17.3.4, 36.17.3.4, 37.17.3.4, 38.17.3.4, 39.17.3.4, 40.17.3.4, 1.18.3.4, 2.18.3.4, 3.18.3.4, 4.18.3.4, 5.18.3.4, 6.18.3.4, 7.18.3.4, 8.18.3.4, 9.18.3.4, 10.18.3.4, 11.18.3.4, 12.18.3.4, 13.18.3.4, 14.18.3.4,15.18.3.4, 16.18.3.4, 17.18.3.4, 18.18.3.4, 19.18.3.4, 20.18.3.4, 21.18.3.4, 22.18.3.4, 23.18.3.4, 24.18.3.4, 25.18.3.4, 26.18.3.4, 27.18.3.4, 28.18.3.4, 29.18.3.4, 30.18.3.4, 31.18.3.4, 32.18.3.4, 33.18.3.4, 34.18.3.4, 35.18.3.4, 36.18.3.4, 37.18.3.4, 38.18.3.4, 39.18.3.4, 40.18.3.4, 1.19.3.4, 2.19.3.4, 3.19.3.4, 4.19.3.4, 5.19.3.4, 6.19.3.4, 7.19.3.4, 8.19.3.4, 9.19.3.4, 10.19.3.4, 11.19.3.4, 12.19.3.4, 13.19.3.4, 14.19.3.4, 15.19.3.4, 16.19.3.4, 17.19.3.4, 18.19.3.4, 19.19.3.4, 20.19.3.4, 21.19.3.4, 22.19.3.4, 23.19.3.4, 24.19.3.4, 25.19.3.4, 26.19.3.4, 27.19.3.4, 28.19.3.4, 29.19.3.4, 30.19.3.4, 31.19.3.4, 32.19.3.4, 33.19.3.4, 34.19.3.4, 35.19.3.4, 36.19.3.4, 37.19.3.4, 38.19.3.4, 39.19.3.4, 40.19.3.4, 1.20.3.4, 2.20.3.4, 3.20.3.4, 4.20.3.4, 5.20.3.4, 6.20.3.4, 7.20.3.4, 8.20.3.4, 9.20.3.4, 10.20.3.4, 11.20.3.4, 12.20.3.4, 13.20.3.4, 14.20.3.4, 15.20.3.4, 16.20.3.4, 17.20.3.4, 18.20.3.4, 19.20.3.4, 20.20.3.4, 21.20.3.4, 22.20.3.4, 23.20.3.4, 24.20.3.4, 25.20.3.4, 26.20.3.4, 27.20.3.4, 28.20.3.4, 29.20.3.4, 30.20.3.4, 31.20.3.4, 32.20.3.4, 33.20.3.4, 34.20.3.4, 35.20.3.4, 36.20.3.4, 37.20.3.4, 38.20.3.4, 39.20.3.4, 40.20.3.4, 1.213.4, 2.21.3.4, 3.21.3.4, 4.21.3.4, 5.21.3.4, 6.21.3.4, 7.21.3.4, 8.21.3.4, 9.21.3.4, 10.21.3.4, 11.21.3.4, 12.21.3.4, 13.21.3.4, 14.21.3.4, 15.21.3.4, 16.21.3.4, 17.21.3.4, 18.21.3.4, 19.21.3.4, 20.21.3.4, 21.21.3.4, 22.21.3.4, 23.21.3.4, 24.21.3.4, 25.21.3.4, 26.21.3.4, 27.21.3.4, 28.21.3.4, 29.21.3.4, 30.21.3.4, 31.21.3.4, 32.21.3.4, 33.21.3.4, 34.21.3.4, 35.21.3.4, 36.21.3.4, 3721.3.4, 38.21.3.4, 39.21.3.4, 40.21.3.4, 1.22.3.4, 2.22.3.4, 3.22.3.4, 4.22.3.4, 5.22.3.4, 6.22.3.4, 7.22.3.4, 8.22.3.4, 9.22.3.4, 10.22.3.4, 11.22.3.4, 12.22.3.4, 13.22.3.4, 1422.3.4, 15.223.4, 16.22.3.4, 17.22.3.4, 18.22.3.4, 19.22.3.4, 20.22.3.4, 21.22.3.4, 22.22.3.4, 23.22.3.4, 24.22.3.4, 25.22.3.4, 26.22.3.4, 27.22.3.4, 28.22.3.4, 29.22.3.4, 30.22.3.4, 31.22.3.4, 32.22.3.4, 33.22.3.4, 34.22.3.4, 35.22.3.4, 36.22.3.4, 37.22.3.4, 38.22.3.4, 39.22.3.4, 40.22.3.4, 1.23.3.4, 2.23.3.4, 3.23.3.4, 4.23.3.4, 5.23.3.4, 6.23.3.4, 7.23.3.4, 8.23.3.4, 9.23.3.4, 10.23.3.4, 11.23.3.4, 12.23.3.4, 13.23.3.4, 14.23.3.4, 15.23.3.4, 16.23.3.4, 17.23.3.4, 18.23.3.4, 19.23.3.4, 20.23.3.4, 21.23.3.4, 22.23.3.4, 23.23.3.4, 24.23.3.4, 25.23.3.4, 26.23.3.4, 27.23.3.4, 28.23.3.4, 29.23.3.4, 30.23.3.4, 31.23.3.4, 32.23.3.4, 33.23.3.4, 34.23.3.4, 35.23.3.4, 36.23.3.4, 37.23.3.4, 38.23.3.4, 39.23.3.4, 40.23.3.4, 1.24.3.4, 2.24.3.4, 3.24.3.4, 4.24.3.4, 5.24.3.4, 6.24.3.4, 7.24.3.4, 8.24.3.4, 9.24.3.4, 10.24.3.4, 11.24.3.4, 12.24.3.4, 13.24.3.4, 14.24.3.4, 15.24.3.4, 16.24.3.4, 17.24.3.4, 18.24.3.4, 19.24.3.4, 20.24.3.4, 21.24.3.4, 22.24.3.4, 23.24.3.4, 24.24.3.4, 25.24.3.4, 26.24.3.4, 27.24.3.4, 28.24.3.4, 29.24.3.4, 30.24.3.4, 31.24.3.4, 32.24.3.4, 33.24.3.4, 34.24.3.4, 35.24.3.4, 36.24.3.4, 37.24.3.4, 38.24.3.4, 39.24.3.4, 40.24.3.4, 1.25.3.4, 2.25.3.4, 3.25.3.4, 4.25.3.4, 5.25.3.4, 6.25.3.4, 7.25.3.4, 8.25.3.4, 9.25.3.4, 10.25.3.4, 11.25.3.4, 12.25.3.4, 13.25.3.4, 14.25.3.4, 15.253.4, 16.25.3.4, 17.25.3.4, 18.25.3.4, 19.25.3.4, 20.25.3.4, 21.25.3.4, 22.25.3.4, 23.25.3.4, 24.25.3.4, 25.25.3.4, 26.25.3.4, 27.25.3.4, 28.25.3.4, 29.25.3.4, 30.25.3.4, 31.25.3.4, 32.25.3.4, 33.25.3.4, 34.25.3.4, 35.25.3.4, 36.25.3.4, 37.25.3.4, 38.25.3.4, 39.25.3.4, 40.25.3.4, 1.26.3.4, 2.26.3.4, 3.26.3.4, 4.26.3.4, 5.26.3.4, 6.26.3.4, 7.26.3.4, 8.26.3.4, 9.26.3.4, 10.26.3.4, 11.26.3.4, 12.26.3.4, 13.26.3.4, 14.26.3.4, 15.26.3.4, 16.26.3.4, 17.26.3.4, 18.26.3.4, 19.26.3.4, 20.26.3.4, 21.26.3.4, 22.26.3.4, 23.26.3.4, 24.26.3.4, 25.26.3.4, 26.26.3.4, 27.26.3.4, 28.26.3.4, 29.26.3.4, 30.26.3.4, 31.26.3.4, 32.26.3.4, 33.26.3.4, 34.26.3.4, 35.26.3.4, 36.26.3.4, 37.26.3.4, 38.26.3.4, 39.26.3.4, 40.26.3.4, 1.27.3.4, 2.27.3.4, 3.27.3.4, 4.27.3.4, 5.27.3.4, 6.27.3.4, 7.27.3.4, 8.27.3.4, 9.27.3.4, 10.27.3.4, 11.27.3.4, 12.27.3.4, 13.27.3.4, 14.27.3.4, 15.27.3.4, 16.27.3.4, 17.27.3.4, 18.27.3.4, 19.27.3.4, 20.27.3.4, 21.27.3.4, 22.27.3.4, 23.27.3.4, 24.27.3.4, 25.27.3.4, 26.27.3.4, 27.27.3.4, 28.27.3.4, 29.27.3.4, 30.27.3.4, 31.27.3.4, 32.27.3.4, 33.27.3.4, 34.27.3.4, 35.27.3.4, 36.27.3.4, 37.27.3.4, 38.27.3.4, 39.27.3.4, 40.27.3.4, 1.28.3.4, 2.28.3.4, 3.28.3.4, 4.28.3.4, 5.28.3.4, 6.28.3.4, 7.28.3.4, 8.28.3.4, 9.28.3.4,10.28.3.4, 11.28.3.4, 12.28.3.4, 13.28.3.4, 14.28.3.4, 15.28.3.4, 16.28.3.4, 17.28.3.4, 18.28.3.4, 19.28.3.4, 20.28.3.4, 21.28.3.4, 22.28.3.4, 23.28.3.4, 24.28.3.4, 25.28.3.4, 26.28.3.4, 27.28.3.4, 28.28.3.4, 29.28.3.4, 30.28.3.4, 31.28.3.4, 32.28.3.4, 33.28.3.4, 34.28.3.4, 35.28.3.4, 36.28.3.4, 37.28.3.4, 38.28.3.4, 39.28.3.4, 40.28.3.4, 1.29.3.4, 2.29.3.4, 3.29.3.4, 4.29.3.4, 5.29.3.4, 6.29.3.4, 7.29.3.4, 8.29.3.4, 9.29.3.4, 10.29.3.4, 11.29.3.4, 12.29.3.4, 13.29.3.4, 14.29.3.4, 15.29.3.4, 16.29.3.4, 17.29.3.4, 18.29.3.4, 19.29.3.4, 20.29.3.4, 21.29.3.4, 22.29.3.4, 23.29.3.4, 24.29.3.4, 25.29.3.4, 26.29.3.4, 27.29.3.4, 28.29.3.4, 29.29.3.4, 30.29.3.4, 31.29.3.4, 32.29.3.4, 33.29.3.4, 34.29.3.4, 35.29.3.4, 36.29.3.4, 37.29.3.4, 38.29.3.4, 39.29.3.4, 40.29.3.4, 1.1.3.5, 2.1.3.5, 3.1.3.5, 4.1.3.5, 5.1.3.5, 6.1.3.5, 7.1.3.5, 8.1.3.5, 9.1.3.5, 10.1.3.5, 11.1.3.5, 12.1.3.5, 13.1.3.5, 14.1.3.5, 15.1.3.5, 16.1.3.5, 17.1.3.5, 18.1.3.5, 19.1.3.5, 20.1.3.5, 21.1.3.5, 22.1.3.5, 23.1.3.5, 24.1.3.5, 25.1.3.5, 26.1.3.5, 27.1.3.5, 28.1.3.5, 29.1.3.5, 30.1.3.5, 31.1.3.5, 32.1.3.5, 33.1.3.5, 34.1.3.5, 35.1.3.5, 36.1.3.5, 37.1.3.5, 38.1.3.5, 39.1.3.5, 40.1.3.5,1.2.3.5, 2.2.3.5, 3.2.3.5, 4.2.3.5, 5.2.3.5, 6.2.3.5, 7.2.3.5, 8.2.3.5, 9.2.3.5, 10.2.3.5, 11.2.3.5, 12.2.3.5, 13.2.3.5, 14.2.3.5, 15.2.3.5, 16.2.3.5, 17.2.3.5, 18.2.3.5, 19.2.3.5, 20.2.3.5, 21.2.3.5, 22.2.3.5, 23.2.3.5, 24.2.3.5, 25.2.3.5, 26.2.3.5, 27.2.3.5, 28.2.3.5, 29.2.3.5, 30.2.3.5, 31.2.3.5, 32.2.3.5, 33.2.3.5, 34.2.3.5, 35.2.3.5, 36.2.3.5, 37.2.3.5, 38.2.3.5, 39.2.3.5, 40.2.3.5, 1.3.3.5, 2.3.3.5, 3.3.3.5, 4.3.3.5, 5.3.3.5, 6.3.3.5, 7.3.3.5, 8.3.3.5, 9.3.3.5, 10.3.3.5, 11.3.3.5, 12.3.3.5, 13.3.3.5, 14.3.3.5, 15.3.3.5, 16.33.5, 17.3.3.5, 18.3.3.5, 19.3.3.5, 20.3.3.5, 21.3.3.5, 22.3.3.5, 23.3.3.5, 24.3.3.5, 25.3.3.5, 26.3.3.5, 27.3.3.5, 28.3.3.5, 29.3.3.5, 30.3.3.5, 31.3.3.5, 32.3.3.5, 33.3.3.5, 34.3.3.5, 35.3.3.5, 36.3.3.5, 37.3.3.5, 38.3.3.5, 39.3.3.5, 40.3.3.5, 1.4.3.5, 2.4.3.5, 3.4.3.5, 4.4.3.5, 5.4.3.5, 6.4.3.5, 7.4.3.5, 8.4.3.5, 9.4.3.5, 10.4.3.5, 11.4.3.5, 12.4.3.5, 13.4.3.5, 14.4.3.5, 15.4.3.5, 16.4.3.5, 17.4.3.5, 18.4.3.5, 19.4.3.5, 20.4.3.5, 21.4.3.5, 22.4.3.5, 23.4.3.5, 24.4.3.5, 25.4.3.5, 26.4.3.5, 27.4.3.5, 28.4.3.5, 29.4.3.5, 30.4.3.5, 31.4.3.5, 32.4.3.5, 33.4.3.5, 34.4.3.5, 35.4.3.5, 36.4.3.5, 37.4.3.5, 38.4.3.5, 39.4.3.5, 40.4.3.5, 1.5.3.5, 2.5.3.5, 3.5.3.5, 4.5.3.5, 5.5.3.5, 6.5.3.5, 7.5.3.5; 8.5.3.5, 9.5.3.5, 10.5.3.5, 11.5.3.5, 12.5.3.5, 13.5.3.5, 14.5.3.5, 15.5.3.5, 16.5.3.5, 17.5.3.5, 18.5.3.5, 19.5.3.5, 20.5.3.5, 21.5.3.5, 22.5.3.5, 23.5.3.5, 24.5.3.5, 25.5.3.5, 26.5.3.5, 27.5.3.5, 28.5.3.5, 29.5.3.5, 30.5.3.5, 31.5.3.5, 32.5.3.5, 33.5.3.5, 34.5.3.5, 35.5.3.5, 36.5.3.5, 37.5.3.5, 38.5.3.5, 39.5.3.5, 40.5.3.5, 1.6.3.5, 2.6.3.5, 3.6.3.5, 4.6.3.5, 5.6.3.5, 6.6.3.5, 7.6.3.5, 8.6.3.5, 9.6.3.5, 10.6.3.5, 11.6.3.5, 12.6.3.5, 13.6.3.5, 14.6.3.5, 15.6.3.5, 16.6.3.5, 17.6.3.5, 18.6.3.5, 19.6.3.5, 20.6.3.5, 21.6.3.5, 22.6.3.5, 23.6.3.5, 24.6.3.5, 25.6.3.5, 26.6.3.5, 27.6.3.5, 28.6.3.5, 29.6.3.5, 30.6.3.5, 31.6.3.5, 32.6.3.5, 33.6.3.5, 34.6.3.5, 35.6.3.5, 36.6.3.5, 37.6.3.5, 38.6.3.5, 39.6.3.5, 40.6.3.5,1.7.3.5, 2.7.3.5, 3.7.3.5, 4.7.3.5, 5.7.3.5, 6.7.3.5, 7.7.3.5, 8.7.3.5, 9.7.3.5, 10.73.5, 11.7.3.5, 12.7.3.5, 13.7.3.5, 14.7.3.5, 15.73.5, 16.7.3.5, 17.7.3.5, 18.7.3.5, 19.7.3.5, 20.7.3.5, 21.7.3.5, 22.7.3.5, 23.7.3.5, 24.7.3.5, 25.7.3.5, 26.7.3.5, 27.7.3.5, 28.7.3.5, 29.7.3.5, 30.7.3.5, 31.7.3.5, 32.7.3.5, 33.7.3.5, 34.7.3.5, 35.7.3.5, 36.7.3.5, 37.7.3.5, 38.7.3.5, 39.7.3.5, 40.7.3.5, 1.8.3.5, 2.8.3.5, 3.8.3.5, 4.8.3.5, 5.8.3.5, 6.8.3.5, 7.8.3.5, 8.8.3.5, 9.8.3.5, 10.8.3.5, 11.8.3.5, 12.8.3.5, 13.8.3.5, 14.83.5, 15.8.3.5, 16.8.3.5, 17.8.3.5, 18.8.3.5, 19.8.3.5, 20.8.3.5, 21.8.3.5, 22.8.3.5, 23.8.3.5, 24.8.3.5, 25.8.3.5, 26.8.3.5, 27.8.3.5, 28.8.3.5, 29.8.3.5, 30.8.3.5, 31.8.3.5, 32.8.3.5, 33.8.3.5, 34.8.3.5, 35.8.3.5, 36.8.3.5, 37.8.3.5, 38.8.3.5, 39.8.3.5, 40.8.3.5, 1.9.3.5, 2.9.3.5, 3.9.3.5, 4.9.3.5, 5.9.3.5, 6.9.3.5, 7.9.3.5, 8.9.3.5, 9.9.3.5, 10.9.3.5, 11.9.3.5, 12.9.3.5, 13.9.3.5, 14.9.3.5, 15.9.3.5, 16.9.3.5, 17.9.3.5, 18.9.3.5, 19.9.3.5, 20.9.3.5, 21.9.3.5, 22.9.3.5, 23.9.3.5, 24.9.3.5, 25.9.3.5, 26.9.3.5, 27.9.3.5, 28.9.3.5, 29.9.3.5, 30.9.3.5, 31.9.3.5, 32.9.3.5, 33.9.3.5, 34.9.3.5, 35.9.3.5, 36.9.3.5, 37.9.3.5, 38.9.3.5, 39.9.3.5, 40.9.3.5, 1.10.3.5, 2.10.3.5, 3.10.3.5, 4.10.3.5, 5.10.3.5, 6.10.3.5, 7.10.3.5, 8.10.3.5, 9.10.3.5, 10.10.3.5, 11.10.3.5, 12.10.3.5, 13.10.3.5, 14.10.3.5, 15.10.3.5, 16.10.3.5,17.10.3.5,18.10.3.5,19.10.3.5, 20.10.3.5, 21.10.3.5, 22.10.3.5, 23.10.3.5, 24.10.3.5, 25.10.3.5, 26.10.3.5, 27.10.3.5, 28.10.3.5, 29.10.3.5, 30.10.3.5, 31.10.3.5, 32.10.3.5, 33.10.3.5, 34.10.3.5, 35.10.3.5, 36.10.3.5, 37.10.3.5, 38.10.3.5, 39.10.3.5, 40.10.3.5, 1.11.3.5, 2.11.3.5, 3.11.3.5, 4.11.3.5, 5.11.3.5, 6.11.3.5, 7.11.3.5, 8.11.3.5, 9.11.3.5, 10.11.3.5, 11.11.3.5, 12.11.3.5, 13.11.3.5, 14.11.3.5, 15.11.3.5, 16.11.3.5, 17.11.3.5, 18.11.3.5, 19.11.3.5, 20.11.3.5, 21.11.3.5, 22.11.3.5, 23.11.3.5, 24.11.3.5, 25.11.3.5, 26.11.3.5, 27.11.3.5, 28.11.3.5, 29.11.3.5, .30.11.3.5, 31.11.3.5, 32.11.3.5, 33.11.3.5, 34.11.3.5, 35.11.3.5, 36.11.3.5, 37.11.3.5, 38.11.3.5, 39.11.3.5, 40.11.3.5, 1.12.3.5, 2.12.3.5, 3.12.3.5, 4.12.3.5, 5.12 .3.5, 6.12.3.5, 7.12.3.5, 8.12.3.5, 9.12.3.5, 10.12.3.5, 11.12.3.5, 12.12.3.5, 13.12.3.5, 14.12.3.5, 15.12.3.5, 16.12.3.5, 17.123.5, 18.12.3.5, 19.12.3.5, 20.12.3.5, 21.12.3.5, 22.12.3.5, 23.12.3.5, 24.12.3.5, 25.12.3.5, 26.12.3.5, 27.12.3.5, 28.12.3.5, 29.12.3.5, 30.12.3.5, 31.12.3.5, 32.12.3.5, 33.12.3.5, 34.12.3.5, 35.123.5, 36.12.3.5, 37.12.3.5, 38.12.3.5, 39.12.3.5, 40.12.3.5, 1.13.3.5, 2.13.3.5, 3.13.3.5, 4.13.3.5, 5.13.3.5, 6.13.3.5, 7.13.3.5, 8.13.3.5, 9.13.3.5, 10.13.3.5, 11.13.3.5, 12.13.3.5, 13.13.3.5, 14.13.3.5, 15.13.3.5, 16.13.3.5, 17.13.3.5, 18.13.3.5, 19.13.3.5, 20.13.3.5, 21.13.3.5, 22.13.3.5, 23.13.3.5, 24.13.3.5, 25.13.3.5, 26.13.3.5, 27.13.3.5, 28.13.3.5, 29.13.3.5, 30.13.3.5, 31.13.3.5, 32.13.3.5, 33.13.3.5, 34.13.3.5, 35.13.3.5, 36.13.3.5, 37.13.3.5, 38.13.3.5, 39.13.3.5, 40.13.3.5,1.14.3.5, 2.14.3.5, 3.14.3.5, 4.14.3.5, 5.14.3.5, 6.14.3.5, 7.14.3.5, 8.14.3.5, 9.14.3.5,10.14.3.5,11.14.3.5, 12.14.3.5, 13.14.3.5, 14.14.3.5, 15.14.3.5, 16.14.3.5, 17.14.3.5, 18.14.3.5, 19.14.3.5, 20.14.3.5, 21.14.3.5, 22.14.3.5, 23.14.3.5, 24.14.3.5, 25.143.5, 26.14.3.5, 27.14.3.5, 28.14.3.5, 29.14.3.5, 30.14.3.5, 31.14.3.5, 32.14.3.5, 33.14.3.5, 34.14.3.5, 35.14.3.5, 36.14.3.5, 37.14.3.5, 38.14.3.5, 39.14.3.5, 40.14.3.5, 1.15.3.5, 2.15.3.5, 3.15.3.5, 4.15.3.5, 5.15.3.5, 6.15.3.5, 7.15.3.5, 8.15.3.5, 9.15.3.5, 10.15.3.5, 11.15.3.5, 12.15.3.5, 13.15.3.5, 14.15.3.5, 15.15.3.5, 16.15.3.5, 17.15.3.5, 18.15.3.5, 19.153.5, 20.15.3.5, 21.15.3.5, 22.15.3.5, 23.15.3.5, 24.15.3.5, 25.15.3.5, 26.15.3.5, 27.15.3.5, 28.15.3.5, 29.15.3.5, 30.15.3.5, 31.15.3.5, 32.15.3.5, 33.15.3.5, 34.15.3.5, 35.153.5, 36.15.3.5, 37.15.3.5, 38.15.3.5, 39.15.3.5, 40.15.3.5,1.16.3.5, 2.16.3.5, 3.16.3.5, 4.16.3.5, 5.16.3.5, 6.16.3.5, 7.16.3.5, 8.16.3.5, 9.16.3.5, 10.16.3.5, 11.16.3.5, 12.16.3.5, 13.16.3.5, 14.16.3.5, 15.16.3.5, 16.16.3.5, 17.16.3.5, 18.16.3.5, 19.16.3.5, 20.16.3.5, 21.16.3.5, 22.16.3.5, 23.16.3.5, 24.16.3.5, 25.16.3.5, 26.16.3.5, 27.16.3.5, 28.16.3.5, 29.16.3.5, 30.16.3.5, 31.16.3.5, 32.16.3.5, 33.16.3.5, 34.16.3.5, 35.16.3.5, 36.16.3.5, 37.16.3.5, 38.16.3.5, 39.16.3.5, 40.16.3.5, 1.17.3.5, 2.17.3.5, 3.17.3.5, 4.17.3.5, 5.17.3.5, 6.17.3.5, 7.17.3.5, 8.17.3.5, 9.17.3.5, 10.17.3.5, 11.17.3.5, 12.17.3.5, 13.17.3.5, 14.17.3.5, 15.17.3.5, 16.17.3.5,17.17.3.5,18.17.3.5,19.17.3.5, 20.17.3.5, 21.17.3.5, 22.17.3.5, 23.17.3.5, 24.17.3.5, 25.17.3.5, 26.17.3.5, 27.17.3.5, 28.17.3.5, 29.17.3.5, 30.17.3.5, 31.17.3.5, 32.17.3.5, 33.17.3.5, 34.17.3.5, 35.17.3.5, 36.17.3.5, 37.17.3.5, 38.17.3.5, 39.17.3.5, 40.17.3.5, 1.18.3.5, 2.18.3.5, 3.18.3.5, 4.18.3.5, 5.18.3.5, 6.18.3.5, 7.18.3.5, 8.18.3.5, 9.18.3.5, 10.18.3.5, 11.18.3.5, 12.18.3.5, 13.18.3.5, 14.18.3.5, 15.18.3.5, 16.18.3.5, 17.18.3.5, 18.18.3.5, 19.18.3.5, 20.18.3.5, 21.18.3.5, 22.18.3.5, 23.18.3.5, 24.18.3.5, 25.18.3.5, 26.18.3.5, 27.18.3.5, 28.18.3.5, 29.18.3.5, 30.18.3.5, 31.18.3.5, 32.18.3.5, 33.18.3.5, 34.18.3.5, 35.18.3.5, 36.18.3.5, 37.18.3.5, 38.18.3.5, 39.18.3.5, 40.18.3.5, 1.19.3.5, 2.19.3.5, 3.19.3.5, 4.19.3.5, 5.19.3.5, 6.19.3.5, 7.19.3.5, 8.19.3.5, 9.19.3.5, 10.19.3.5, 11.19.3.5, 12.19.3.5, 13.19.3.5, 14.19.3.5, 15.19.3.5, 16.19.3.5, 17.19.3.5, 18.19.3.5, 19.19.3.5, 20.19.3.5, 21.19.3.5, 22.19.3.5, 23.19.3.5, 24.19.3.5, 25.19.3.5, 26.19.3.5, 27.19.3.5, 28.19.3.5, 29.19.3.5, 30.19.3.5, 31.19.3.5, 32.19.3.5, 33.19.3.5, 34.19.3.5, 35.19.3.5, 36.19.3.5, 37.19.3.5, 38.19.3.5, 39.19.3.5, 40.19.3.5, 1.20.3.5, 2.20.3.5, 3.20.3.5, 4.20.3.5, 5.20.3.5, 6.20.3.5, 7.20.3.5, 8.20.3.5, 9.20.3.5, 10.20.3.5, 11.20.3.5, 12.20.3.5, 13.20.3.5, 14.20.3.5, 15.20.3.5, 16.20.3.5, 17.20.3.5, 18.20.3.5, 19.20.3.5, 20.20.3.5, 21.20.3.5, 22.20.3.5, 23.20.3.5, 24.20.3.5, 25.20.3.5, 26.20.3.5, 27.20.3.5, 28.20.3.5, 29.20.3.5, 30.20.3.5, 31.20.3.5, 32.20.3.5, 33.20.3.5, 34.20.3.5, 35.20.3.5, 36.20.3.5, 37.20.3.5, 38.20.3.5, 39.20.3.5, 40.20.3.5, 1.21.3.5, 2.21.3.5, 3.21.3.5, 4.21.3.5, 5.21.3.5, 6.21.3.5, 7.21.3.5, 8.21.3.5, 9.21.3.5, 10.213.5, 11.21.3.5, 12.21.3.5, 13.21.3.5, 14.21.3.5, 15.21.3.5, 16.21.3.5, 17.21.3.5, 18.21.3.5, 19.21.3.5, 20.21.3.5, 21.21.3.5, 22.21.3.5, 23.21.3.5, 24.21.3.5, 25.21.3.5, 26.21.3.5, 27.21.3.5, 28.21.3.5, 29.21.3.5, 30.21.3.5, 31.21.3.5, 32.21.3.5, 33.21.3.5, 34.21.3.5, 35.21.3.5, 36.21.3.5, 37.21.3.5, 38.21.3.5, 39.21.3.5, 40.21.3.5, 1.22.3.5, 2.22.3.5, 3.22.3.5, 4.22.3.5, 5.22.3.5, 6.22.3.5, 7.22.3.5, 8.22.3.5, 9.22.3.5, 10.22.3.5, 11.22.3.5, 12.22.3.5, 13.22.3.5, 14.22.3.5, 15.22.3.5, 16.22.3.5, 17.22.3.5, 18.22.3.5, 19.22.3.5, 20.22.3.5, 21.22.3.5, 22.22.3.5, 23.22.3.5, 24.22.3.5, 25.22.3.5, 26.22.3.5, 27.22.3.5, 28.22.3.5, 29.22.3.5, 30.22.3.5, 31.22.3.5, 32.22.3.5, 33.22.3.5, 34.22.3.5, 35.22.3.5, 36.22.3.5, 37.22.3.5, 38.22.3.5, 39.22.3.5, 40.22.3.5, 1.23.3.5, 2.23.3.5, 3.23.3.5, 4.23.3.5, 5.23.3.5, 6.23.3.5, 7.23.3.5, 8.23.3.5, 9.23.3.5, 10.23.3.5, 11.23.3.5, 12.23.3.5, 13.23.3.5, 14.23.3.5, 15.23.3.5, 16.23.3.5, 17.23.3.5, 18.23.3.5, 19.23.3.5, 20.23.3.5, 21.23.3.5, 22.23.3.5, 23.23.3.5, 24.23.3.5, 25.23.3.5, 26.23.3.5, 27.23.3.5, 28.23.3.5, 29.23.3.5, 30.23.3.5, 31.23.3.5, 32.23.3.5, 33.23.3.5, 34.23.3.5, 35.23.3.5, 36.23.3.5, 37.23.3.5, 38.23.3.5, 39.23.3.5, 40.23.3.5, 1.24.3.5, 2.24.3.5, 3.24.3.5, 4.24.3.5, 5.24.3.5, 6.24.3.5, 7.24.3.5, 8.24.3.5, 9.24.3.5, 10.24.3.5, 11.24.3.5, 12.24.3.5, 13.24.3.5, 14.24.3.5, 15.24.3.5, 16.24.3.5, 17.24.3.5, 18.24.3.5, 19.24.3.5, 20.24.3.5, 21.24.3.5, 22.24.3.5, 23.24.3.5, 24.24.3.5, 25.24.3.5, 26.24.3.5, 27.24.3.5, 28.24.3.5, 29.24.3.5, 30.24.3.5, 31.24.3.5, 32.24.3.5, 33.24.3.5, 34.24.3.5, 35.24.3.5, 36.24.3.5, 37.24.3.5, 38.24.3.5, 39.24.3.5, 40.24.3.5, 1.25.3.5, 2.25.3.5, 3.25.3.5, 4.25.3.5, 5.25.3.5, 6.25.3.5, 7.25.3.5, 8.25.3.5, 9.25.3.5, 10.25.3.5, 11.25.3.5, 12.25.3.5, 13.25.3.5, 14.25.3.5, 15.25.3.5, 16.25.3.5, 17.25.3.5, 18.25.3.5, 19.25.3.5, 20.25.3.5, 21.25.3.5, 22.25.3.5, 23.25.3.5, 24.25.3.5, 25.25.3.5, 26.25.3.5, 27.25.3.5, 28.25.3.5, 29.25.3.5, 30.25.3.5, 31.25.3.5, 32.25.3.5, 33.25.3.5, 34.25.3.5, 35.25.3.5, 36.25.3.5, 37.25.3.5, 38.25.3.5, 39.25.3.5, 40.25.3.5, 1.26.3.5, 2.26.3.5, 3.26.3.5, 4.26.3.5, 5.26.3.5, 6.26.3.5, 7.26.3.5, 8.26.3.5, 9.26.3.5, 10.26.3.5, 11.26.3.5, 12.26.3.5, 3.26.3.5, 14.26.3.5, 15.26.3.5, 16.26.3.5, 17.263.5, 18.26-3.5, 19.26.3.5, 20.26.3.5, 21.26.3.5, 22.26.3.5, 23.26.3.5, 24.26.3.5, 25.26.3.5, 26.26.3.5, 27.26.3.5, 28.26.3.5, 29.26.3.5, 30.26.3.5, 31.26.3.5, 32.26.3.5, 33.26.3.5, 34.26.3.5, 35.26.3.5, 36.26.3.5, 37.26.3.5, 38.26.3.5, 39.26.3.5, 40.26.3.5, 1.27.3.5, 2.27.3.5, 3.27.3.5, 4.27.3.5, 5.27.3.5, 6.27.3.5, 7.27.3.5, 8.27.3.5, 9.27.3.5, 10.27.3.5, 11.27.3.5, 12.27.3.5, 13.27.3.5, 14.27.3.5, 15.27.3.5, 16.27.3.5, 17.27.3.5, 18.27.3.5, 19.27.3.5, 20.27.3.5, 21.27.3.5, 22.27.3.5, 23.27.3.5, 24.27.3.5, 25.27.3.5, 26.27.3.5, 27.27.3.5, 28.27.3.5, 29.27.3.5, 30.27.3.5, 31.27.3.5, 32.27.3.5, 33.27.3.5, 34.27.3.5, 35.27.3.5, 36.27.3.5, 37.27.3.5, 38.27.3.5, 39.27.3.5, 40.27.3.5, 1.28.3.5, 2.28.3.5, 3.28.3.5, 4.28.3.5, 5.28.3.5, 6.28.3.5, 7.28.3.5, 8.28.3.5, 9.28.3.5, 10.28.3.5, 11.28.3.5, 12.28.3.5, 13.28.3.5, 14.28.3.5, 15.28.3.5, 16.28.3.5, 17.28.3.5, 18.28.3.5, 19.28.3.5, 20:28.3.5, 21.28.3.5, 22.28.3.5, 23.28.3.5, 24.28.3.5, 25.28.3.5, 26.28.3.5, 27.28.3.5, 28.28.3.5, 29.28.3.5, 30.28.3.5, 31.28.3.5, 32.28.3.5, 33.28.3.5, 34.28.3.5, 35.28.3.5, 36.28.3.5, 37.28.3.5, 38.28.3.5, 39.28.3.5, 40.28.3.5,1.29.3.5, 2.29.3.5, 3.29.3.5, 4.29.3.5, 5.29.3.5, 6.29.3.5, 7.29.3.5, 8.293.5, 9.29.3.5, 10.29.3.5, 11.29.3.5, 12.29.3.5, 13.29.3.5, 14.29.3.5, 15.29.3.5, 16.29.3.5, 17.29.3.5, 18.29.3.5, 19.29.3.5, 20.29.3.5, 21.29.3.5, 22.29.3.5, 23.29.3.5, 24.29.3.5, 25.29.3.5, 26.29.3.5, 27.29.3.5, 28.29.3.5, 29.29.3.5, 3029.3.5, 31.29.3.5, 32.29.3.5, 33.29.3.5, 34.29.3.5, 35.29.3.5, 36.29.3.5, 37.29.3.5, 38.29.3.5, 39.29.3.5 and 40.293.5.

Identification of Active Precursors. It is desirable to select the amino acid residue or sequence of the invention compounds having one or more peptide bonds, such as formula VII compounds, based on the substrate specificity of esterases and/or carboxypeptidases expected to be found within cells where precursor hydrolysis is desired. To the extent that the specificity of these enzymes is unknown, one will screen a plurality of nucleotide analogs or esters until the desired substrate specificity is found. This will be apparent from assay either of the generation of free phosphonate or of antimicrobial activity. One selects compounds that are (i) not hydrolyzed or hydrolyzed comparatively slowly in the upper gut, (ii) gut and cell permeable and (iii) hydrolyzed in the cell cytoplasm and/or systemic circulation. Screens with cells from particular tissues are used to identify precursors that are released in organs susceptible to a target viral or microbial infection, e.g. in the case of liver, precursor drugs capable of hydrolysis in the liver. Other infections, e.g. CMV or HIV, are treated with a precursor that is hydrolyzed at substantially the same rate and to substantially the same degree in all tissues, with no one tissue preferentially hydrolyzing the precursor nucleosides.

The assays used can be those known in the art including intestinal lumen stability, cell permeation, liver homogenate stability and plasma stability assays. These assays are used to determine the bioavailability characteristics of particular active precursors according to routinely used methods.

Therapeutic Indications. The hydrolysis products of the invention compounds have activity against viruses, malignant cells and/or parasitic protozoans. For example, 9-(3-hydroxy-2-phosphonylmethoxypropyl (HPMP) and (2-phosphonylmethoxy)ethyl (PME) analogs of purine (adenine (A), guanine (G), 2,6-diaminopurine (DAP), 2-monoaminopurine (MAP), hypoxanthine (Hx) and pyrimidine (cytosine (C), uracil (U), thymine (T) were evaluated for antiviral properties. (S)-HPMPA, (S)-cyclic HPMPA, (S)-HPMPC, (S)-HPMPG, (S)-HPMPDAP, PMEDAP, PMEG and PMEA were active against herpes simplex virus, type 1 and 2 (HSV-1 and -2). (S)-HPMPA and (S)-cyclic HPMPA were active against varicella zoster virus (VZV). (S)-HPMPC was active against human cytomegalovirus (HCMV). (S)-HPMPA and (S)-cyclic HPMPA were shown to be active against adenovirus and vaccinia virus. PMEA, PMEDAP, and PMEMAP are active against human immunodeficiency virus (HIV).

Acyclic nucleotide analogs having a common PME side chain covalently linked to a purine or pyrimidine heterocyclic base were prepared and tested for in vivo antiviral activity against retroviruses and herpes viruses. The adenine analog, PMEA, was active in vitro against HIV and Rauscher murine leukemia virus (R-MuLV), and was more potent in vivo than 3'-azido-3'-deoxythymidine (AZT) in the treatment of R-MuLV in mice. PMEA also had a significant antiviral effect in vivo against murine cytomegalovirus (MCMV), and in vitro activity against HCMV. The guanine analog, PMEG, was active in vitro against herpes viruses. In vivo, PMEG was >50-fold more potent than acyclovir against HSV 1 infection in mice.

(S)-HPMPA has potent and selective activity against a broad spectrum of DNA viruses, including HSV-1 and 2, VZV, thymidine kinase-deficient (TK⁻) mutants of herpes simplex virus, HCMV, phocid herpesvirus type 1 (seal herpesvirus, SeHV), simian herpesvirus type 1 (SHV-1), or pseudorabies virus or Aujeszky's disease virus), bovid herpesvirus type 1 (infectious bovine rhinotracheitis virus, BHV-1), equid herpesvirus type 1 (equine abortion virus, EHV-1), African swine fever (ASF) virus, vaccinia virus; and human adenoviruses, and retroviruses such as murine sarcoma virus (MSV). It is also reported that, in mice and rabbits in vivo, the compound is effective against both local and systemic infections with herpes simplex virus type 1, including herpetic keratitis caused by a TK⁻ mutant which is resistant to the classical antiherpes drugs (DeClercq, E., et al, Antiviral Res (1987) 8:261-272; DeClercq, E., et al, Nature (1986) 322:464-467; Gil-Fernandez, C., et al, Antiviral Res (1987) 7:151-160; Baba, M., et al, Antimicrob Agents Chemother (1987) 31:337-339).

Phosphonylmethoxyalkylpurine analogs have also been evaluated for their antitumor activity in murine tumor models. HPMPA, PMEA, and PMEG were found to be active against intraperitoneal P388 leukemia. PMEG was also found to be active against B16 melanoma.

As indicated above, the compounds of the invention are useful for treatment of microbial infections, for treatment of tumors or for other indications described below. Microbial infections include infection by viruses, parasites, yeasts and fungi. Exemplary viral infections that may be treated include infections mediated by DNA or RNA viruses including herpesviruses (CMV, HSV 1, HSV 2, EBV, varicella zoster virus , bovid herpesvirus type 1, equid herpesvirus type 1), papillomaviruses (HPV types 1-55), flaviviruses (including African swine fever virus and Japanese encephalitis virus), togaviruses (including Venezuelan equine encephalomyelitis virus), influenza viruses (types A-C), retroviruses (HIV 1, HIV 2, HTLV I, HTLV II, SIV, HBV, FeLV, FIV, MoMSV), adenoviruses (types 1-8), poxviruses (vaccinia virus), enteroviruses (polio virus type 1-3, hepatitis A virus), gastroenteritis viruses (Norwalk viruses, rotaviruses), hantaviruses (Hantaan virus), papovaviruses, rhinoviruses, parainfluinza virus types 1-4, rabies virus, and the like.

Some of the phosphonate compounds (such as PMEA) have a broad spectrum of antimicrobial activity and are thus unusual antiviral or antiparasitic agents. The activity of individual nucleotide analogs and nucleotide analog amidates is determined by routine assay of antiviral (or other antimicrobial) activity using enzyme inhibition assays, tissue culture assays, animal model assays and/or other acceptable assays.

Nucleotide analogs (phosphonates such as HPMPC, PMEA, etc) are believed to exert their antimicrobial activity, at least in part, by a two step enzyme-mediated conversion to a diphosphate, followed by incorporation of the diphosphorylated nucleotide analog into nucleic acids. The incorporation of the diphosphates into nucleic acid is mediated by viral or other microbial DNA or RNA polymerases (bacterial, retroviral, etc). Thus, nucleotide analogs (when diphosphorylated) are useful as chain terminators for dideoxynucleotide-type DNA sequencing protocols, provided that the nucleotide analog lacks a free hydroxyl group suitable for polymerase mediated chain elongation. These compounds will not have a hydroxyl group at R²⁷ in compounds of formulas IV and VI or are acyclic. Nucleotide analogs of formula XV, (HO)₂P(O)-O-P(O)(OH)-O-(HO)P(O)-Z-B, can be prepared (Otvos, et al, Nucl Acids Res (1987) 15:1763-1777) and provided in a kit with other reagents (such as klenow polymerase or T4 polymerase, dNTPs, etc) needed for DNA sequencing. The invention nucleotide analogs and nucleotide analog amidates can also be (1) applied to tissue culture systems to eliminate or reduce viral spread or growth during the production of biopharmaceuticals or other products (such as proteins or vaccines), (2) used to eliminate or reduce viral spread or growth in clinical samples (such as blood), and (3) used to stop growth of tissue culture or bacterial cells (using toxic amounts of compound) without interfering with protein production.

Infections mediated by protozoan parasites can be treated using the compounds of the invention. Such infections can also be treated using the corresponding nucleotide analogs of the invention nucleotide analog amidates. The term protozoa is intended to include those members of the subphyla *Sarcomastigophora* and *Sporozoa* of the phylum *Protozoa.* More particularly, the term protozoa as used herein is intended to include those genera of parasitic protozoa which are important to man because they either cause disease in man or in his domestic animals. These genera are for the most part found classified in the superclass *Mastighphora* of the subphylum *Sarcomastigophora* and the class *Telosporea* of the subphylum *Sporozoa* in the classification according to Baker (1969). Illustrative genera of these parasitic protozoa include *Histomonas, Pneumocystis, Trypanosoma, Giardia, Trichomonas, Eimeria, Isopora, Leishmania, Entamoeba, Toxoplasma* and *Plasmodium.* Parasitic protozoans include *Plasmodium falciparum, Plasmodium berghei, Plasmodium malariae, Plasmodium vivax, Leishmania braziliensis, Leishmania donovani, Trypanosoma cruzi, Trypanosoma brucei, Trypanosoma rhodesiense, Pneumocystis carinii, Entamoeba histolytica, Trichomonas vaginalis* and the like (de Vries, E., et al, Mol Biochem Parasitol (1991) 47:43-50). Nucleoside analog amidates of the invention and/or their corresponding nucleotide analogs can also be used to treat yeast or fungal infections caused by *Candida glabrata, Candida tropicalis, Candida albicans,* and other *Candida* species *Cryptococcus* species including *Cryptococcus neoformans, Blastomyces* species including *Blastomyces dermatidis, Torulopsis* species including *Torulopsis glabrata, Coccidioides* species including *Coccidioides immitis, Aspergillus* species and the like.

Pharmaceutical formulations. Compounds of the invention and their physiologically acceptable salts (hereafter collectively referred to as the active ingredients) may be administered by any route appropriate to the condition to be treated, suitable routes including oral, rectal, nasal, topical (including ocular, buccal and sublingual), vaginal and parenteral (including subcutaneous, intramuscular, intravenous, intradermal, intrathecal and epidural). The preferred route of administration may vary with for example the condition of the recipient.

While it is possible for the active ingredients to be administered alone it is preferably to present them as pharmaceutical formulations. The formulations, both for veterinary and for human use, of the present invention comprise at least one active ingredient, as above defined, together with one or more acceptable carriers therefor and optionally other- therapeutic ingredients. The carrier(s) must be "acceptable" in the sense of being compatible with the other ingredients of the formulation and not deleterious to the recipient thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For infections of the eye or other external tissues e.g. mouth and skin, the formulations are preferably applied as a topical ointment or cream containing the active ingredient(s) in an amount of, for example, 0.075 to 20% w/w (including active ingredient(s) in a range between 0.1% and 20% in increments of 0.1% w/w such as 0.6% w/w, 0.7% w/w, etc), preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol (including PEG 400) and mixtures thereof. The topical formulations may desirably include a compound which enhances absorption or penetration of the active ingredient through the skin or other affected areas. Examples of such dermal penetration enhancers include dimethyl sulphoxide and related analogs.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

Emulgents and emulsion stabilizers suitable for use in the formulation of the present invention include Tween^{®} 60, Span^{®} 80, cetostearyl alcohol, benzyl alcohol, myristyl alcohol, glyceryl mono-stearate and sodium lauryl sulfate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns (including particle sizes in a range between 20 and 500 microns in increments of 5 microns such as 30 microns, 35 microns, etc), which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient. Formulations suitable for aerosol administration may be prepared according to conventional methods and may be delivered with other therapeutic agents such as pentamidine for treatment of pneumocystis pneumonia.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described. Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavoring agents.

The present invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

Compounds of the invention can be used to provide controlled release pharmaceutical formulations containing as active ingredient one or more compounds of the invention ("controlled release formulations") in which the release of the active ingredient can be controlled and regulated to allow less frequency dosing or to improve the pharmacokinetic or toxicity profile of a given invention compound. Controlled release formulations adapted for oral administration in which discrete units comprising one or more compounds of the invention can be prepared according to conventional methods. Controlled release formulations may be employed for the treatment or prophylaxis of various microbial infections particularly human bacterial, human parasitic protozoan or human viral infections caused by microbial species including *Plasmodium, Pneumocystis,* herpesviruses (CMV, HSV 1, HSV 2, VZV, and the like), retroviruses, adenoviruses and the like. The controlled release formulations can be used to treat HIV infections and related conditions such as tuberculosis, malaria, pneumocystis pneumonia, CMV retinitis, AIDS, AIDS-related complex (ARC) and progressive generalized lymphadeopathy (PGL), and AIDS-related neurological conditions such as multiple sclerosis, and tropical spastic paraparesis. Other human retroviral infections that may be treated with the controlled release formulations according to the invention include Human T-cell Lymphotropic virus (HTLV)-I and IV and HIV-2 infections.

The invention accordingly provides pharmaceutical formulations for use in the treatment or prophylaxis of the above-mentioned human or vetrinary conditions and microbial infections.

Therapeutic Administration. For each of the above-indicated utilities and indications the amount required of an active ingredient (as above defined) will depend upon a number of factors including the severity of the condition to be treated and the identity of the recipient and will ultimately be at the discretion of the attendant physician or veterinarian. In general however, for each of these utilities and indications, a suitable, effective dose will be in the range 0.1 to 250 mg per kilogram bodyweight of recipient per dose (including active ingredient(s) in a range between 0.1 mg and 250 mg/Kg/dose in increments of 0.5 mg/Kg/dose such as 2.5 mg/Kg/dose, 3.0 mg/Kg/dose, 3.5 mg/Kg/dose, etc), preferably in the range 0.5 to 50 mg per kilogram body weight per dose and most preferably in the range 1 to 15 mg per kilogram body weight per dose; an optimum dose is about 3.0 mg per kilogram body weight per dose. (Unless otherwise indicated all weights of active ingredient are calculated as the parent compound of formula I: for salts thereof the figures would be increased proportionately). The desired dose is preferably presented as one dose or two sub-doses administered at appropriate intervals throughout a period of one to seven days. It is preferred to administer a dose once every 2, 3, 4, 5 or 6 days. The doses may be administered in unit dosage forms. The desired dose is may be presented as one, two, or three sub-doses administered at appropriate intervals throughout the one to seven day period. These sub-doses may be administered in unit dosage form, for example, containing 10 to 1000 mg, and or 100 to 500 mg of active ingredient per unit dosage form. The formulations should be desirably administered to achieve peak plasma concentrations of the active compound of from about 1 to about 100 pM, preferably about 2 to 50 µM, most preferably about 3 to about 30 µM.

Compounds such as those of structures XXXI, XXXII and XXXIII (defined below) will generally (1) have a higher oral bioavailability than the corresponding uncyclized nucleotide analog (e.g., cHPMPC compared to HPMPC) and/or (2) will exibit reduced toxicity when compared with the same dose of the corresponding uncyclized nucleotide analog, and/or (3) will have greater efficacy when compared with the same dose of the corresponding uncyclized nucleotide analog.

The compounds of the invention may be employed in combination with other therapeutic agents for the treatment or prophylaxis of the infections or conditions indicated above. Examples of such further therapeutic agents include agents that are effective for the treatment or prophylaxis of viral, parasitic or bacterial infections or associated conditions or for treatment of tumors or related conditions include 3'-azido-3'-deoxythymidine (zidovudine, AZT), 2'-deoxy-3'-thiacytidine (3TC), 2',3'-dideoxy-2',3'-didehydroadenosine (D4A), 2',3'-dideoxy-2',3'-didehydrothymidine (D4T), carbovir (carbocyclic 2',3'-dideoxy-2',3'-didehydroguanosine), 3'-azido-2',3'-dideoxyuridine, 5-fluorothymidine, (E)-5-(2-bromovinyl)-2'-deoxyuridine (BVDU), 2-chlorodeoxyadenosine, 2-deoxycoformycin, 5-fluorouracil, 5-fluorouridine, 5-fluoro-2'-deoxyuridine, 5-trifluoromethyl-2'-deoxyuridine, 6-azauridine, 5-fluoroorotic acid, methotrexate, triacetyluridine, 1-(2'-deoxy-2'-fluoro-1-β-arabinosyl)-5-iodocytidine (FIAC), tetrahydro-imidazo(4,5,1-jk)-(1,4)-benzodiazepin-2(1H)-thione (TIBO), 2'-nor-cyclicGMP, 6-methoxypurine arabinoside (ara-M), 6-methoxypurine arabinoside 2'-O-valerate, cytosine arabinoside (ara-C), 2',3'-dideoxynucleosides such as 2',3'-dideoxycytidine (ddC), 2',3'-dideoxyadenosine (ddA) and 2',3'-dideoxyinosine (ddI), acyclic nucleosides such as acyclovir, penciclovir, famciclovir, ganciclovir, HPMPC, PMEA, PMEG, PMPA, PMPDAP, FPMPA, HPMPA, HPMPDAP, (2R, 5R)-9-[tetrahydro-5-(phosphonomethoxy)-2-furanyl]adenine, (2R, 5R)-1-[tetrahydro-5-(phosphonomethoxy)-2-furanyl]thymine, other antivirals including ribavirin (adenine arabinoside), 2-thio-6-azauridine, tubercidin, aurintricarboxylic acid, 3-deazaneoplanocin, neoplanocin, rimantidine, adamantine, and foscarnet (trisodium phosphonoformate), antibacterial agents including bactericidal fluoroquinolones (ciprofloxacin, pefloxacin and the like), aminoglycoside bactericidal antibiotics (streptomycin, gentamicin, amicacin and the like) β-lactamase inhibitors (cephalosporins, penicillins and the like), other antibacterials including tetracycline, isoniazid, rifampin, cefoperazone, claithromycin and azithromycin, antiparasite or antifungal agents including pentamidine (1,5-bis(4'-aminophenoxy)pentane), 9-deazainosine, sulfamethoxazole, sulfadiazine, quinapyramine, quinine, fluconazole, ketoconazole, itraconazole, Amphotericin B, 5-fluorocytosine, clotrimazole, hexadecylphosphocholine and nystatin, renal excretion inhibitors such as probenicid, nucleoside transport inhibitors such as dipyridamole, dilazep and nitrobenzylthioinosine, immunomodulators such as FK506, cyclosporin A, thymosin α-1, cytokines including TNF and TGF-β, interferons including IFN-α, IFN-β and IFN-γ, interleukins including interleukin I, II, III, IV, V, VI, VII, VIII, X, XII, XIII macrophage/granulocyte colony stimulating factors including GM-CSF, G-CSF, M-CSF, cytokine antagonists including anti-TNF antibodies, anti-interleukin antibodies, soluble interleukin receptors, protein kinase C inhibitors and the like.

Immunogens and Antibodies. The compounds of this invention, or the biologically active substances produced from these compounds by hydrolysis in vivo, are used as immunogens to prepare antibodies capable of binding specifically to the compounds or their hydrolysis products. The immunogenic compositions therefore are useful as intermediates in the preparation of antibodies for use in diagnostic or quality control assays for the compounds or their hydrolysis products. The antibodies are useful for measuring the presence, absence or amounts of the compounds by any convenient homogenous or heterogenous procedure such as fluorescence polarization immunoassay, fluorescence immunoassay (using fluorescent labels such as fluorescein and the like), radioimmunoassay, enzyme immunoassay (using enzyme indicators such as alkaline phosphatase, horseradish peroxidase, glucose oxidase, urease and the like) and nephelometric inhibition assay by described methods (WO 92/22639). Such assays usually require a tracer (such as a fluorescent or radiolabeled labeled invention compound), an antibody and the sample to be analyzed containing the compound.

The hydrolysis products of interest are the phosphonates resulting from the hydrolysis of the amidate or ester bond(s) of the precursor compounds of this invention, for example HPMPC, 6-aza-HPMPC, cyclic HPMPC, PMEA, PMEG, PMPDAP, PMPA, D4TMPI, D4AMPI, cyclic HPMPA, FPMPA, PMEDAP, PMEMAP, 7-deaza-8-aza-FPMPA, 7-deaza-8-aza-HPMPA, cyclic 7-deaza-8-aza-HPMPA, 7-deaza-8-aza-PMPA, 8-aza-FPMPA, 8-aza-HPMPA, cyclic 8-aza-HPMPA, 8-aza-PMPA, PMPG, PMPMAP, 1-deaza-HPMPA, cyclic 1-deaza-HPMPA, 1-deaza-PMPA, 1-deaza-PMPG, 1-deaza-PMPMAP, I-deaza-PMPDAP, 3-deaza-HPMPA, cyclic 3-deaza-HPMPA or 3-deaza-PMPA. Thus, the antibodies of this invention will be capable of binding to the precursors without binding to the hydrolysis products, will be capable of binding to the hydrolysis products without binding to the precursors, or will be capable of binding specifically to both. The antibodies will not cross-react with naturally-occurring nucleotides or nucleosides. The immunogens of this invention contain the precursor or hydrolytic products in association with an immunogenic substance such as a protein or peptide. Immunogenic substances include adjuvants such as Freund's adjuvant, immunogenic proteins such as viral, bacterial, yeast, plant and animal polypeptides, in particular keyhole limpet hemocyanin, serum albumin, bovine thyroglobulin or soybean trypsin inhibitor, and immunogenic polysaccharides. Typically, the precursor or a compound having the structure of a precursor hydrolytic product is covalently conjugated to an immunogenic polypeptide or polysaccharide by the use of a polyfunctional (ordinarily bifunctional) cross-linking agent. Methods for the manufacture hapten immunogens are conventional per se, and any of the methods used heretofore for conjugating haptens to immunogenic polypeptides or the like are suitably employed here as well, taking into account the functional groups on the precursors or hydrolytic products which are available for cross-linking.

Typically the polypeptide is conjugated to a site on the heterocyclic base functionality of the compound or hydrolysis product rather than to a site on the alkyl or substituted-alkyl phosphonate moiety. In general, the site will be an amino group located on the purine or pyrimidine moiety of the nucleoside phosphonate, at the 5 position of pyrimidines (such as cytosine or uracil), at the 1 position of purines (such as adenosine or guanine) or, for compounds having a cyclic structure corresponding to a sugar or sugar analog and having a free hydroxyl group, through the hydroxyl group (usually at the 3' or 2' positions). Alternatively, the precursor compound is cross-linked through the phosphonate, typically by amidation or esterification of the phosphonate by the polypeptide itself or by a cross-linking functionality covalently bonded to the polypeptide. Thus, the groups L¹ or L² in structures (L¹)(L²)-P(O)-Z-B can be immunogenic proteins (having more than 50 amino acid residues, usually less than 1000 residues) or peptides (about 5 to 50 amino acid residues).

The conjugates are prepared in conventional fashion. For example, N-hydroxysuccinimide, succinic anhydride or alkN=C=Nalk are useful in preparing the conjugates of this invention. The conjugates contain a precursor, its hydrolysis product, or both. Ordinarily, the conjugates will comprise the hydrolysis product, i.e., the biologically active drug. The conjugates are separated from starting materials and byproducts using chromatography or the like, and then are sterile filtered and vialed for storage.

Animals are typically immunized against the immunogenic conjugates or derivatives by combining 1 mg or 1 µg of conjugate (for rabbits or mice, respectively) with 3 volumes of Freund's complete adjuvant and injecting the solution intradermally at multiple sites. One month later the animals are boosted with 1/5 to 1/10 the original amount of conjugate in Freund's complete adjuvant (or other suitable adjuvant) by subcutaneous injection at multiple sites. 7 to 14 days later animals are bled and the serum is assayed for the desired antibody titer. Animals are boosted until the titer plateaus. Preferably, the animal is boosted with the conjugate in which the precursor or product is linked to a different protein, through a different cross-linking agent or both. Optionally, aggregating agents such as alum are used to enhance the immune response.

After immunization, monoclonal antibodies are prepared by recovering immune lymphoid cells (typically spleen cells or lymphocytes from lymph node tissue) from immunized animals and immortalizing the cells in conventional fashion, e.g., by fusion with myeloma cells or by Epstein-Barr virus transformation and screening for clones expressing the desired antibody. The hybridoma technique described originally be Kohler and Milstein, Eur. J. Immunol. (1976) 6:511 has been widely applied to produce hybrid cell lines that secrete high levels of monoclonal antibodies against many specific antigens.

It is possible to fuse cells of one species with another. However, it is preferably that the source of the immunized antibody producing cells and the myeloma be from the same species.

The hybrid cell lines are maintained in culture in vitro. The cell lines of this invention are selected or maintained in a hypoxanthine-aminopterin thymidine (HAT) medium. However, the established hybridoma cell line can be maintained on a variety of nutritionally adequate media. The secreted antibody is recovered from culture by conventional methods such as precipitation, ion exchange chromatography, affinity chromatography, or the like. The antibodies described herein are also recovered from hybridoma cell cultures by conventional methods for purification of IgG or IgM as the case may be that heretofore have been used to purify immunoglobulins from pooled plasma, e.g., ethanol or polyethylene glycol precipitation procedures. The purified antibodies are sterile filtered, and optionally are conjugated to a detectable marker such as an enzyme or spin label for use in diagnostic assays of test samples.

The antibodies of this invention are obtained from any animal species, but ordinarily are murine or rat. Once a monoclonal antibody having the desired specificity and affinity is obtained, other conventional modifications of the antibodies are within the scope of this invention. For example, the complementarity determining regions of an animal antibody, together with as much of the framework domain as is needed, are substituted into an antibody of another animal species or class to produce a cross-class or cross-species chimeric antibody. Fragments or other amino acid sequence variants of monoclonal antibodies also are encompassed within the meaning of antibody as that term is used herein, for example, Fab, Fab' or (Fab')2 fragments, single chain antibodies, bi or polyspecific antibodies, and the like.

The antibodies of this invention are from any suitable class or isotype, e.g. IgG, IgM, IgA, IgD or IgE. They may or may not participate in complement binding or ADCC.

Typically, hybridomas which are capable of binding to the immunogen are screened for the ability to bind to the hapten itself in typical test samples (plasma, serum and the like) with the requisite degree of affinity. The desired affinity will depend upon the use intended for the antibody, but should be adequate to function in a conventional competitive-type ELISA or radioimmunoassays, or in conventional EMIT immunoassays.

The antibodies of this invention are used in such assays together with a labeled from of the precursor or its hydrolytic product. Alternatively, the antibody is labeled. Suitable labels are well-known and include radioisotopes, enzymes, stable free radicals, fluorophors, chemiluminescent moieties and other detectable groups heretofore employed to prepare covalent conjugates for use in assays. Methods for linking the labels to ligand amino groups, or amino acid side chains or termini of polypeptides, are known and are suitable for use herein. Other suitable linking methods will be apparent to the ordinary artisan.

The antibodies and labeled ligands herein optionally are assembled into kits for use in therapeutic drug monitoring or evaluation, or for process quality control, and used in the conventional manner.

Diagnostic applications. Novel compounds described herein are useful as intermediates in the preparation of detectable labels for oligonucleotide probes. The compounds are hydrolyzed to the diacid, diphosphorylated and then incorporated into an oligonucleotide by conventional enzymatic or chemical means. The incorporated heterocyclic base from the invention will generally be capable of participating in heterocyclic base pairing and thus will not interfere substantially with the binding of the oligonucleotide to its complementary sequence (E. DeClerq (1993) 3:85-96); should it interfere with oligonucleotide binding to its complementary sequence, the nucleotide analog is incorporated as the final 3' terminal residue, an innocuous position and a conventional site for oligonucleotide labeling. The nucleotide analog compound in the oligonucleotide is detected by any means, such as NMR, immune, fluorescence or radiolabel detection.

Bis amidate synthesis. Synthesis of bis-phosphoroamidate nucleotide analogs of Formula Id, 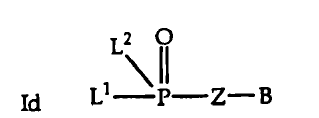 where L¹ and L² are the same and are an amino acid, dipeptide, tripeptide or oligopeptide (4, 5 or 6 amino acid residues) are prepared by conversion of a nucleotide analog (such as PMEA, HPMPC, HPMPA, PMEG, FPMPA, PMPDAP, 9-[2,3-dideoxy-2,3-didehydro-4-phosphonomethoxy-β-D-erythrofuranosyl]adenine (D4AMPI; reg no. 132178-53-1),1-[2,3-dideoxy-2,3-didehydro-4-phosphonomethoxy-β-D-erythrofuranosyl]thymine (D4TMPI; reg no. 132178-49-5) and the like) directly to the corresponding bis-phosphoroamidate compound. L¹ is a protein, an amino acid, dipeptide, tripeptide or oligopeptide (4 to 6 amino acid residues) which is esterified at free α-carboxyl group(s) by R⁴. Suitable R⁴ groups include methyl, ethyl, propyl, isopropyl, butyl, t-butyl, phenyl, benzyl, N-ethylmorpholino, pivaloyloxymethyl and the like. The amino acids can comprise an aliphatic or aromatic side group (such as ala, phe, pro, leu, ile, met, trp and the like) or a dipeptide comprising amino acids having aliphatic or aromatic side groups (such as gly-gly, ala-ala, gly-ala, ala-gly, phe-gly, gly-phe, ala-phe, phe-ala, leu-ala, ala-leu and the like), or is a tripeptide comprising amino acids having aliphatic or aromatic side groups or is an oligopeptide comprising amino acids having aliphatic or aromatic side groups.

The procedure is suitable for all of the nucleotide analogs described herein. The synthesis is accomplished by suspension of the nucleotide analog and approximately 2 equivalents of the L¹ species in a solvent such as dry pyridine or DMF (dimethylformamide) optionally containing a non-nucleophilic organic base such as triethylamine (about 3 to 10 equivalents). The dehydration step is accomplished by modification of a described reaction (Mukaiyama, T. et al, J Am Chem Soc (1972) 94:8528-8532) by adding a 1:1 mixture of triphenylphosphine (reg. no. 603-35-0; Aldrich) and 2,2'-dipyridyl disulfide (2 to 4 equivalents; reg. no. 2127-03-9; Aldrich) in pyridine to the nucleotide analog/amino acid mixture and (a) stirring at room temperature for about 4 to 16 hours or (b) heating to 60° C to 100° C (including any temperature in one degree C increments between 60° and 100° C such as 70°, 80° or 90° C) for about 4 to 16 hours. The resulting reaction mixture is then concentrated and the final bis-amidate product is recovered and purified by conventional methods.

An alternative reaction suitable for synthesizing most amidate compounds is converting a nucleotide analog phosphonate to the corresponding chloridate by reaction with thionyl chloride in solvent (DMF) as described in EP 481 214. An amino acid, dipeptide or other molecule bearing a free amine is then reacted with the chloridate to yield the corresponding bis-amidate.

Synthesis of compounds of Formula Id having amino acids that contain amino, guanidino or carboxyl groups (such as lys, arg, his, asn, gln, lys-lys, arg-arg, lys-arg and the like) is accomplished by the same method, but using protected amine or carboxyl groups. After synthesis of the protected bis-amidate compound, the protecting groups are removed by conventional methods. Suitable protecting groups are well known and include acid labile groups such as p-tosyl, BOC (t-butoxycarbonyl) and FMOC (fluorene methoxycarbonyl) for protecting amine groups. Groups such as t-butyl, methyl, ethyl, benzyl and the like can be used to protect carboxyl groups. These groups can be removed under acid, base or hydrogenolysis conditions or can be removed with an esterase according to conventional methods.

Synthesis of compounds of Formula Id having amino acids such as tyr, cys, ser and thr is accomplished by optionally protecting hydroxyl or thiol groups using protecting groups know in the art. For example, the hydroxyl group of ser, thr or tyr can be protected using benzyl, ethyl and the like and the thiol group of cys can be protected using trityl, p-methylbenzyl and the like. The choice of a protecting group will depend on the stability of the bis-amidate toward conditions used to remove a particular protecting group. Appropriate protecting groups can be selected or determined by the skilled artisan using routine methods.

Amidate-ester synthesis. Synthesis of mixed amidate-ester nucleotide analog amidates of Formula Id where L¹ is an amino acid ester and L² is a group of the formula OR, SR or OR³¹ is accomplished by conversion of a nucleotide analog (such as PMEA, HPMPC, HPMPA, PMEG, FPMPA, PMPDAP, D4AMPI, D4TMPI and the like) di- or bis-ester to a corresponding mixed ester-phosphoroamidate compound. A bis ester is converted to a mono ester by treatment with a base such as ammonia to remove one ester group. The resulting mono ester is then converted to a mixed amidate-ester as described for synthesis of bis amidate compounds.

Bis ester synthesis. Bis esters of the formula (RO)₂P(O)-Z-B are generally synthesized as described in EP 481214 or as described in Mukaiyama, T. et al, J Am Chem Soc (1972) 94:8528-8532. Dialkyl phosphonate esters are synthesized via conversion of a dichlorophosphonate (chloridate) such as (Cl)₂P(O)-Z-B (Quast, H. et al, Synthesis (1974) 7:489-490; Quast, H. et al, Synthesis (1974) 7:490; Moedritzer, K. et al, Synth Reac Inorg Met - Org Chem (1974) 5:417-27; Moedritzer, K., Chem Abs 82:86340: Stowell, M.H.B., et al Tet Lett (1990) 31:3261-3262) to a corresponding dialkylester (or dialkylamide) by reaction with alcohols (or amines). Monoalkylesters (or mono alkylamides) are obtained by hydrolysis of the disubstituted phosphonate in base (NaOH, KOH and the like). Disubstituted diacyloxyalkyl phosphonates are obtained by reaction of the unsubstituted phosphonate with a substituted chloromethyl ester (R-C(O)-O-CH(R)-Cl). A corresponding monosubstituted acyloxyalkyl phosphonate is obtained by hydrolysis in acid or base.

For synthesis of Z substructures having a free hydroxyl group, such as (RO)₂P(O)-CH₂-O-CH(CH₂OH)-CH₂-, the hydroxyl is, in some cases, protected by a protecting group such as benzyl, acetyl, trityl, dimethoxytrityl and the like.

Bis esters having aryl, substituted aryl, alkyl-aryl or substituted alkyl-aryl (such as phenyl, alkoxyphenyl, benzyl, alkoxybenzyl) are also synthesized as described by reaction of (OH)₂P(O)-B-Z with thionyl chloride and a catalytic amount of DMF in a solvent such as acetonitrile. The resulting dichloridate, P(O)(Cl)₂-Z-B is then reacted with about 4, 5 or 6 equivalents of the sodium or potassium alkoxide or a sodium or potassium aryloxide obtained from reaction with sodium hydride or potassium hydride and the alcohol (such as phenol, benzyl alcohol and the like) in a solvent such as THF or acetonitrile at a reduced temperature (below about -70°C, preferably about -76°C to -78°C).

cHPMPC and the cyclic analogues of other cHPMPs are prepared by a number of methods from the free hydroxy phosphonic acid. These methods include treatment with DCC in DMF, reaction with Vilsmeier's reagent (ClCH=N(CH₃)₂Cl), or methods of phosphate activation known per se. In one embodiment of this invention for the preparation of a cHPMP from the corresponding phosphonate nucleotide analog, the phosphonate is (a) treated with ClCH=N(CH₃)₂Cl to yield the phosphonylchloridate and (b) optionally the phosphonylchoridate is reacted with a nucleophile (preferably at low temperature, e.g. lower than about -20°C) such as an alcohol or amine to produce one of the intermediates described above. In a further step the product of steps (a) or (b) are subject to hydrolysis or protonolysis (typically acid protonolysis) respectively to yield the cHSNA (treatment of the product of step (a)) or its intermediate (treatment of the product of step (b)). Vilsmeier's reagent is advantageously produced *in situ* by combining SOCl₂, PCl₅, POCl₃, COCl₂ or the like with DMF. Advantageously, the product of step (a) is not purified or separated from the reaction mixture before being reacted with the nucleophile, a distinct economic advantage for this synthetic route. The compounds of structure (Ia) and (Va) are readily made from their uncyclized counterparts by the same methods, e.g. treatment with DCC in DMF.

Substituted and unsubstituted alkyl, aryl, heteroaryl, arylalkyl, heteroarylalkyl and other L¹ esters and amidates of cHPMPs typically are made by reacting the appropriate HPMP compound with SOCl₂/DMF to yield the activated phosphonylchloride (see Scheme 1), followed by treatment with the corresponding nucleophile (e.g. alkoxide, phenolate, amine, etc.) to yield the protected intermediate formamidine which is subsequently hydrolyzed to the target compound. Alternatively, esters can also be prepared as depicted in Scheme 2. The N-,O- protected intermediate phosphonate diester is obtained from the three building blocks by known methods. The N- and O- protecting groups are subsequently removed followed by treatment of the phosphonate diester 3 with NaH leading to cyclization yielding target compound 4. A third method for the synthesis of cHSNA esters entails alkylation of the cHSNA using common alkylating agents D¹L (where L is a leaving group) such as alkyl halides, tosylates, diazoalkanes and the like (see Scheme 3). This method is particularly useful for preparing acyloxyalkyl esters by treatment of the cHSNA with the corresponding acyloxyalkylhalide. In an exemplary method for the preparation of acyloxyalkyl esters of cHPMPs, as shown in more detail in Example 12, DCC and R⁴⁵C(O)OCH₂Cl are reacted with the cyclic compound; but in contradistinction with prior methods the stoichiometric proportion of DCC: R⁴⁵C(O)OCH₂Cl, cyclic HPMP is 1-2:1-2:1. Use of such low proportions of reactants lessens side reactions with any exocyclic amino group of B and thereby greatly improves yields. R⁴⁵ is H or is C₃-C₁₂ alkyl which is unsubstituted or substituted by substituents independently selected from the group consisting of OH, O, N and halogen, C₃-C₆ aryl which is unsubstituted or substituted by substituents independently selected from the group consisting of OH, O, N and halogen or C₃-C₉ aryl-alkyl which is unsubstituted or substituted by substituents independently selected from the group consisting of OH, O, N and halogen.

Mixed bis amidate synthesis. Synthesis of compounds of formula Id where L¹ and L² are both amino acids or where L¹ is an amino acid and L² is an amine (NH₂, NHR⁶, N(R⁶)₂) but are not both the same is accomplished by direct conversion as described above for bis amidates followed by separation of the final products. Another method to synthesize mixed bis amidates is amidation of an appropriate phosphonate monoester to give a compound of formula Id, followed by removal of the ester group under conditions that do not remove the first amide. Synthesis of phosphonate monoester compounds has been described (EP 481 214). This compound is then converted to a mixed bis amide by condensation with a second amino acid to yield the final product as described (i.e., using a 1:1 mixture of triphenylphosphine and 2,2'-dipyridyl disulfide).

Mono amidate synthesis. Synthesis of compounds of formula Ib where L¹ is an amino acid and X¹ is O (oxygen) is accomplished essentially as described for bis amidate synthesis using a cyclic nucleotide analog such as cHPMPC (cyclic HPMPC), cHFMPA, cHPMPDAP, cHPMPG and the like. Cyclic HPMP series compounds (cHPMPC, etc) are prepared by direct dehydration of the corresponding HPMP nucleotide analog using DCC (dicyclohexylcarbodiimide) or using 4-morpholino-N,N'-dicyclohexylcarboxamide as described (Ho et al Mol Pharmacol (1992) 41:197-202). The cyclic phosphonate is condensed with an optionally protected amino acid ester in the presence of a 1:1 mixture of triphenylphosphine and 2,2'-dipyridyl disulfide in a suitable solvent such as pyridine or DMF.

Synthesis of formula Ib compounds where X¹ is S is accomplished as shown in Figure 1. Conversion of the six-membered heterocycle to an amidate is accomplished in essentially the same manner as described (i.e., using triphenylphosphine and 2,2'-dipyridyl disulfide).

Synthesis of formula IV compounds where R²⁶ is S and R²⁵ and R²⁹ are O is accomplished as shown in Figure 3. The starting material is synthesized by reaction of thiolacetic acid (Aldrich Cat. No. T3,080-5), bromoacetaldehyde diethyl acetal (Aldrich Cat. No. 12,398-6) and potassium tert-butoxide (Aldrich Cat. No. 15,667-1) in DMF. Synthesis of **1** where R³⁴ is H is accomplished using neat (EtO)₃CH. Synthesis of **1** where R³⁴ is CH₂CN or CF₃ is accomplished using (EtO)₃CH₂CN or (EtO)₃CF₃ in methylene chloride with a catalytic acid (such as p-toluenesulfonic acid). Conversion of the thiaorthoester **1** to the phosphonate **2** is accomplished using an acid such as tosic acid or perchloric acid in catalytic amounts. The resulting bis ester is then converted to a bis amidate in essentially the same manner as described (i.e., using triphenylphosphine and 2,2'-dipyridyl disulfide). Mixed ester-amidate compounds are obtained by removing a single ester from the bis ester using base (NaOH, NH₄OH, etc) as described. The phosphonate **3** is obtained by treatment with a base such as TMSBr or TMSI in a solvent (such as methylene chloride, DMF or acetonitrile) in the presence of lutidine (where R is alkyl, aryl or substituted aryl, acyloxyalkyl such as isopropyl, phenyl, 2-ethoxyphenyl) or by treatment with Pd/C/H₂ (where R is alkaryl or substituted alkaryl such as benzyl and the like). R³⁴ in Figure 3 is H, CF₃ or CH₂CN.

Example 1: Synthesis of phosphonate amidate compounds. The compounds of structural formula Id shown are in Table 6 (bis(glycyl benzyl ester)PMEA (compound Ex 4), bis(alanyl benzyl ester)PMEA (Ex 1), bis(phenylalanyl benzyl ester)PMEA (Ex 5), etc. Compounds Ex 1 - Ex 12 were synthesized by the following procedure. PMEA (Z-B = -CH₂-O-CH₂-CH₂-B, where B is adenin-9-yl) (0.3 g;1.1 mmol) and amino acid ester•HCl (2.2 mmol; Sigma) were suspended in dry pyridine (6 mL) containing triethylamine (0.3 mL; 22.2 mmol), followed by addition to a mixture of freshly prepared triphenylphosphine (3.3 mmol) and 2,2'-dipyridyl disulfide (3.3 mmol) in pyridine (3 mL). The mixture was stirred at room temperature overnight, concentrated and partitioned between methylene chloride and water. The organic solution was dried over MgSO₄, concentrated and purified by flash column chromatography on silica gel.

Ex 14 was synthesized using freshly prepared triphenylphosphine (6.0 mmol) and 2,2'-dipyridyl disulfide (6.0 mmol) in pyridine (20 mL) at room temperature to which PMEA (2.0 mmol) was added. The suspension was stirred for 10 min. and ethyl sarcosine HCl (N-methylglycine HCl ethyl ester; 1.2 g, 8.0 mmol) was added. The suspension was warmed to 90°C and stirred for 24 hours. Crude product was concentrated by rotary evaporation and purified by silica flash chromatography (mobile phase 1% methanol gradient to 20% methanol/80% methylene chloride).

Compound Ex 13 was synthesized in a similar manner using PMEA and phenylalanine N-ethylmorpholino ester.

**TABLE 6**

| Compound | L¹ |
|---|---|
| Ex 1 | -NH-CH(CH₃)-C(O)OCH₂C₆H₅ |
| Ex 2 | -NH-CH(CH₂C₆H₅)-C(O)OCH₂C₆H₅ |
| Ex 3 | -NH-CH(CH₂CH(CH₃)₂)-C(O)OCH₂C₆H₅ |
| Ex 4 | -NH-CH₂-C(O)OCH₂C₆H₅ |
| Ex 5 | -NH-CH(CH₃)-C(O)OC₂H₅ |
| Ex 6 | -NH-CH(CH₂CH(CH₃)₂)-C(O)OC₂H₅ |
| Ex 7 | -NH-CH₂-C(O)OC₂H₅ |
| Ex 8 | -NH-CH(CH₂C₆H₅)-C(O)OC(CH₃)₃ |
| Ex 9 | -NH-CH(CH₂CH(CH₃)₂)-C(O)OC(CH₃)₃ |
| Ex 10 | -NH-CH(CH₃)-C(O)OC(CH₃)₃ |
| Ex 11 | -NH-CH₂-C(O)OC(CH₃)₃ |
| Ex 12 | -NH-CH(CH₂C₆H₅)-C(O)OC₂H₅ |
| Ex 13 | -NH-CH(CH₂C₆H₅)C(O)O-(CH₂)₂-N[(CH₂)₂(CH₂)₂]O |
| Ex 14 | -N(CH₃)-CH₂-C(O)OC₂H₅ |

Example 2: Antiviral activity. Compounds were individually tested for activity against HSV-1 and/or HSV-2. HSV-2 (strain 414-92) was tested using MA 104 cells in the following assay protocol. 96-Well plates were seeded with 1 x 10⁴ MA 104 cells per well using 200 µL minimal essential medium (MEM) containing 10% calf serum per well, and incubated overnight at 37°C. The compounds were dissolved in MEM Earle's Salts without serum. The medium was removed by aspiration and 100 µL MEM Earle's Salts without serum was added to the wells. Serial 3-fold dilutions of the compounds were prepared by serial transfer of 50 µL of medium from wells containing compound to wells lacking compound. The plates were incubated 15 minutes at 37°C followed by addition of 100 PFU/well of virus in MEM Earle's Salts with 2% fetal bovine serum. The plates were then incubated at 37°C for three days until approximately 90% of the cells in virus infected control wells containing no compound were killed. Following incubation, medium was aspirated and the wells were washed with sterile PBS. 100 µL 0.5%·crystal violet in 20% methanol was then added to the wells for 5 minutes, aspirated and the wells were washed two or three times with distilled water. 200µL of 0.01 N HCl was added to the wells and the absorbance of each well at 595 nm was determined. The results, shown in Table 6, were expressed as the IC₅₀, the concentration (µM) that inhibits cell killing mediated by HSV-2 by 50%. IC₅₀ values varied from 2 µM to >100 µM compared to an IC₅₀ for PMEA of 21 µM. Thus, some of the compounds were more active against HSV-1 than PMEA.. The toxicity of the compounds were expressed as the CC₅₀, the concentration that kills 50% of uninfected cells.

The compounds were also tested for activity against the KOS strain of HSV-1 in VERO cells. The results, shown in Table 7, were expressed as the EC₅₀, the concentration (µM) that inhibits cell killing mediated by HSV-2 by 50%. EC₅₀ values varied from 2 µM to >200 µM compared to an EC₅₀ for PMEA of 138 µM. Thus, some of the compounds were more active against HSV-2 than PMEA.

**Table 7**

| | HSV-1 | HSV-2 | |
|---|---|---|---|
| compound | EC₅₀ | IC₅₀ | CC₅₀ |
| Ex 7 | >200 | >100 | >100 |
| Ex 5 | nt* | >100 | >100 |
| Ex 6 | 20 | 33 | >100 |
| Ex 12 | nt | 20 | 80 |
| Ex 11 | >200 | >100 | >100 |
| Ex 10 | >200 | >100 | >100 |
| Ex 9 | 63 | 63 | >100 |
| Ex 8 | 3 | 9 | 20 |
| Ex 4 | nt | 60 | >100 |
| Ex 1 | nt | 20 | >100 |
| Ex 3 | nt | 2 | 30 |
| Ex 2 | nt | 4 | 20 |

| | | | |
|---|---|---|---|
| * nt - not tested | | | |

Example 3: PMFA, monophenyl ester, mono N-ethylmorpholino-phenylalanyl phosphoroamidate. Bis(phenyl)PMEA is selectively hydrolyzed to the monophenyl ester of PMEA using NaOH in THF. The reaction mixture is neutralized with acid (1 N HCl), and the monophenyl PMEA is isolated by filtration. The anhydrous monophenyl PMEA and 2 equivalents of a freshly prepared 1:1 mixture of triphenylphosphine and 2,2'-dipyridyl disulfide in pyridine is condensed with 1 equivalent of phenylalanine N-ethyl-morpholino ester in triethylamine and pyridine to afford the title compound. The title compound is recovered by evaporation of the solvents under reduced pressure and purified by silica gel chromatography.

Example 4: Antiviral activity of PMEA esters. PMEA and PMEA esters were tested for inhibition of cytopathic effects by HSV II in MA 104 cells as described except that CPE was determined after incubation with virus by addition of 100µL XTT, 1 mg/mL in deficient DME containing 25 µM PMF followed by measuring absorbance. The esters tested were bis(POM)PMEA, bis(phenyl)PMEA, monophenylPMEA, bis(3-dimethylaminophenyl)PMEA, bis(3-methoxyphenyl)PMEA, bis(2-carboethoxyphenyl)PMEA, bis(adamantoyl oxymethyl)PMEA, bis(4-fluorophenyl)PMEA and bis(2-ethoxyphenyl)PMEA. All of the compounds tested were active, which indicated that the ester groups were removed, thereby allowing free PMEA to inhibit virus replication and/or cytopathic effects. The IC₅₀ and CC₅₀ of PMEA in the assay was 19.3 µM and 2000 µM respectively and the IC₅₀ and CC₅₀ of bis(POM)PMEA in the assay was 0.5 µM and >10 µM respectively. IC₅₀ values for the mono and bis esters ranged from 1.1 µM to 67.5 µM and the CC₅₀ values ranged from 70 µM to 500 µM.

Example 5: Oral bioavailability of nucleotide analog amidates and PMEA esters. Nucleotide analog amidates and nucleotide analogs are tested for their bioavailabililty when administered to cynomologous (or rhesus) monkeys by oral, subcutaneous or intramuscular routes. Bioavailability is determined by measuring PMEA levels in plasma or urine at different times after administering the drug using radiolabeled (³H, ¹⁴C, etc) compound or, for compounds having adenine, essentially as described (Naesens, et al, Clin Chem (1992) 38:480-485; Russell, et al, I Chromatogr (Netherlands) (1991) 572:321-326). Radiolabeled compounds are obtained commercially (Moravek Biochemicals, Brea, CA) or by standard procedures, such as catalytic hydrogen exchange for ³H labeling. Compounds such as bis(2-ethoxyphenyl)PMEA, bis(2-carboethoxyphenyl)PMEA, bis(O-benzylphenylalanyl)PMEA, bis(3,5-dimethoxyphenyl)PMEA, bis(4-fluorophenyl)PMEA, bis(adamantoyl oxymethyl)PMEA, bis(phenyl)PMEA, bis(3-methoxyphenyl)PMEA are tested for oral bioavailability by administering about 10 - 30 mg/Kg (usually 15 to 25 mg/Kg) containing about 20 - 50 µCi/Kg (usually about 40 µCi/Kg) of radiolabeled compound, followed by withdrawing blood samples at several times after administration (exemplary time points are 0.1, 0.25, 0.5, 1.0, 1.5, 2.0, 2.5, 3.0, 4, 6, 12, 18, 24, 36, 48, 72, 96 hours after administration), obtaining plasma and determining the amount of radiolabeled compound present per volume (about 0.1 - 1.0 mL) of serum. Oral bioavailability of the tested compounds is 2 - 80% (or any value between 2% and 80% in 1% increments), preferably 10 - 80% and more preferably 15 to 80%. The oral bioavailability of bis(POM)PMEA by this type of assay is typically about 25% in monkeys and PMEA is about 2 - 4% (Balzarini et al, Animal Models in AIDS (1990) p. 131-138, Schellekens, H. et al (ed), Elsevier Science Publications, Amsterdam) while nucleotide analog amidates and nucleotide analogs (including mono- and diesters) can have oral bioavailabilities of about 5%, 10%, 15%, 30%, 40%, 50%, 60% or 80%.

Total radioactivity in plasma is determined by mixing about 200 µL of plasma with a scintillation counting cocktail (such as 10 mL of Scinti-Safe plus LSC cocktail) and counting in a scintillation counter (usually for about 5 - 30 minutes). Detailed analysis of the radiochemical composition is accomplished using about 350 µL of plasma, denaturing proteins in the serum (using about 700 µL 0.1% trifluoroacetic acid in acetonitrile for example), drying the resulting sample under reduced pressure, suspending the sample in an appropriate buffer (for example using about 100 µL of 2% acetonitrile in 25 mM potassium phosphate buffer with 10 mM tetrabutyl ammonium hydrogen phosphate (TBAHP), pH 6.0 for HPLC analysis), centrifuging the sample and analyzing the supernatant for individual radiolabeled species by reverse phase HPLC on commercially available columns (The Separation Group, Hesperia, CA; Vydac C18, 5 µm, 250 x 4.6 mm column with an injection volume of about 50 µL and a flow rate of about 1.0 mL/min. at about 35°C using buffer for 2 minutes followed by a linear gradient to about 65% acetonitrile in 25 mM potassium phosphate buffer with 10 mM TBAHP, pH 6.0 over 13 about minutes). Radiolabel detection is accomplished using means such as commercially available radioactive flow detection systems or scintillation counting systems (Packard, Meridian, CT).

Fluorescence detection of PMEA in plasma is accomplished by measuring fluorescence emission (420 nm, with excitation at about 236 nm) with a detector (model F2000, Spectra Physics, San Jose, CA) from the HPLC gradient essentially as described above (2 to 65% acetonitrile). Samples for analysis are prepared from plasma (200 µL) by protein precipitation with TFA (400 µL 0.1% in acetonitrile), drying and conversion of adenine to N6-ethenoadenine in 200 µL of reaction buffer (0.34% chloroacetaldehyde, 100 mM sodium acetate, pH 4.5) for 40 minutes at 95°C followed by HPLC analysis using 50 µL.

## Claims

1. A compound of the formula Id 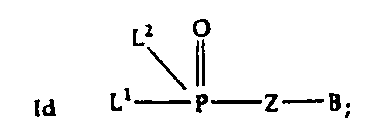 wherein L¹ is of the formula III 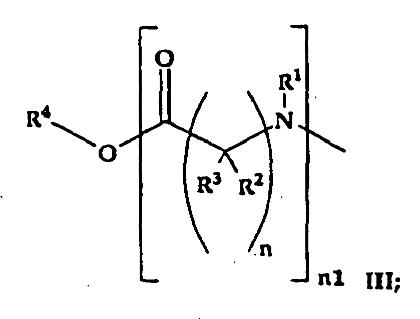 wherein L² is OR, SR or is independently of the formula III;
wherein n and n1 are 1;
R is N-ethylmorpholino, pivaloyloxymethyl, phenyl, benzyl, isopropyl, t-butyl, ethyl, butyl, adamantoyloxymethyl, 3-methoxyphenyl, 2-carboethoxyphenyl, 4-fluorophenyl, 2,4-difluorophenyl, 3,5-dimethoxyphenyl, 2,4-dlchlorophenyl, 2-ethoxyphenyl, 3-dimethylaminophenyl, 4-trifluoromethylbenzyl, 2-ethylsalicyl, -CH₂-O-C(O)-C₁₀H₁₅, -C₆H₄-CH₂-N(CH₃)₂, -CH₂-CH₂F, -CH₂-CH₂Cl, -CH₂-CF₃, -CH₂-CCl₃, R⁵, NHR⁶ or N(R⁶)₂;
wherein,
R⁵ is CH₂C(O)N(R⁶)₂, CH₂C(O)OR⁶, CH₂OC(O)R⁶, CH(R⁶)OC(O)R⁶, CH₂C(R⁶)₂CH₂OH, or CH₂OR⁶, and
R⁶ is C₁-C₂₀ alkyl which is unsubstituted or substituted by substituents independently selected from the group consisting of OH, O, N and halogen (1 to 5 halogen atoms), C₆-C₂₀ aryl which is unsubstituted or substituted by substituents independently selected from the group consisting of OH, O, N and halogen (1 to 5 halogen atoms) or C₇-C₂₀ aryl-alkyl which is unsubstituted or substituted by substituents independently selected from the group consisting of OH, O, N and halogen (1 to 5 halogen atoms);
R¹ is H, methyl, ethyl, isopropyl, phenyl or benzyl;
R² is H;
R³ is H, -CH₃, -CH(CH₃)₂, -CH₂-CH(CH₃)₂, -CHCH₃-CH₂-CH₃, -CH₂-C₆H₅, -CH₂CH₂-S-CH₃, -CH₂OH, -CH(OH)-CH₃, -CH₂-SH, -CH₂-CO-NH₂, -CH₂-CH₂-CO-NH₂, -CH₂-COOH, -CR₂-CH₂-COOH, -(CH₂)₄-NH₂, 1-guanidinoprop-3-yl, benzyl, 4-hydroxybenzyl, imidazol-4-yl, indol-3-yl, methoxyphenyl or ethoxyphenyl;
R⁴ is methyl, ethyl, propyl, isopropyl, butyl, t-butyl, phenyl, benzyl, 1-pyridyl, 3-pyridy1,1-pyrimidinyl, pivaloyloxymethyl, N-ethylmorpholino, N-2-propylmorpholino, methoxyethyl, 4-N-methylpiperidyl, 3-N-methylpiperidyl, 2-, 3-, and 4-N,N-dimethylaminophenyl and 2-, 3-, and 4-N,N-diethylaminaphenyl or 1-ethylplperazinyl;
Z is -CH₂-O-CH₂-CH₂-, or is of formula IV or V 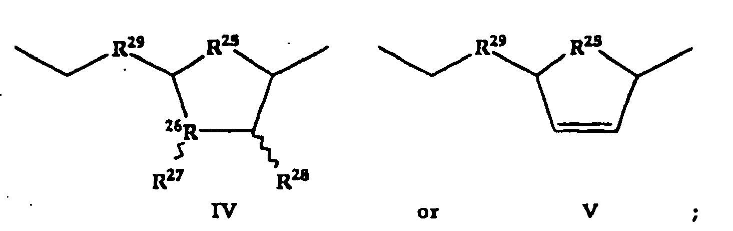 wherein
R²⁵ and R²⁹ are O;
R²⁶ is CH;
R²⁷ and R²⁸ are H; and
B is adenin-9-yl, 1-deazaadenin-9-yl, 3-deazaadenin-9-yl, 7-deaza-8-azaadenin-9-yl, 8-azaadenin-9-yl, guanin-9-yl, 2, 6-diaminopurin-9-yl, 2-aminopurin-9-yl, thymin-1-yl, cytosin-1-yl, 5-fluorocytosin-1-yl, 6-azacytosin-1-yl, 5-methylcytoain-1-yl, 5-bromovinyluracil-1-yl, 5-fluorouracil-1-yl or 5-trifluoromethyluracil-1-yl.

2. A pharmaceutical composition comprising a compound according to Claim 1.

3. A pharmaceutical composition according to Claim 2 for the treatment of a viral infection.

4. A pharmaceutical composition according to Claim 2 or Claim 3 which is formulated for oral administration.

5. A compound according to Claim 1 for the treatment of a viral infection.

6. Use of a compound according to Claim 1 in the preparation of a medicament for the treatment of a viral infection.

7. Use according to Claim 6 wherein the medicament is formulated for oral administration.

## Patentansprüche

1. Verbindung der Formel Id 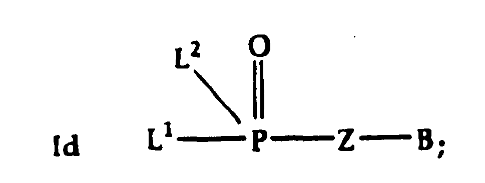 worin L¹ der Formel III entspricht, 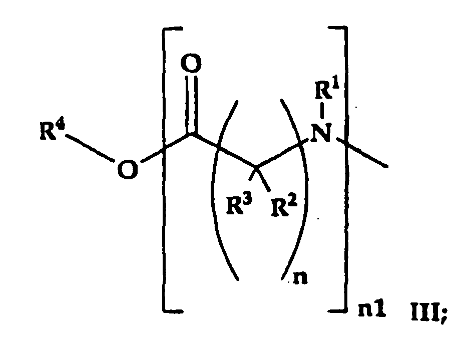 worin L² für OR oder SR steht oder unabhängig der Formel III entspricht;
worin n und n1 gleich 1 sind;
R für N-Ethylmorpholino, Pivaloyloxymethyl, Phenyl, Benzyl, Isopropyl, t-Butyl, Ethyl, Butyl, Adamantoyloxymethyl, 3-Methoxyphenyl, 2-Carboethoxyphenyl, 4-Fluorphenyl, 2,4-Difluorphenyl, 3,5-Dimethoxyphenyl, 2,4-Dichlorphenyl, 2-Ethoxyphenyl, 3-Dimethylaminophenyl, 4-Trifluormethylbenzyl, 2-Ethylsalicyl, - CH₂-O-C(O)-C₁₀H₁₅, -C₆H₄-CH₂-N(CH₃)₂, -CH₂-CH₂F, -CH₂-CH₂Cl, -CH₂-CF₃, - CH₂-CCl₃, R⁵, NHR⁶ oder N(R⁶)₂ steht_{;}
worin
R⁵ für CH₂C(O)N(R⁶)₂, CH₂C(O)OR⁶, CH₂OC(O)R⁶, CH(R⁶)OC(O)R⁶, CH₂C(R ⁶)₂CH₂OH, oder CH₂OR⁶ steht, und
R⁶ für C₁-C₂₀-Alkyl, welches unsubstituiert oder mit Substituenten substituiert ist, die unabhängig voneinander ausgewählt sind aus der Gruppe, bestehend aus OH, O, N und Halogen (1 bis 5 Halogenatome), C₆-C₂₀-Aryl, welches unsubstituiert oder mit Substituenten substituiert ist, die unabhängig voneinander ausgewählt sind aus der Gruppe, bestehend aus OH, O, N und Halogen (1 bis 5 Halogenatome), oder C₇-C₂₀-Arylalkyl, welches unsubstituiert oder mit Substituenten substituiert ist, die unabhängig voneinander ausgewählt sind aus der Gruppe, bestehend aus OH, O, N und Halogen (1 bis 5 Halogenatome), steht;
R¹ für H, Methyl, Ethyl, Isopropyl, Phenyl oder Benzyl steht;
R² für H steht;
R³ für H, -CH₃, -CH(CH₃)₂, -CH₂-CH(CH₃)₂, -CHCH₃-CH₂-CH₃, -CH₂CH₂-S-CH₃, -CH₂OH, -CH(OH)-CH₃, -CH₂-SH, -CH₂-C₆H₄OH, -CH₂-CO-NH₂, -CH₂-CH₂-CO-NH₂, -CH₂-COOH, -CH₂-CH₂-COOH, -(CH₂)₄-NH₂, 1-Guanidinoprop-3-yl, Benzyl, 4-Hydroxybenzyl, Imidazol-4-yl, Indol-3-yl, Methoxyphenyl oder Ethoxyphenyl steht;
R⁴ für Methyl, Ethyl, Propyl, Isopropyl, Butyl, t-Butyl, Phenyl, Benzyl, 1-Pyridyl, 3-Pyridyl, 1-Pyrimidinyl, Pivaloyloxymethyl, N-Ethylmorpholino, N-2-Propylmorpholino, Methoxyethyl, 4-N-Methylpiperidyl, 3-N-Methylpiperidyl, 2-, 3-, und 4-N,N-Dimethylaminophenyl und 2-, 3- und 4-N,N-Diethylaminophenyl oder 1-Ethylpiperazinyl steht;
Z für -CH₂-O-CH₂-CH₂ oder für Formel IV oder V steht; 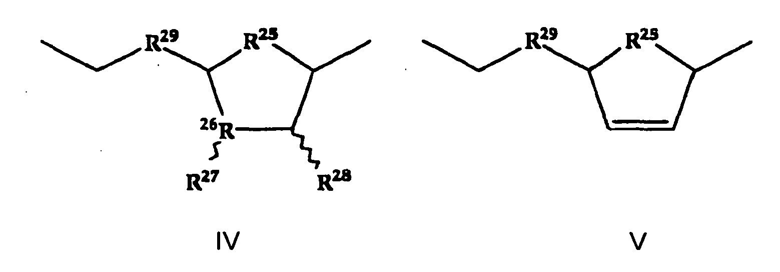 worin
R²⁵ und R²⁹ für O stehen;
R²⁶ für CH steht;
R²⁷ und R²⁸ für H stehen; und
B für Adenin-9-yl, 1-Deazaadenin-9-yl, 3-Deazaadenin-9-yl, 7-Deaza-8-azaadenin-9-yl, 8-Azaadenin-9-yl, Guanin-9-yl, 2,6-Diaminopurin-9-yl, 2-Aminopurin-9-yl, Thymin-1-yl, Cytosin-1-yl, 5-Fluorcytosin-1-yl, 6-Azacytosin-1-yl, 5-Methylcytosin-1-yl, 5-Bromvinyluracil-1-yl, 5-Fluoruracil-1-yl oder 5-Trifluormethyluracil-1-yl steht.

2. Pharmazeutische Zusammensetzung, umfassend eine Verbindung nach Anspruch 1.

3. Pharmazeutische Zusammensetzung nach Anspruch 2 zur Behandlung einer viralen Infektion.

4. Pharmazeutische Zusammensetzung nach Anspruch 2 oder 3, die für die orale Verabreichung formuliert ist.

5. Verbindung nach Anspruch 1 zur Behandlung einer viralen Infektion.

6. Verwendung einer Verbindung nach Anspruch 1 bei der Herstellung eines Medikaments zur Behandlung einer viralen Infektion.

7. Verwendung nach Anspruch 6, wobei das Medikament für die orale Verabreichung formuliert ist.

## Revendications

1. Composé de formule Id : 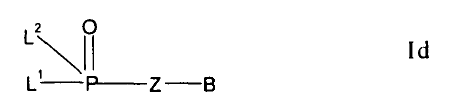 dans laquelle :
L¹ représente un fragment de formule III : 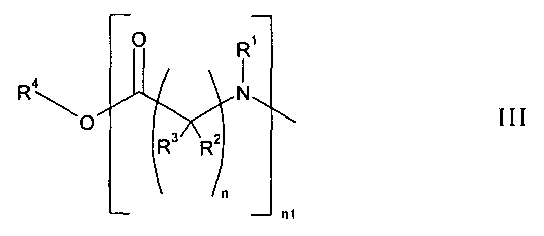
et L² représente un groupe symbolisé par OR ou SR, ou indépendamment, un fragment de formule III ;
dans laquelle formule III les indices n et n1 valent 1 ;
R représente un groupe N-éthyl-morpholinyle, pivaloyloxy-méthyle, phényle, benzyle, isopropyle, tertiobutyle, éthyle, butyle, adamantoyloxy-méthyle, 3-méthoxy-phényle, 2-carbéthoxy-phényle, 4-fluoro-phényle, 2,4-difluoro-phényle, 3,5-diméthoxy-phényle, 2,4-dichloro-phényle, 2-éthoxy-phényle, 3-diméthylamino-phényle, 4-trifluorométhyl-benzyle ou 2-éthyl-salicyle, un groupe de formule -CH₂-O-C(O)-C₁₀H₁₅, -C₆H₄-CH₂-N(CH₃)₂, -CH₂-CH₂F, -CH₂-CH₂Cl, -CH2-CF₃ ou -CH₂-CCl₃, ou un groupe symbolisé par R⁵, NHR⁶ ou N(R⁶)₂,
étant entendu que R⁵ représente un groupe de formule CH₂C(O)N(R⁶)₂, CH₂C(O)OR⁶, CH₂OC(O)R⁶, CH(R⁶)OC(O)R⁶, CH₂C(R⁶)₂CH₂OH ou CH₂OR⁶,
et que R⁶ représente un groupe alkyle en C₁₋₂₀ qui ne porte aucun substituant ou qui porte un ou des substituant(s) choisi(s) indépendamment parmi les substituants symbolisés par OH, O et N et les substituants halogéno (1 à 5 atomes d'halogène), un groupe aryle en C₆₋₂₀ qui ne porte aucun substituant ou qui porte un ou des substituant(s) choisi(s) indépendamment parmi les substituants symbolisés par OH, O et N et les substituants halogéno (1 à 5 atomes d'halogène), ou un groupe aryl-alkyle en C₇₋₂₀ qui ne porte aucun substituant ou qui porte un ou des substituant(s) choisi(s) indépendamment parmi les substituants symbolisés par OH, O et N et les substituants halogéno (1 à 5 atomes d'halogène) ;
R¹ représente un atome d'hydrogène ou un groupe méthyle, éthyle, isopropyle, phényle ou benzyle ;
R² représente un atome d'hydrogène ;
R³ représente un atome d'hydrogène, un groupe de formule -CH₃, -CH(CH₃)₂, -CH₂-CH(CH₃)₂, -CH(CH₃)-CH₂-CH₃, -CH₂CH₂-S-CH₃, - CH₂OH, -CH(OH)-CH₃, -CH₂-SH, -CH₂-C₆H₄OH, -CH₂-CO-NH₂, -CH₂-CH₂-CO-NH₂, -CH₂-COOH, -CH₂-CH₂-COOH, -(CH₂)₄-NH₂, 1-guanidino-prop-3-yle, benzyl, ou un groupe 4-hydroxy-benzyle, imidazole-4-yle, indole-3-yle, méthoxy-phényle ou éthoxy-phényle ;
R⁴ représente un groupe méthyle, éthyle, propyle, isopropyle, butyle, tertiobutyle, phényle, benzyle, pyridine-1-yle, pyridine-3-yle, pyrimidine-1-yole, pivaloyloxy-méthyle, N-éthyl-morpholinyle, N-(prop-2-yl)-morpholinyle, méthoxy-éthyle, N-méthyl-pipéridine-4-yle, N-méthyl-pipéridine-3-yle, 2-(N,N-diméthylamino)-phényle, 3-(N,N-diméthylamino)-phényle, 4-(N,N-diméthylamino)-phényle, 2-(N,N-diéthylamino)-phényle, 3-(N,N-diéthylamino)-phényle, 4-(N,N-diéthylamino)-phényle, ou 1-éthyl-pipérazinyle ;
Z représente un groupe de formule -CH₂-O-CH₂-CH₂-, ou un groupe de formule IV ou V : 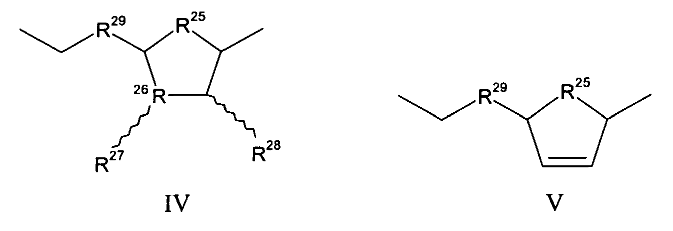 dans lesquelles formules R²⁵ et R²⁹ représentent des atomes d'oxygène, R²⁶ représente un groupe de formule CH, et R²⁷ et R²⁸ représentent des atomes d'hydrogène ;
et B représente un reste adénine-9-yle, 1-déaza-adénine-9-yle, 3-déaza-adénine-9-yle, 7-déaza-8-aza-adénine-9-yle, 8-aza-adénine-9-yle, guanine-9-yle, 2,6-diamino-purine-9-yle, 2-amino-purine-9-yle, thymine-1-yle, cytosine-1-yle, 5-fluoro-cytosine-1-yle, 6-aza-cytosine-1-yle, 5-méthyl-cytosine-1-yle, 5-bromovinyl-uracile-1-yle, 5-fluoro-uracile-1-yle,
ou 5-trifluorométhyl-uracile-1-yle.

2. Composition pharmaceutique comprenant un composé conforme à la revendication 1.

3. Composition pharmaceutique conforme à la revendication 2, pour le traitement d'une infection virale.

4. Composition pharmaceutique conforme à la revendication 2 ou 3, qui est formulée en vue d'une administration par voie orale.

5. Composé conforme à la revendication 1, pour le traitement d'une infection virale.

6. Utilisation d'un composé conforme à la revendication 1 dans la préparation d'un médicament conçu pour le traitement d'une infection virale.

7. Utilisation conforme à la revendication 6, le médicament étant formulé en vue d'une administration par voie orale.
